Europäisches Patentamt

European Patent Office

Office européen des brevets

(19)

(11) Publication number: **0 155 549 B1**

# EUROPEAN PATENT SPECIFICATION

(45) Date of publication of patent specification: **20.03.91**

(21) Application number: **85102093.3**

(22) Date of filing: **26.02.85**

(51) Int. Cl.5: **C12N 15/28, C12N 15/70, C12N 15/62, C12N 1/20, C12P 21/02, C07K 7/10, A61K 37/02,** //(C12N1/20, C12R1:19)

(54) DNA encoding human tumor necrosis factor and human tumor necrosis factor polypeptide.

(30) Priority: **06.03.84 JP 43617/84**
**23.04.84 JP 82653/84**
**17.08.84 JP 172307/84**

(43) Date of publication of application:
**25.09.85 Bulletin 85/39**

(45) Publication of the grant of the patent:
**20.03.91 Bulletin 91/12**

(84) Designated Contracting States:
**AT BE CH DE FR GB IT LI NL SE**

(56) References cited:
**EP-A- 0 158 286**
**EP-A- 0 166 996**
**EP-A- 0 168 214**
**EP-A- 0 183 198**
**WO-A-86/02381**

(73) Proprietor: **Dainippon Pharmaceutical Co., Ltd.**
**25, Doshomachi 3-chome Higashi-ku**
**Osaka-shi, Osaka 541(JP)**

(72) Inventor: **Yamada, Masaaki**
**13, Murasakino Nishirendaino-cho**
**Kita-ku Kyoto-shi Kyoto-fu(JP)**
Inventor: **Furutani, Yasuji**
**6-25-110, Kamishinden 2-chome**
**Toyokana-shi Osaka-fu(JP)**
Inventor: **Notake, Mitsue**
**15-5-601, Yamada Kita**
**Suita-shi Osaka-fu(JP)**
Inventor: **Yamagishi, Juniti**
**23-14-309, Kamishinden 2-chome**
**Toyonaka-shi Osaka-fu(JP)**

(74) Representative: **Kraus, Walter, Dr. et al**
**Patentanwälte Kraus, Weisert & Partner**
**Thomas-Wimmer-Ring 15**
**W-8000 München 22(DE)**

NATURE, vol. 312, no. 5996, December 1984 - January 1985, pages 724-728, London, GB; D. PENNICA et al.: "Human tumour necrosis factor: precursor structure, expression and homology to lymphotoxin"

Affidavits pg. 205-308 from EP 85104109.5

## Description

The present invention relates to

(1) a DNA having or containing a base sequence corresponding to a human tumor necrosis factor polypeptide, its principal portion, or a polypeptide derived from an allelic mutant of the DNA encoding human tumor necrosis factor polypeptide (hereinafter referred to as allelic mutant polypeptide) or a base sequence resulting from modification of said base sequence;

(2) a vector having said DNA inserted thereinto, a host transformed with said vector, a process for producing a polypeptide corresponding to said DNA by using said host;

(3) a polypeptide having or containing a human tumor necrosis factor polypeptide or its principal portion, a human tumor necrosis factor-like substance or its principal portion, or their chemically or enzymatically modified substances; and

(4) a pharmaceutical composition containing said polypeptide or substance, or its use as an antitumor agent in patients.

Carswell et al. found that the sera of mice infected with bacillus Calmette-Guerin (BCG) and then treated with endotoxin contain a substance which necrotizes transplanted Meth A sarcoma; and named it tumor necrosis factor (hereinafter referred to as TNF) [Proc. Nat. Acad. Sci., USA, 72, 3666 (1975)].

TNF is considered to be a physiologically active substance released from macrophages, and is known to be characterized in that

(i) when it is administered to animals bearing a certain kind of tumor (for example, Meth A sarcoma), it causes necrosis in the tumor and cures the animals; (ii) it has a cytotoxic effect in vitro on a certain kind of tumor cells (such as mouse L cells) but has scarcely any injurious effect on normal cells; and (iii) its activity is not animal species-specific.

Because of these characteristics, it has been strongly desired to develop TNF as a new type of antitumor agent.

TNF or a TNF-like substance has been reported in the following literature references or published in the following patent documents.

Green et al., Proc. Nat. Acad. Sci., USA, 73 , 381 (1976).

Matthews et al., Br. J. Cancer, 42 , 416 (1980).

Ruff et al., J. Immunol., 125 , 1671 (1980).

Mannel et al., Infect. Immunity, 28 , 204 (1980).

Haranaka et al., Japan. J. Exp. Med., 51 , 191 (1981).

European Patent Publication No. 90892.

European Patent Publication No. 86475.

Japanese Patent Publication No. 21621 1983.

The processes disclosed in these documents are characterized by involving purification of TNF from body fluids (e.g., blood) or tissues of rabbits, mice, hamsters or guinea pigs as raw materials. The products, however, are not so clearly defined, and it is evident that various restrictions are imposed on these processes in regard to the supply of raw materials and the purities of the final products.

In clinical application as a potent antitumor agent, TNF originated from humans is desirable in consideration of immunogenicity. The above-described processes, however, cannot be applied to the production of human TNF.

The following have so far been reported on an antitumor cytotoxin originated from humans: cytotoxins produced from human peripheral monocytes and human myelocytic monocytic leukemia cells [Matthews, immunology, 44 , 135 (1981)]. from the adherent cells in human peripheral blood cells [Reed, et al., J. Immunol., 115 , 395 (1975)]. and from human B cell lines [Williamson, et al., Proc. Nat. Acad. Sci., USA, 80 , 5397 (1983)]. These substances have cytotoxic activity, but are neither defined clearly.

The present inventors have made various investigations by applying the recombinant DNA technology. These investigations have led to successful cloning of cDNA encoding human TNT polypeptide. In the course of these investigations, the present inventors also found that the human TNF cDNA codes for its precursor polypeptide. Furthermore, the present inventors succeeded in producing human TNF polypeptide in a host transformed with an expression vector having the cloned human TNF cDNA inserted thereinto, and in purifying the human TNF polypeptide to homogeneity.

For simplification of the description, the following abbreviations are used in the present specification and claims.

A:            adenine

C:            cytosine

G:            guanine

| | |
|---|---|
| T: | thymine |
| Ala: | alanine |
| Arg: | arginine |
| Asn | : asparagine |
| Asp: | aspartic acid |
| Cys: | cysteine |
| Gln: | glutamine |
| Glu: | glutamic acid |
| Gly: | glycine |
| His: | histidine |
| Ile: | isoleucine |
| Leu: | leucine |
| Lys: | lysine |
| Met: | methionine |
| Phe: | phenylalanine |
| Pro: | proline |
| Ser: | serine |
| Thr: | threonine |
| Trp: | tryptophan |
| Tyr: | tyrosine |
| Val: | valine |
| DNA: | deoxyribonucleic acid |
| cDNA: | complementary DNA |
| sscDNA: | single-stranded cDNA |
| dscDNA: | double-stranded cDNA |
| RNA: | ribonucleic acid |
| mRNA: | messenger RNA |
| poly(A)mRNA: | poly(A)-containing mRNA |
| dATP: | deoxyadenosine triphosphate |
| dCTP: | deoxycytidine triphosphate |
| dGTP: | deoxyguanosine triphosphate |
| dTTP: | deoxythymidine triphosphate |
| oligo(dC): | oligodeoxycytidylic acid |
| oligo(dG): | oligodeoxyguanylic acid |
| oligo(dT): | oligodeoxythymidylic acid |
| poly(A): | polyadenylic acid |
| poly(U): | polyuridylic acid |
| poly(dC): | polydeoxycytidylic acid |
| poly(dG) | : polydeoxyguanylic acid |
| ATP: | adenosine triphosphate |
| EDTA: | ethylenediaminetetraacetic acid |
| kb: | kilobases |
| kbp: | kilobase pairs |
| bp: | base pairs |
| Meth A sarcoma: | methylcholanthrene-induced sacroma |
| TNF: | tumor necrosis factor |
| rHu-TNF: | recombinant human TNF |

In the present specification, the base sequence shown by a single strand is the base sequence of a sense strand, and the left end is a 5'-terminus and the right end, a 3'-terminus. In the amino acid sequence, the left end is an N-terminus, and the right end, a C-terminus.

A detailed description of the present invention follows together with an account of the background that has led to the present invention. [I-1] DNA encoding a rabbit TNF and the rabbit TNF

The present inventors made various investigations with an eye on the application of recombinant DNA technology, and then succeeded in cloning cDNA encoding a rabbit TNF and elucidated of what the rabbit TNF is. More specifically, the present inventors cultivated rabbit macrophages in vitro together with suitable inducers, and ascertained that the rabbit TNF was produced and released in the culture medium, subsequently, the present inventors found cultivation conditions which caused the rabbit TNF mRNA to be produced and accumulated in high concentrations in the macrophages. The present inventors further

4

succeeded in cloning of cDNAs encoding the rabbit TNF and determined their base sequences, and also found that the rabbit TNF is formed as a precursor. Furthermore, the present inventors succeeded in producing the rabbit TNF in a host transformed with an expression vector having the cloned cDNA inserted thereinto.

Details of the above cDNA encoding the rabbit TNF and the rabbit TNF are described, for example, in U. S. Patent Application serial No. 677,680 filed on November 30, 1984 and European Patent Application No. 84114325.8 filed on November 27, 1984. Their main points are briefly described below.

A typical DNA which encodes a mature rabbit TNF is represented by a base sequence represented by formula [1-1] in the attached Table 1-1.

The DNA having a base sequence represented by formula [1-1] encodes a polypeptide represented by formula [1-2] in the attached Table 1-2.

A DNA encoding a rabbit TNF codes for a precursor of the rabbit TNF and a typical DNA encoding the rabbit TNF precursor is represented by formula [2-1] in the attached Table 2-1 or a DNA resulting from the addition of ATG to its 5'-terminus.

The DNA having the base sequence represented by formula [2-1] encodes a polypeptide represented by formula [2-2] in the attached Table 2-2.

A polypeptide having the amino acid sequence represented by the formula [1-2] in Table 1-2 which is the mature rabbit TNF is not species-specific and is useful as an antitumor agent because it has a selective cytotoxic effect on tumor cells and regresses tumors of tumor-bearing animals.

Table 3 shows the base sequence of one example of cDNA where the rabbit TNF is encoded. The base sequence shown in Table 2-1 corresponds to the base sequence from the 37th to 738th bases in Table 3. However, the first 15 bases are an oligo[dG] tail added for insertion of the cDNA into a vector.

[1-2] The DNAs of this invention are DNAs having or containing a base sequence corresponding to a-human tumor necrosis factor (human TNF) polypeptide or its principal portion(s), or its allelic mutant polypeptide, or a base sequence resulting from modification of said base sequence, and more specifically DNAs having or containing a base sequence corresponding to an amino acid sequence represented by the following formula [I]

$$(Y)_p - (X)_n - (B)_m - A \qquad [I]$$

or a base sequence having a termination codon at the 3'-terminus of said base sequence,
wherein

A is a polypeptide of the formula [Ia] below in which one or more amino acids may be deleted or replaced by other amino acid(s),

B is a peptide of the formula [Ib] below in which one to three amino acids may be deleted or replaced by other amino acid(s),

x is a polypeptide,

Y is Met, and

m, n and p are 1 or 0; the formula [Ia] being as follows:

```
Thr Pro Ser Asp Lys Pro Val Ala His Val
Val Ala Asn Pro Gln Ala Glu Gly Gln Leu
Gln Trp Leu Asn Arg Arg Ala Asn Ala Leu
Leu Ala Asn Gly Val Glu Leu Arg Asp Asn
Gln Leu Val Val Pro Ser Glu Gly Leu Tyr
Leu Ile Tyr Ser Gln Val Leu Phe Lys Gly
Gln Gly Cys Pro Ser Thr His Val Leu Leu
Thr His Thr Ile Ser Arg Ile Ala Val Ser
Tyr Gln Thr Lys Val Asn Leu Leu Ser Ala
Ile Lys Ser Pro Cys Gln Arg Glu Thr Pro
Glu Gly Ala Glu Ala Lys Pro Trp Tyr Glu
Pro Ile Tyr Leu Gly Gly Val Phe Gln Leu
Glu Lys Gly Asp Arg Leu Ser Ala Glu Ile
Asn Arg Pro Asp Tyr Leu Asp Phe Ala Glu
Ser Gly Gln Val Tyr Phe Gly Ile Ile Ala
Leu                             ... [Ia];
```

and the formula [Ib] being as follows:

```
Ser-Ser-Ser-Arg                 ... [Ib].
```

A preferred example of the polypeptide represented by X in formula [I] is a polypeptide represented by the following formula [Ic]:

```
Ser Thr Glu Ser Met Ile Arg Asp Val Glu
Leu Ala Glu Glu Ala Leu Pro Lys Lys Thr
Gly Gly Pro Gln Gly Ser Arg Arg Cys Leu
Phe Leu Ser Leu Phe Ser Phe Leu Ile Val
Ala Gly Ala Thr Thr Leu Phe Cys Leu Leu
His Phe Gly Val Ile Gly Pro Gln Arg Glu
Glu Phe Pro Arg Asp Leu Ser Leu Ile Ser
Pro Leu Ala Gln Ala Val Arg    ... [Ic].
```

Specific examples of the base sequences corresponding to the amino acid sequences represented by the above formulae [Ia], [Ib] and [Ic] are those represented respectively by the following formulae [IIa], [IIb] and [IIc].

```
(5')-ACC CCG AGT GAC AAG CCT GTA GCC
CAT GTT GTA GCA AAC CCT CAA GCT GAG GGG
CAG CTC CAG TGG CTG AAC CGC CGG GCC AAT
GCC CTC CTG GCC AAT GGC GTG GAG CTG AGA
GAT AAC CAG CTG GTG GTG CCA TCA GAG GGC
CTG TAC CTC ATC TAC TCC CAG GTC CTC TTC
AAG GGC CAA GGC TGC CCC TCC ACC CAT GTG
CTC CTC ACC CAC ACC ATC AGC CGC ATC GCC
GTC TCC TAC CAG ACC AAG GTC AAC CTC CTC
TCT GCC ATC AAG AGC CCC TGC CAG AGG GAG
ACC CCA GAG GGG GCT GAG GCC AAG CCC TGG
TAT GAG CCC ATC TAT CTG GGA GGG GTC TTC
CAG CTG GAG AAG GGT GAC CGA CTC AGC GCT
GAG ATC AAT CGG CCC GAC TAT CTC GAC TTT
GCC GAG TCT GGG CAG GTC TAC TTT GGG ATC
ATT GCC CTG-(3')                ...  [IIa]


(5')-TCA-TCT-TCT-CGA-(3')        ...  [IIb]


(5')-AGC ACT GAA AGC ATG ATC CGG GAC
GTG GAG CTG GCC GAG GAG GCG CTC CCC AAG
AAG ACA GGG GGG CCC CAG GGC TCC AGG CGG
TGC TTG TTC CTC AGC CTC TTC TCC TTC CTG
ATC GTG GCA GGC GCC ACC ACG CTC TTC TGC
CTG CTG CAC TTT GGA GTG ATC GGC CCC CAG
AGG GAA GAG TTC CCC AGG GAC CTC TCT CTA
ATC AGC CCT CTG GCC CAG GCA GTC AGA-(3')
                        ...  [IIc].
```

It should be understood that the DNAs in accordance with this invention include the following DNAs.

(1) DNAs encoding a human TNF polypeptide or its principal portoin(s).

(2) DNAs being an allelic mutant of a DNA encoding a human TNF polypeptide or its principal portion(s).

(3) DNAs resulting from modification of DNAs encoding a human TNF polypeptide or its allelic mutant polypeptide.

(4) DNAs resulting from chemical or enzymatic modification cf DNAs encoding a human TNF polypeptide or its allelic mutant polypeptide.

(5) Partially or fully chemically synthesized DNAs corresponding to DNAs encoding a human TNF polypeptide or its principal portion(s).

(6) DNAs encoding a polypeptide of which activity is essentially equivalent to that of a human TNF polypeptide or its principal portion(s).

(7) Any degenerative DNAs encoding a human TNF polypeptide or its principal portion(s).

(8) DNAs encoding a human TNF polypeptide in which one or more codons are deleted or replaced by other codon(s).

(9) DNAs encoding a human TNF polypeptide or its principal portion(s) and having an initiation codon and/or termination codon and/or a promoter followed by Shine-Dalgarno sequence upstream of the initiation codon.

(10) DNAs containing a base sequence which differs from the base sequence encoding rabbit TNF but has high homology to a portion(s) of the base sequence encoding rabbit TNF.

Preferred DNAs in accordance with this invention are

(1) DNA having a base sequence corresponding to an amino acid sequence represented by formula [I] in which A is formula [Ia], B is formula [Ib], X is formula [Ic], m and n are 1, and Y and p are the same as defined in formula [I];

(2) DNA having a base sequence corresponding to an amino acid sequence represented by formula [I] in which A is formula [Ia], B is formula [Ib], m is 1, n is 0, and y and p are the same as defined in formula [I]; and

(3) DNA having a base sequence corresponding to an amino acid sequence represented by formula [I] in which A is formula [Ia], m and n are 0 and Y and p are the same as defined in formula [I].

An especially preferred DNA is one having a base sequence corresponding to an amino acid sequence represented by formula [I] in which A is formula [Ia], B is formula [Ib], m is 1 or 0, and n and p are 0.

The base sequence corresponding to an amino acid sequence of formula [I] in which A is formula [Ia], B is formula [Ib], X is formula [Ic], and m, n and p are each 1 is shown by the sequence of the bases Nos. 1 to 699 in Table 4 in which the bases are numbered. The base sequence corresponding to the amino acid sequence of formula [Ia] is shown by the sequence of the bases Nos. 247 to 699 in Table 4, and the total number of amino acids corresponding to this base sequence is 151.

In Table 4, the upper rows show the base sequence, and the lower rows, the corresponding amino acid sequence.

Examples of the polypeptide of formula [Ia] in which one or more amino acids are deleted or replaced include

(4) a polypeptide of formula [Ia] in which Thr in the first position from the N-terminus has been deleted;

(5) a polypeptide of formula [Ia] in which the amino acid sequence from Thr in the 1st position to Pro in the 6th position from the N-terminus has been deleted;

(6) a polypeptide of formula [Ia] in which the amino acid sequence from Thr in the 1st position to His in 9th position from the N-terminus has been deleted;

(7) a polypeptide of formula [Ia] in which the amino acid sequence from Thr in the 1st position to Ala in the 12th position from the N-terminus has been deleted; and

(8) a polypeptide of formula [Ia] in which the Thr and His in the 66th and 67th positions from the N-terminus have been replaced by His, Thr or Tyr.

The present inventors hold the view that at least

(9) a base sequence of the 577th to the 708th bases in the upper rows of Table 5, and especially

(10) a base sequence of the 658th to the 708th bases in the upper rows of Table 5 are important base sequences of DNA coding for a polypeptide having biological activity.

[I-3] Processes for the production of the DNAs of the invention will be described hereinbelow.

According to this invention, the DNA encoding a human TNF polypeptide or a principal portion thereof can be produced by cultivating human macrophages together with inducer(s), separating a fraction containing human TNF mRNA from the induced cells, preparing a cDNA library from the fraction, and cloning the human TNF cDNA by a differential hybridization method using a suitable probe, for example a rabbit TNF cDNA fragment, or by a differential hybridization method followed by mRNA hybridization-translation assay.

In other words, it can be produced through the following steps.

A. Cultivating human macrophages with inducer(s),

B. separating a fraction containing human TNF mRNA from the induced cells,

C. preparing sscDNA from the mRNA by using reverse transcriptase and then converting it to dscDNA.

D. inserting the dscDNA into a vector,

E. introducing the recombinant vector into a host to transform it and construct a cDNA (colony) library, and

F. cloning cDNA encoding a human TNF polypeptide or its principal portion from the library.

If desired, modification (step G) of the DNA produced as above can give other DNAs of this invention which have or contain a base sequence corresponding to the amino acid sequence represented by formula [I].

Now, the processes for producing the DNA of this invention will be described in more detail. It should be understood however that the operations and conditions in the individual steps of the processes to be described hereinbelow are well known in the art, and the processes of this invention are never limited to these specific processes.

(1) Preparation of human TNF mRNA

The human TNF mRNA can be obtained from human macrophages, for example by the following method.

Human macrophages are obtained, for example, from human alveolus by the method of Sone, et al. [J. Immunol., 129 , 1313 (1982)], from blood by the method of Matthews [Br. J. Cancer, 48 , 405 (1983)], from placenta by the method of Wilson, et al., [J. Immunological Methods, 56 , 305 (1983)], or from other tissues.

The macrophages obtained thus are seeded in a dish at a cell density of about $2 \times 10^4$ to $1 \times 10^6$ cells per $cm^2$, and pre-cultivated at 35 to 38°C, preferably about 37°C in a fully humidified atmosphere containing 5% carbon dioxide for about 30 minutes to 2 hours.

Then, endotoxin obtained from a gram-negative bacterium, preferably lipopolysaccharide derived from Escherichia coli, Pseudomonas aeruginosa or Salmonella typhi , is added as an inducer, and cycloheximide is added as a protein synthesis inhibitor. The cultivation is continued further for 3 to 8 hours to accumulate human TNF mRNA in the macrophages. The pre-cultivation may be omitted. The amount of endotoxin is generally about 0.1 to 1000 micrograms/ml, preferably about 1 to 100 micro-grams, ml. At this time, a phorbol ester such as phorbol-12-myristate-13-acetate, phorbol-12,13-didecanoate or phorbol-11,13-dibenzoate may be further added as an inducer in an amount of about 1 to 2000 ng ml. The amount of the protein synthesis inhibitor varies depending upon its type. For example, in the case of cycloheximide, it is 0.1 to 50 micrograms/ml. Various culture media suitable for the cultivation of mammalian cells can be used as the culture medium. Examples include RPMI-1640, Eagle's MEM medium, and Dulbecco's modified MEM medium [for the compositions of the above media, see, for example, "Cell Cultivation Manual" edited by y. Sohmura, Kodansha (1982), and J. Paul "Cell and Tissue Culture", E. & S. Livingstone Ltd. (1970)]. Preferably, an animal serum (such as fetal bovine serum or calf serum) is added to the culture medium in an amount of about 1 to 20%.

After the cultivation, total RNA is extracted from the cells by a customary method, for example the method of Chirgwin et al. [Biochemistry, 18 , 5294 (1979)], and then by affinity column chromatography on oligo(dT)-cellulose or poly(U)Sepharose, or by a batch method. a fraction containing poly(A)mRNA is separated. An enriched mRNA fraction with human TNF mRNA can be obtained by subjecting the poly(A)-mRNA fraction to acid-urea agarose gel electrophoresis or sucrose density gradient centrifugation.

To confirm that the resulting mRNA fraction is the desired one containing mRNA encoding human TNF polypeptide, the mRNA is translated into a protein and its biological activity is examined. This can be carried out, for example, by injecting the mRNA into the oocytes of Xenopus laevis or adding it to a suitable protein synthesizing system, such as a reticulocyte lysate or wheat germ cell-free protein synthesizing system and by confirming that the translated protein has cytotoxic activity on mouse L cells in vitro.

(2) Cloning of human TNF cDNA

The poly(A)mRNA or the enriched mRNA fraction obtained in step (1) above is used as a template and an oligo(dT) is used as a primer in order to synthesize sscDNA by using reverse transcriptase [for example. that derived from avian myeloblastosis virus (AMV)] in the presence of dATP, dGTP. dCTP and dTTP. Then, the sscDNA is used as a template, and dscDNA is synthesized by using reverse transcriptase or E. coli DNA polymerase I (large fragment).

The resulting dscDNA is inserted, for example, into the restriction endonuclease Pst I cleavage site of plasmid pBR322 by a conventional method, for example the poly(dG)-poly(dC) homopolymer extension method [T. S. Nelson "Methods in Enzymology", 68 , 41 (1979). Academic Press Inc.. New York]. The resulting recombinant plasmids are introduced into a host such as E. coli x 1776 in accordance with the method of Cohen et al. [Proc. Nat. Acad. Sci.. USA. 69 . 2110 (1972)] to transform it. and by selecting tetracycline-resistant colonies, a cDNA (colony) library is prepared.

The cDNA library is subjected to colony hybridization [D. Hanahan et al., Gene. 10 . 63 (1980)] by using a $^{32}$P-labelled rabbit TNF cDNA fragment obtained in Referential Example 1 as a probe. and the desired clones harboring recombinant plasmids containing a cDNA insert encoding human TNF polypeptide are screened.

If such a suitable TNF cDNA probe as shown above cannot be obtained. the desired clones are screened by colony hybridization using induction plus and minus probes. and by hybridization translation assay as follows.

A $^{32}$P-labelled cDNA is synthesized using the poly(A)mRNA fraction or the enriched mRNA fraction containing the human TNF mRNA obtained in step (1) as a template and used as an induction plus probe. Separately, a mRNA fraction, obtained by the same procedure as above except that non-induced macrophages are used as a starting material, is used as a template and $^{32}$P-labelled cDNA is synthesized. The $^{32}$P-labelled cDNA is used as an induction minus probe. From the above cDNA library, plasmid clones which are strongly hybridized with the induction plus probe but not hybridized with the induction minus probe are selected.

The following method is carried out in order to confirm that the resulting clones harbor a cDNA insert encoding human TNF polypeptide. The plasmid DNAs are isolated from the above clones, converted to a single-stranded DNA by heating or alkali treatment, and fixed onto nitrocellulose filters. The mRNA fraction containing human TNF mRNA is added to the filters to hybridize with the fixed DNA. Then, the hybridized mRNA is eluted and recovered. The recovered mRNA is injected into the oocytes of Xenopus laevis to determine whether the recovered mRNA encodes human TNF polypeptide.

The above methods give transformants havoring a recombinant plasmid having a DNA fragment containing a base sequence complementary to the human TNF mRNA.

When the obtained cloned cDNAs do not contain the whole coding region of human TNF polypeptide, cDNAs of a larger size are selected by screening the cDNA library using as a probe the cloned TNF cDNA fragments from the transformants selected as above.

The cloned cDNA encoding a polypeptide containing the amino acid sequence of human TNF polypeptide can be proned finally by analyzing the base sequences of some of the resulting cloned cDNA fragments in accordance with, for example, the Maxam-Gilbert method [Proc. Nat, Acad. Sci., USA, 74 , 560 (1977)] searching for base sequence which have a homology with the base sequence of rabbit TNF cDNA, and selecting cDNAs containing a base sequence corresponding to the whole coding region of human TNF polypeptide.

Homology between the base sequence coding for rabbit TNF and that coding for human TNF polypeptide, and homology between the deduced amino acid sequences of rabbit TNF and human TNF polypeptide are shown in Tables 5 and 6, respectively.

By judging from these homologies and the determined N-terminal and C-terminal amino acid sequences of rabbit plasma TNF (see Referential Example 3 below), it is found that human TNF cDNA codes for its precursor polypeptide of 233 amino acid residues and a mature human TNF polypeptide is a polypeptide corresponding to the 155 amino acid residues from the carboxy-terminus of its precursor.

The mature human TNF polypeptide is coded in the base sequence corresponding to an amino acid sequence represented by formula [I] in which A is formula [Ia], B is formula [Ib], m is 1, and n and p are 0.

The human TNF precursor polypeptide is coded in the base sequence corresponding to an amino acid sequence represented by formula [I] in which A is formula [Ia], B is formula [Ib], X is formula [Ic], and m, n and p are 1, and Y is Met.

High homologies in the base sequences and the deduced amino acid sequences as above may indicate that the human and rabbit TNFs are phylogenetically derived from the same gene. and suggest that a considerable portion(s) of the common regions are a sequence necessary for expressing their biological activities. However, mature human TNF polypeptide did not cross immunologically with rabbit plasma TNF as shown below. It is indicated that the whole amino acid sequence of mature human TNF polypeptide is not always necessary for expressing the activities, and that a partially modified human TNF polypeptide also has the activities so long as it contains an active site of the mature TNF polypeptide.


(3) Modification of the DNA encoding human TNF polypeptide


The DNA encoding the human TNF polypeptide can be modified by techniques known per se to form DNAs having or containing a base sequence corresponding to the amino acid sequence represented by formula [I]. Modification is carried out, for example, by cleaving the DNA with suitable restriction endonuclease(s) and splitting off one or more codons with suitable exonucleases and or endonucleases singly or in combination, followed by replacing with degenerative or other codons, for example, those synthesized chemically by the phosphotriester method [Ohtsuka, E., et al., Heterocycles. 15 , 395 (1981)]. or by ligating without any supplement of codons to prepare DNA having one or more codons deleted.

[II-1] The present invention, as stated above, also relates to a polypeptide having or containing a human tumor necrosis factor polypeptide or its principal portion(s), a human tumor necrosis factor-like substance, its principal portion(s) and their chemically or enzymatically modified substances. They are more specifically a polypeptide of the following formula [I].

$$(Y)_p - (X)_n - (B)_m - A \qquad\qquad [I]$$

or its derivative or a salt thereof,
wherein
A is a polypeptide of the formula [Ia] below in which one or more amino acids may be deleted or replaced by other amino acid(s),
B is a peptide of the formula [Ib] below in which one to three amino acids may be deleted or replaced by other amino acid(s),
X is a polypeptide,
Y is Met, and
m, n and p are 1 or 0; the formula [Ia] being as follows:

```
Thr Pro Ser Asp Lys Pro Val Ala His Val
Val Ala Asn Pro Gln Ala Glu Gly Gln Leu
Gln Trp Leu Asn Arg Arg Ala Asn Ala Leu
Leu Ala Asn Gly Val Glu Leu Arg Asp Asn
Gln Leu Val Val Pro Ser Glu Gly Leu Tyr
Leu Ile Tyr Ser Gln Val Leu Phe Lys Gly
Gln Gly Cys Pro Ser Thr His Val Leu Leu


Thr His Thr Ile Ser Arg Ile Ala Val Ser
Tyr Gln Thr Lys Val Asn Leu Leu Ser Ala
Ile Lys Ser Pro Cys Gln Arg Glu Thr Pro
Glu Gly Ala Glu Ala Lys Pro Trp Tyr Glu
Pro Ile Tyr Leu Gly Gly Val Phe Gln Leu
Glu Lys Gly Asp Arg Leu Ser Ala Glu Ile
Asn Arg Pro Asp Tyr Leu Asp Phe Ala Glu
Ser Gly Gln Val Tyr Phe Gly Ile Ile Ala
Leu                                 ... [Ia];
```

and the formula [Ib] being as follows:

**Ser-Ser-Ser-Arg** ... [Ib].

A preferred example of the polypeptide represented by x in formula [I] is a polypeptide represented by the following formula [Ic].

```
Ser Thr Glu Ser Met Ile Arg Asp Val Glu
Leu Ala Glu Glu Ala Leu Pro Lys Lys Thr
Gly Gly Pro Gln Gly Ser Arg Arg Cys Leu
Phe Leu Ser Leu Phe Ser Phe Leu Ile Val
Ala Gly Ala Thr Thr Leu Phe Cys Leu Leu
His Phe Gly Val Ile Gly Pro Gln Arg Glu
Glu Phe Pro Arg Asp Leu Ser Leu Ile Ser
Pro Leu Ala Gln Ala Val Arg    ... [Ic].
```

It should be understood that the polypeptides in accordance with this invention include the following polypeptides.

(1) A polypeptide corresponding to the DNA of this invention described in section [I] above which is produced by host transformed with an expression vector having the aforesaid DNA inserted thereinto;

(2) a human TNF polypeptide or its precursor polypeptide;

(3) a human TNF polypeptide in which one or more amino acids are deleted or replaced by other amino acid(s);

(4) a polypeptide consisting of a principal portion of the human TNF polypeptide;

(5) a polypeptide which differs from rabbit TNF polypeptide but contains or has a peptide having high homology to a portion(s) of rabbit TNF polypeptide;

(6) a degradated product of the human TNF polypeptide degradated in a host;

(7) a polypeptide resulting from chemical or enzymatic modification of the human TNF polypeptide, or its derivative; and

(8) a polypeptide possessing or potentially possessing biological activities substantially equivalent to those of the human TNF polypeptide.

Preferred polypeptides in accordance with this invention are as follows:-

(1) A polypeptide of formula [I] in which A is formula [Ia], B is formula [Ib], m is 1, n is 0, and Y and p are the same as defined in formula [I],

(2) a polypeptide of formula [I] in which A is formula [Ia], m and n are 0 and Y and p are the same as defined in formula [I], and

(3) a polypeptide of formula [I] in which A is for-mula [Ia], B is formula [Ib], X is formula [Ic], m and n are 1, and Y and p are the same as defined in formula [I].

An especially preferred polypeptide is a polypeptide of formula [I] in which A is formula [Ia], B is formula [Ib], m is 1 or 0, and n and p are 0, or its physiologically acceptable salts.

The polypeptide of formula [Ia] is a polypeptide having the amino acid sequence from the 86th to 236th bases in the upper rows of Table 6.

Polypeptides of formula [Ia] in which one or more amino acids have been deleted or replaced include, for example, the following polypeptides.

(4) A polypeptide of formula [Ia] in which Thr in the 1st position from the N-terminus has been deleted;

(5) a polypeptide of formula [Ia] in which the amino acid sequence from Thr in the 1st position to Pro in the 6th position from the N-terminus has been deleted;

(6) a polypeptide of formula [Ia] in which the amino acid sequence from Thr in the 1st position to His in the 9th position from the N-terminus has been deleted;

(7) a polypeptide of formula [Ia] in which the amino acid sequence from Thr in the 1st position to Ala in the 12th position from the N-terminus has been deleted; and

(8) a polypeptide of formula [Ia] in which the Thr and His in the 66th and 67th positions from the N-terminus have been replaced by His, Thr or Tyr.

The present inventors hold the view that at least

(9) an amino acid sequence from Trp in the 193rd position to Leu in the 236th position in the upper rows of Table 6, and especially

(10) an amino acid sequence from Tyr in the 220th position to Leu in the 236th position in the upper rows of Table 6 are important amino acid sequences of a polypeptide having biological activity.

The derivatives of the polypeptides of formula [I] may, for example, be those formed by utilizing the side chain functinal groups on the chain of the polypeptide of formula [I], the amino group at the N-terminus, or the carboxyl group at the C-terminus, such as an ester formed between the carboxyl group and an aliphatic alcohol, an acid amide formed between the primary or secondary amine with an acid or its derivative, or an O-acyl derivative of the hydroxyl group.

The salts of the polypeptides [I] are salts formed with the carboxyl or amino group of the polypeptides [I], for example salts formed with sodium hydroxide, potassium hydroxide, arginine, caffeine, procaine, hydrochloric acid, and gluconic acid.

The polypeptides [I] may exist as their aggregates, such as a trimer, and such aggregates are naturally included within the polypeptides of this invention. [II-2] Processes for the production of the polypeptides of this invention will be described hereinbelow.

According to this invention, a polypeptide having or containing a human TNF polypeptide or a principal portion thereof can be produced by the following steps.

A. Inserting the DNA having or containing a base sequence encoding human TNF polypeptide or its principal portion, or its modified base sequence into an expression vector,

B. introducing the recombinant vector into a host,

C. cultivating the host transformed with the recombinant vector to produce the polypeptide,

D. collecting the cultured cells and extracting the polypeptide produced from them, and

E. purifying the polypeptide by conventional purifying methods for proteins.

If desired, the polypeptide produced through the above steps may be modified (step F) to produce other polypeptides of this invention represented by formula [I], or their derivatives or salts.

(1) Production of human TNF polypeptide

A detailed description will follow of the processes for producing the human TNF polypeptide by using the DNA of this invention.

An expression vector for production of human TNF polypeptide can be obtained by inserting the cloned cDNA encoding human TNF polypeptide into a suitable vector. All vectors which proliferate in microorganisms to be transformed can be used. Examples include plasmids (such as E . coli plasmid pBR322), phages (such as phage derivatives), and viruses (such as SV40). They may be used singly or in combination, for example as a pBR322-SV40 hybrid plasmid. The site of insertion of the DNA can be properly selected. In other words, a suitable site of a suitable vector may be cleaved with a suitable restriction endonuclease in a customary manner, and the cloned cDNA of a suitable length may be inserted into the cleavage site.

More specifically, an expression vector for production of the non-fused polypeptide is constructed by joining a DNA fragment containing the base sequence encoding the human TNF polypeptide in which the initiation codon ATG is added to the 5'-terminus and the termination codon (TAA, TAG or TGA) exists at the 3'-terminus, to a DNA fragment with a suitable promoter and the Shine-Dalgarno sequence and inserting it into a vector. An expression vector for the production of the fused polypeptide may be constructed by inserting the cDNA fragment having the base sequence encoding the human TNF polypeptide in which the termination codon is added to the 3'-terminus into the vector so that the translational reading frame coincides with that of the structure gene to be fused. The process for the production of human TNF polypeptide as a fused polypeptide has the advantage of minimizing degradation of the product in the transformed host cells. In this case. human TNF polypeptide should be cut out from the fused product. Mature human TNF polypeptide corresponding to an amino acid sequence from Ser in the 82nd position to Leu in the 236th position in the upper rows of Table 6 does not contain any methionine as a component. Therefore, by using an expression vector constructed by inserting the human TNF cDNA fragment ligated with the 3'-terminus of the base sequence of a structure gene to be fused through a methionine codon (ATG), the human TNF polypeptide is easily obtained from the fused product by a method of cleavage of methionyl peptide bond. for example. by a cyanogen bromide treatment [Itakura. K., et al., Science 198. 1054 (1977)].

Examples of the promoters are lac. trp tac. phoS . phoA, PL and SV40 early promoters.

Transformants are obtained by introducing the expression vector into a host such as microorganism. animal or plant cells. For example, E . coli is transformed by the method of Cohen et al. [Proc. Nat. Acad. Sci., USA. 69 . 2110 (1972)]. Then, by cultivating one of the transformants. a human TNF polypeptide or the polypeptide with a methionine at its N-terminus is produced. The product can be accumulated either in the cytoplasm or in the periplasm of the host cells depending upon the method of constructing the expression vector. To cause the polypeptide to be secreted in the periplasm. one can construct an expression vector by using a gene coding for a secretory protein, such as an alkaline phosphatase gene (phoa ) or a phosphate binding protein gene (phos ), and joining DNA encoding the human TNF polypeptide in the correct translational reading frame to the above gene at a suitable site following a DNA region encoding the signal peptide.

The resulting transformants are cultivated under suitable conditions for the transformants until the polypeptide desired is fully produced. Then, the polypeptide is extracted from the culture. When the produced polypeptide is accumulated in the cytoplasm, the host cells are destroyed by lysozyme digestion and freezing and thawing or sonication or by using a French press, and then centrifuged or filtered to collect the extract. When it is accumulated in the periplasm, it can be extracted, for example, by the method of Willsky et al. [J. Bacteriol., 127 , 595 (1976)].

The crude polypeptide so obtained can be purified by conventional purifying methods for proteins, for example by combinations of salting out, ultrafiltration, dialysis, ion exchange chromatography, gel filtration, electrophoresis, affinity chromatography, etc.

By the foregoing process, the human TNF polypeptide of the invention and/or the polypeptide with a methionine at the N-terminus of the polypeptide can be produced.

Polypeptides in accordance with this invention other than the human TNF polypeptide and the polypeptide with a methionine at the N-terminus of the polypeptide can also be produced by using the desired DNA substantially in accordance with the above process, or by using proper combinations of known processes.

(2) Modification of human TNF polypeptide

The modified human TNF polypeptides mean polypeptides derived from the allelic mutants of the DNA encoding human TNF polypeptide (allelic mutant polypeptide), a polypeptide resulting from addition of an amino acid or peptide (consisting of two or more amino acids) to the N-terminus or C-terminus of the human TNF polypeptide or the allelic mutant polypeptide, a polypeptide resulting from deletion of one or more amino acids from the human TNF polypeptide or the allelic mutant-polypeptide (for example, deletion of 4 amino acids from the N-terminus of human TNF polypeptide as shown in Section III-1, (6) below), derivatives such as esters, acyl-derivatives or acid amides, formed by using a functional group in the molecule, an amino residue of N-terminus or a carboxy residue of the C-terminus, and its salt formed by using amino residues or carboxy residues with, for example, sodium hydroxide, potassium hydroxide, arginine, caffeine, procaine, hydrochloric acid, gluconic acid and so on.

Modification of the human TNF polypeptide or its allelic mutant polypeptide is carried out by techniques known per se , which are published in, for example, "Chemical Modification of Proteins, by Means, G.E. and Feeney, R.E., Holden-Day. Inc. California (1971)", to produce the modified human TNF polypeptide as mentioned above.

[III] The chemical and physicochemical properties, biological activities and immunological property of the typical polypeptides of this invention will be described below in detail.

Purified human TNF polypeptide obtained in Example 5 (to be referred to as recombinant human TNF, abbreviated to rHu-TNF) was used for analyses as shown below.

[III-1] Chemical and physicochemical properties

(1) Molecular weight

The molecular weight of rHu-TNF was measured by gel filtration analysis with TSK-gel G3000 SW column (7.5 x 600 mm, Toyo Soda) in accordance with high-performance liquid chromatography using 0.2M phosphate (pH 7) buffer with and without 8M urea and 0.5% 2-mercaptoethanol as a solvent. As molecular weight marker proteins, the following proteins were used: bovine serum albumin: (MW: $6.6 \times 10^4$). rabbit triosephosphate isomerase (MW: $5.3 \times 10^4$), ovalbumin (MW: $4.5 \times 10^4$). porcine pepsin (MW: $3.27 \times 10^4$). soybean trypsin inhibitor (MW: $2.05 \times 10^4$). horse myoglobin (MW: $1.78 \times 10^4$). and horse cytochrome c (MW: $1.24 \times 10^4$).

As a result, rHu-TNF had a molecular weight of 45,000 ± 5,000 daltons and 18,000 ± 3,000 daltons in the absence and presence of urea and 2-mercaptoethanol. respectively.

The cDNA inserted into the expression plasmid pHTR91 encoded 155 amino acid residues (omitting a methionine derived from an initiation codon ATG). The theoretical molecular weight of rHu-TNF was calculated as 17,097 daltons from the amino acid sequence deduced. The calculated molecular weight agreed with the value determined in the presence of urea and 2-mercaptoethanol.

This finding indicates that rHu-TNF occurs as a monomer (subunit) in the presence of urea and 2-mercaptoethanol, but exists as an aggregate, for example a trimer in the absence of denaturants.

(2) Isoelectric Point

The isoelectric point was determined by isoelectrofocusing gel electrophoresis at 3 watt for 3 hours using a 5% polyacrylamide flat gel with a pH gradient ranging from pH 4.0 to pH 6.5 created with Pharmalyte (Pharmacia).

Protein was stained with Coomassie brilliant blue. Separately, the gel was sliced into 3 mm width and immersed in 20 mM Tris-HCl (pH 7.8) buffer to elute a protein. Cytotoxic activity was obviously detected in an elute from the gel sliced from the position corresponding to the position of a protein detected by the staining.

Isoelectric point of rHu-TNF was found to be 5.9 ± 0.3.

(3) Amino acid composition

The amino acid composition of rHu-TNF was determined with a micro-amino acid analyzer (Shimadzu Seisakusho) by a fluorometric method using orthophthalaldehyde after the sample was hydrolyzed with hydrochloric acid.

Fifty micrograms of rHu-TNF was hydrolyzed in 6N HCl at 110°C. The contents of the amino acids were calculated by correcting on the basis of the values determined with each sample hydrolyzed for 24, 48 and 72 hours. Cystine (or cysteine) was determined as a cysteic acid converted by performic acid oxidation. Tryptophan was determined by a fluorometric method of Pajot [Eur. J. Biochem., 63 . 263 (1976)].

The results are summarized in Table 7.

The amino acid composition well agreed with that deduced from the base sequence encoding human TNF polypeptide.

(4) Determination of N-terminal amino acid sequence

The N-terminal amino acid sequence of rHu-TNF was determined by the Edman degradation method [Arch. Biochem. Biophys., 22 . 475 (1949)].

A phenylthiohydantoin-amino acid derived from a N-terminal amino acid by the Edman degradation method was identified by high-performance liquid chromatography using a column (4.6 x 250 mm) of TSK-gel ODS-120A (Toyo Soda). These procedures were serially repeated to determine a newly formed N-terminal amino acid sequentially.

It was consequently found that the N-terminal amino acid sequence of rHu-TNF was as follows:

$$NH_2-Ser-Ser-Ser-Arg-Thr-Pro-Ser-Asp-\ -\ -$$

It has been known that some polypeptides produced in microorganisms by application of the recombinant DNA technology have a methionine residue derived from an initiation codon (ATG) at its N-terminus. However, in the case of rHu-TNF obtained in Example 5. a methionine residue could not be detected at the N-terminus and completely removed off.

(5) Determination of C-terminal amino acid sequence

The C-terminal amino acid sequence of rHu-TNF was determined by the enzymatic method using carboxypeptidases.

The rHu-TNF was digested with carboxypeptidase-A and carboxypeptidase-Y at the molar ratios of enzyme to substrate of 1:25 and 1:1,000, respectively. The free amino acids released from the C-terminus of rHu-TNF by the double digestion were identified by a micro-amino acid anayzer (Shimadzu Seisakusho) at appropriate intervals from 2 minutes to 180 minutes after the digestion.

It was consequently found that the C-terminal amino acid sequence of rHu-TNF was as follows:

$$-\ -\ -Tyr-Phe-Gly-Ile-Ile-Ala-Leu-COOH$$

(6) Trypsin digestion of rHu-TNF

Five hundred micrograms of rHu-TNF was digested with 20 micrograms of TPCK-treated trypsin (type XIII, SIGMA Chemical Co.) in 5 mM Tris-HCl (pH 7.8) buffer at room temperature for 5 hours. The digested product was subjected to a preparative isoelectrofocusing gel electrophoresis as shown in Example 5-(2). The proteins were stained with Coomassie brilliant blue. Separately, the gel was sliced into 3 mm width and the sliced gels were immersed in 20 mM Tris-HCl (pH 7.8) buffer to elute a protein.

As a result, the digested product eluted from the sliced gel corresponding to the pH zone being about 0.3 lower than the isoelectric point of rHu-TNF had cytotoxic activity.

The digested product with cytotoxic activity was subjected to determination of the N-terminal and C-terminal amino acid sequences by the methods as described in sections (5) and (6), respectively.

Consequently, the partial amino acid sequence of the digested product were as follows:

$$N\text{-terminal;}\quad NH_2\text{-Thr-Pro-Ser-Asp-}\ -\ -$$
$$C\text{-terminal;}\quad -\ -\ -Ile\text{-Ala-Leu-COOH}$$

By judging from the amino acid sequence, the digested product is a polypeptide resulting from splitting off of the four N-terminal amino acids (Ser-Ser-Ser-Arg) from the rHu-TNF, and has a cytotoxic activity as mentioned above. It means that at least four amino acids at the N-terminus of rHu-TNF are not essential to its biological activity.

[III-2] Biological activities

(1) Cytotoxic activity against mouse L-M cell

The method of measuring the cytotoxic activity against mouse L-M cell (ATCC, CCL 1.2) was as follows:

A sample (0.1 ml) diluted serially with the below-mentioned medium and 0.1 ml of a mouse L-M cell suspension (1 x 10⁵ cells ml) were added to each well of a 96 well multi-well plate (Flow Labs.). The Eagle's minimum essential medium [see, for example, J. Paule "Cell and Tissue Culture", E & S. Livingstone Ltd. (1970)] containing 1% fetal bovine serum was used. The plate was incubated at 37° C for 48 hours in a fully humidified atmosphere containing 5% carbon dioxide. After incubation, 20 microliters of 25% glutaraldehyde was added to fix the viable cells. After fixation, the plate was washed and dried. Then, 0.1 ml of 0.05% methylene blue solution was added to stain the fixed cells. The excess of methylene blue was washed off, and the plate was dried. Methylene blue associated with the fixed cells was eluted with 200 microliters of 0.36N HCl and its absorbance at 665 nm was measured with a Titertek Multiscan (Flow Labs.). The absorbance is proportional to the number of the viable cells. The concentration of biological activity required to kill 50% of the L-M cells was defined as one unit ml. The cytotoxic acitivty against mouse L-M cells determined under the conditions as above was represented by "units (LM)". to distinguish from the cytotoxic activity against mouse L-929 cells as a target cell.

The protein content was determined by the method of Lowry. O.H., et al. [J. Bio. Chem., 193 . 265 (1951)].

As a result the rHu-TNF had a specific activity of 2 x 10⁶ units (LM) or more per mg of protein.

(2) Antitumor effect on Meth A sarcoma transplanted into mice

The antitumor effect on mice bearing Meth A sarcoma was evaluated by the following method.

BALB.c mice weighing about 23 g were intradermally transplanted with 2 x 10⁵ Meth A sarcoma cells into the abdominal skin, and seven days later, mice were selected whose tumor was 6-7 mm in diameter. On the 7th day after the tumor transplantation, the rhu-TNF was administered into the tumor mass or

16

intravenously. Endotoxin content of the rHu-TNF preparation was less than 0.01 ng per $1 \times 10^4$ units (LM) of its cytotoxic activity.

As a result, by administration into the tumor mass, a necrotic response in the tumor transplant was observed in all mice injected with rHu-TNF at a dose of $1 \times 10^3$, $3 \times 10^3$ and $1 \times 10^4$ units (LM) per mouse within 24 hours after the injection, and the tumor was completely regressed at a ratio of 3/5, 5/5 and 5/5 at each dose as shown above, respectively. By intravenous administration, a necrotic response was observed in all mice injected with rHu-TNF at a dose of $3 \times 10^3$ and $1 \times 10^4$ units (LM) per mouse, and the ratio of complete regression was 3/5 and 4/5, respectively.

(3) Inhibitory effect of rHu-TNF on the growth of human tumor cells in vitro

The inhibitory effect of rHu-TNF on the growth of human tumor cells and normal cells was evaluated in vitro under the following conditions. Human tumor cells or normal cells were seeded at $1 \times 10^4$ cells per well in 1 ml of Eagle's minimum essential medium containing 10% fetal bovine serum using a 24 well multi-well plate. rHu-TNF was added at a final concentration of 100 units (LM)/ml and then cultivated at $37^\circ$ C for 4 days in a fully humidified atmosphere containing 5% carbon dioxide. Four hours before the termination of cultivation, 1 microcurie of $^3$H-thymidine was added into each well. After cultivation, the cells were washed with phosphate buffered saline, and lysed with 0.5% sodium dodecylsulfate. The amount of H-thymidine incorporated into the cells was determined by counting the radioactivity in the lysate.

The inhibitory effect was represented by a ratio of the growth inhibition calculated by the following equation:

Ratio of growth inhibition (%) $= (a-b)/a \times 100$

wherein, a and b are the radioactivities incorporated into the cells in the absence of rHu-TNF and in the presence of rHu-TNF, respectively.

The results summarized in Table 8 show that rHu-TNF significantly inhibited the growth of human tumor cells, but did not affect normal cells. This finding indicates that rHu-TNF attacks tumor cells selectively.

[III-3] Immunological property

The rHu-TNF solution [100 units (LM)/ml] was mixed with an equal volume of a 100-fold dilution of the purified anti-rabbit plasma TNF antibody obtained in Referential Example 4. After incubation at $37^\circ$ C for 2 hours, the cytotoxic activity of the reaction mixture was measured by the method as described above using L-M cells as a target cell.

As a result, the cytotoxic activity of rHu-TNF was not neutralized with the antibody at all. It was found therefore that human TNF polypeptide was immunologically distinguishable from rabbit TNF.

[IV] For formulating the polypeptides of this invention, they may be in the form of a solution or a lyophilized product. From the standpoint of long-term stability, they are desirably in the form of lyophilized products. It is preferred to add vehicles or stabilizers to the preparations. Examples of the stabilizers include albumin, globulin, gelatin, protamine, protamine salts, glucose, galactose, xylose, mannitol, glucuronic acid, trehalose, dextran, hydroxyethyl starch, and nonionic surface-active agents (such as polyoxyethylene fatty acid esters, polyoxyethylene alkyl ethers, polyoxyethylene alkyl phenyl ethers, polyoxyethylene sorbitan fatty acid esters, polyoxyethylene glycerin fatty acid esters, polyoxyethylene hardened castor oil, polyoxyethyene castor oil, polyoxyethylene polyoxypropylene alkyl ethers, polyoxyethylene polyoxypropylene block copolymer, sorbitan fatty acid esters, sucrose fatty acid esters and glycerin fatty acid esters).

[V] The polypeptides of this invention are useful as antitumor agents because they have a selective cytotoxic effect on tumor cells and regress tumors of tumor-bearing animals.

Such polypeptide preparations are preferably administered parenterally or topically. Parenteral routes such as intraveous and intramuscular routes are used when tumor cells extend over a wide range or metastasize, or when prevention of metastasis is intended. Against local tumor tissues, direct intratumor administration is preferred. The dosage varies depending upon the type and size of tumors, the condition of the patient and the route of administration. Usually, it is $1 \times 10^2$ to $1 \times 10^7$ units (LM)/kg, preferably $1 \times 10^3$ to $1 \times 10^5$ units (LM)/kg.

## Table 1-1

```
(5')-TCA GCT TCT CGG GCC CTG AGT GAC AAG
CCT CTA GCC CAC GTA GTA GCA AAC CCG CAA
GTG GAG GGC CAG CTC CAG TGG CTG AGC CAG
CGT GCG AAC GCC CTG CTG GCC AAC GGC ATG
AAG CTC ACG GAC AAC CAG CTG GTG GTG CCG
GCC GAC GGG CTG TAC CTC ATC TAC TCC CAG
GTT CTC TTC AGC GGT CAA GGC TGC CGC TCC
TAC GTG CTC CTC ACT CAC ACT GTC AGC CGC
TTC GCC GTC TCC TAC CCG AAC AAG GTC AAC
CTC CTC TCT GCC ATC AAG AGC CCC TGC CAC
CGG GAG ACC CCC GAG GAG GCT GAG CCC ATG
GCC TGG TAC GAG CCC ATC TAC CTG GGC GGC
GTC TTC CAG TTG GAG AAG GGT GAC CGG CTC
AGC ACC GAG GTC AAC CAG CCT GAG TAC CTG
GAC CTT GCC GAG TCC GGG CAG GTC TAC TTT
GGG ATC ATT GCC CTG-(3')        .....[1-1]
```

## Table 1-2

```
Ser Ala Ser Arg Ala Leu Ser Asp Lys Pro
Leu Ala His Val Val Ala Asn Pro Gln Val
Glu Gly Gln Leu Gln Trp Leu Ser Gln Arg
Ala Asn Ala Leu Leu Ala Asn Gly Met Lys
Leu Thr Asp Asn Gln Leu Val Val Pro Ala
Asp Gly Leu Tyr Leu Ile Tyr Ser Gln Val
Leu Phe Ser Gly Gln Gly Cys Arg Ser Tyr
Val Leu Leu Thr His Thr Val Ser Arg Phe
Ala Val Ser Tyr Pro Asn Lys Val Asn Leu
Leu Ser Ala Ile Lys Ser Pro Cys His Arg
Glu Thr Pro Glu Glu Ala Glu Pro Met Ala
Trp Tyr Glu Pro Ile Tyr Leu Gly Gly Val
Phe Gln Leu Glu Lys Gly Asp Arg Leu Ser
Thr Glu Val Asn Gln Pro Glu Tyr Leu Asp
Leu Ala Glu Ser Gly Gln Val Tyr Phe Gly
Ile Ile Ala Leu                  .....[1-2]
```

## Table 2-1

(5')-AGC ACT GAG AGT ATG ATC CGG GAC GTC

GAG CTG GCG GAG GGG CCG CTC CCC AAG AAG

GCA GGG GGG CCC CAG GGC TCC AAG CGC TGC

CTC TGC CTC AGC CTC TTC TCT TTC CTG CTC

GTG GCT GGA GCC ACC ACG CTC TTC TGC CTG

CTG CAC TTC AGG GTG ATC GGC CCT CAG GAG

GAA GAG CAG TCC CCA AAC AAC CTC CAT CTA

GTC AAC CCT GTG GCC CAG ATG GTC ACC CTC

AGA TCA GCT TCT CGG GCC CTG AGT GAC AAG

CCT CTA GCC CAC GTA GTA GCA AAC CCG CAA

GTG GAG GGC CAG CTC CAG TGG CTG AGC CAG

CGT GCG AAC GCC CTG CTG GCC AAC GGC ATG

AAG CTC ACG GAC AAC CAG CTG GTG GTG CCG

GCC GAC GGG CTG TAC CTC ATC TAC TCC CAG

GTT CTC TTC AGC GGT CAA GGC TGC CGC TCC

TAC GTG CTC CTC ACT CAC ACT GTC AGC CGC

TTC GCC GTC TCC TAC CCG AAC AAG GTC AAC

CTC CTC TCT GCC ATC AAG AGC CCC TGC CAC

CGG GAG ACC CCC GAG GAG GCT GAG CCC ATG

GCC TGG TAC GAG CCC ATC TAC CTG GGC GGC

GTC TTC CAG TTG GAG AAG GGT GAC CGG CTC

AGC ACC GAG GTC AAC CAG CCT GAG TAC CTG

GAC CTT GCC GAG TCC GGG CAG GTC TAC TTT

GGG ATC ATT GCC CTG-(3')      ..... [2-1]

## Table 2-2

Ser Thr Glu Ser Met Ile Arg Asp Val Glu
Leu Ala Glu Gly Pro Leu Pro Lys Lys Ala
Gly Gly Pro Gln Gly Ser Lys Arg Cys Leu
Cys Leu Ser Leu Phe Ser Phe Leu Leu Val
Ala Gly Ala Thr Thr Leu Phe Cys Leu Leu
His Phe Arg Val Ile Gly Pro Gln Glu Glu
Glu Gln Ser Pro Asn Asn Leu His Leu Val
Asn Pro Val Ala Gln Met Val Thr Leu Arg
Ser Ala Ser Arg Ala Leu Ser Asp Lys Pro
Leu Ala His Val Val Ala Asn Pro Gln Val
Glu Gly Gln Leu Gln Trp Leu Ser Gln Arg
Ala Asn Ala Leu Leu Ala Asn Gly Met Lys
Leu Thr Asp Asn Gln Leu Val Val Pro Ala
Asp Gly Leu Tyr Leu Ile Tyr Ser Gln Val
Leu Phe Ser Gly Gln Gly Cys Arg Ser Tyr
Val Leu Leu Thr His Thr Val Ser Arg Phe
Ala Val Ser Tyr Pro Asn Lys Val Asn Leu
Leu Ser Ala Ile Lys Ser Pro Cys His Arg
Glu Thr Pro Glu Glu Ala Glu Pro Met Ala
Trp Tyr Glu Pro Ile Tyr Leu Gly Gly Val
Phe Gln Leu Glu Lys Gly Asp Arg Leu Ser
Thr Glu Val Asn Gln Pro Glu Tyr Leu Asp
Leu Ala Glu Ser Gly Gln Val Tyr Phe Gly
Ile Ile Ala Leu                    ..... [2-2]

## Table 3

```
          10        20        30   HaeII 40        50        60
           •         •         •         •         •         •
GGGGGGGGGGGGGGGGGGCCCTCTGGAGAGAGCGCCATGAGCACTGAGAGTATGATCCGGGAC
CCCCCCCCCCCCCCCCGGGAGACCTCTCTCGCGGTACTCGTGACTCTCATACTAGGCCCTG

          70        80        90       100       110       120
           •         •         •         •         •         •
GTCGAGCTGGCGGAGGGGCCGCTCCCCAAGAAGGCAGGGGGGCCCCAGGGCTCCAAGCGC
CAGCTCGACCGCCTCCCCGGCGAGGGGTTCTTCCGTCCCCCCGGGGTCCCGAGGTTCGCG
                                                       HaeII

         130       140       150       160       170       180
           •         •         •         •         •         •
TGCCTCTGCCTCAGCCTCTTCTCTTTCCTGCTCGTGGCTGGAGCCACCACGCTCTTCTGC
ACGGAGACGGAGTCGGAGAAGAGAAAGGACGAGCACCGACCTCGGTGGTGCGAGAAGACG

         190       200       210       220       230       240
           •         •         •         •         •         •
CTGCTGCACTTCAGGGTGATCGGCCCTCAGGAGGAAGAGCAGTCCCCAAACAACCTCCAT
GACGACGTGAAGTCCCACTAGCCGGGAGTCCTCCTTCTCGTCAGGGGTTTGTTGGAGGTA

         250       260       270       280   AvaI290       300
           •         •         •         •         •         •
CTAGTCAACCCTGTGGCCCAGATGGTCACCCTCAGATCAGCTTCTCGGGCCCTGAGTGAC
GATCAGTTGGGACACCGGGTCTACCAGTGGGAGTCTAGTCGAAGAGCCCGGGACTCACTG

         310       320       330       340       350       360
           •         •         •         •         •         •
AAGCCTCTAGCCCACGTAGTAGCAAACCCGCAAGTGGAGGGCCAGCTCCAGTGGCTGAGC
TTCGGAGATCGGGTGCATCATCGTTTGGGCGTTCACCTCCCGGTCGAGGTCACCGACTCG

         370       380       390       400       410       420
           •         •         •         •         •         •
CAGCGTGCGAACGCCCTGCTGGCCAACGGCATGAAGCTCACGGACAACCAGCTGGTGGTG
GTCGCACGCTTGCGGGACGACCGGTTGCCGTACTTCGAGTGCCTGTTGGTCGACCACCAC
```

− to be continued −

EP 0 155 549 B1

### Table 3 (continued)

```
      430       440       450       460       470       480
CCGGCCGACGGGCTGTACCTCATCTACTCCCAGGTTCTCTTCAGCGGTCAAGGCTGCCGC
GGCCGGCTGCCCGACATGGAGTAGATGAGGGTCCAAGAGAAGTCGCCAGTTCCGACGGCG

      490       500       510       520       530       540
TCCTACGTGCTCCTCACTCACACTGTCAGCCGCTTCGCCGTCTCCTACCCGAACAAGGTC
AGGATGCACGAGGAGTGAGTGTGACAGTCGGCGAAGCGGCAGAGGATGGGCTTGTTCCAG

      550       560       570       580       590       600
AACCTCCTCTCTGCCATCAAGAGCCCCTGCCACCGGGAGACCCCCGAGGAGGCTGAGCCC
TTGGAGGAGAGACGGTAGTTCTCGGGGACGGTGGCCCTCTGGGGGCTCCTCCGACTCGGG
                                              Ava I

      610       620       630       640       650       660
ATGGCCTGGTACGAGCCCATCTACCTGGGCGGCGTCTTCCAGTTGGAGAAGGGTGACCGG
TACCGGACCATGCTCGGGTAGATGGACCCGCCGCAGAAGGTCAACCTCTTCCCACTGGCC

      670       680       690       700       710       720
CTCAGCACCGAGGTCAACCAGCCTGAGTACCTGGACCTTGCCGAGTCCGGGCAGGTCTAC
GAGTCGTGGCTCCAGTTGGTCGGACTCATGGACCTGGAACGGCTCAGGCCCGTCCAGATG

      730       740       750       760       770       780
TTTGGGATCATTGCCCTGTGAGGGGACTGACCACCACTCCTCCCCCTCTCCCACCCCAGC
AAACCCTAGTAACGGGACACTCCCCTGACTGGTGGTGAGGAGGGGGAGAGGGTGGGGTCG

      790       800
CCCCTCACTCTGGGCGCCCTCAG
GGGGAGTGAGACCCGCGGGAGTC
```

## Table 4

```
                                    GACCCACGG
     -30        -20        -10          -1
      :          :          :            :
      CTCCACCCTCTCTCCCCTGGAAAGGACACC

      1         10         20         30
      :          :          :          :
      ATGAGCACTGAAAGCATGATCCGGGACGTG
      MetSerThrGluSerMetIleArgAspVal

                40         50         60
                 :          :          :
      GAGCTGGCCGAGGAGGCGCTCCCCAAGAAG
      GluLeuAlaGluGluAlaLeuProLysLys

                70         80         90
                 :          :          :
      ACAGGGGGGCCCCAGGGCTCCAGGCGGTGC
      ThrGlyGlyProGlnGlySerArgArgCys

               100        110        120
                 :          :          :
      TTGTTCCTCAGCCTCTTCTCCTTCCTGATC
      LeuPheLeuSerLeuPheSerPheLeuIle

               130        140        150
                 :          :          :
      GTGGCAGGCGCCACCACGCTCTTCTGCCTG
      ValAlaGlyAlaThrThrLeuPheCysLeu

               160        170        180
                 :          :          :
      CTGCACTTTGGAGTGATCGGCCCCCAGAGG
      LeuHisPheGlyValIleGlyProGlnArg

               190        200        210
                 :          :          :
      GAAGAGTTCCCCAGGGACCTCTCTCTAATC
      GluGluPheProArgAspLeuSerLeuIle

               220        230        240
                 :          :          :
      AGCCCTCTGGCCCAGGCAGTCAGATCATCT
      SerProLeuAlaGlnAlaValArgSerSer

               250        260        270
                 :          :          :
      TCTCGAACCCCGAGTGACAAGCCTGTAGCC
      SerArgThrProSerAspLysProValAla

               280        290        300
                 :          :          :
      CATGTTGTAGCAAACCCTCAAGCTGAGGGG
      HisValValAlaAsnProGlnAlaGluGly

               310        320        330
                 :          :          :
      CAGCTCCAGTGGCTGAACCGCCGGGCCAAT
      GlnLeuGlnTrpLeuAsnArgArgAlaAsn

               340        350        360
                 :          :          :
      GCCCTCCTGGCCAATGGCGTGGAGCTGAGA
      AlaLeuLeuAlaAsnGlyValGluLeuArg
```

                                    - to be continued -

23

## Table 4 (continued)

```
                370        380        390
                 |          |          |
        GATAACCAGCTGGTGGTGCCATCAGAGGGC
        AspAsnGlnLeuValValProSerGluGly

                400        410        420
                 |          |          |
        CTGTACCTCATCTACTCCCAGGTCCTCTTC
        LeuTryLeuIleTyrSerGlnValLeuPhe

                430        440        450
                 |          |          |
        AAGGGCCAAGGCTGCCCCTCCACCCATGTG
        LysGlyGlnGlyCysProSerThrHisVal

                460        470        480
                 |          |          |
        CTCCTCACCCACACCATCAGCCGCATCGCC
        LeuLeuThrHisThrIleSerArgIleAla

                490        500        510
                 |          |          |
        GTCTCCTACCAGACCAAGGTCAACCTCCTC
        ValSerTyrGlnThrLysValAsnLeuLeu

                520        530        540
                 |          |          |
        TCTGCCATCAAGAGCCCCTGCCAGAGGGAG
        SerAlaIleLysSerProCysGlnArgGlu

                550        560        570
                 |          |          |
        ACCCCAGAGGGGGCTGAGGCCAAGCCCTGG
        ThrProGluGlyAlaGluAlaLysProTrp

                580        590        600
                 |          |          |
        TATGAGCCCATCTATCTGGGAGGGGTCTTC
        TyrGluProIleTyrLeuGlyGlyValPhe

                610        620        630
                 |          |          |
        CAGCTGGAGAAGGGTGACCGACTCAGCGCT
        GlnLeuGluLysGlyAspArgLeuSerAla

                640        650        660
                 |          |          |
        GAGATCAATCGGCCCGACTATCTCGACTTT
        GluIleAsnArgProAspTyrLeuAspPhe

                670        680        690
                 |          |          |
        GCCGAGTCTGGGCAGGTCTACTTTGGGATC
        AlaGluSerGlyGlnValTyrPheGlyIle

                700        710        720
                 |          |          |
        ATTGCCCTGTGAGGAGGACGAACATCCAAC
        IleAlaLeu

                730        740
                 |          |
        CTTCCCAAACGCCTCCCCTGC
```

## Table 5

```
                                              30
ATGAGCACTGAAAGCATGATCCGGGACGTG
ATGAGCACTGAGAGTATGATCCGGGACGTC
                                              60
GAGCTGGCCGAGGAGGCGCTCCCCAAGAAG
GAGCTGGCGGAGGGGCCGCTCCCCAAGAAG
                                              90
ACAGGGGGGCCCCAGGGCTCCAGGCGGTGC
GCAGGGGGGCCCCAGGGCTCCAAGCGCTGC
                                             120
TTGTTCCTCAGCCTCTTCTCCTTCCTGATC
CTCTGCCTCAGCCTCTTCTCTTTCCTGCTC
                                             150
GTGGCAGGCGCCACCACGCTCTTCTGCCTG
GTGGCTGGAGCCACCACGCTCTTCTGCCTG
                                             180
CTGCACTTTGGAGTGATCGGCCCCCAGAGG
CTGCACTTCAGGGTGATCGGCCCTCAGGAG
                                             210
GAAGAG---TTCCCCAGGGACCTCTCTCTA
GAAGAGCAGTCCCCAAACAACCTCCATCTA
                                             240
ATCAGCCCTCTGGCCCAG------GCAGTC
GTCAACCCTGTGGCCCAGATGGTCACCCTC
                                             270
AGATCATCTTCTCGAACCCCGAGTGACAAG
AGATCAGCTTCTCGGGCCCTGAGTGACAAG
                                             300
CCTGTAGCCCATGTTGTAGCAAACCCTCAA
CCTCTAGCCCACGTAGTAGCAAACCCGCAA
                                             330
GCTGAGGGGCAGCTCCAGTGGCTGAACCGC
GTGGAGGGCCAGCTCCAGTGGCTGAGCCAG
```

Upper rows: base sequence encoding human TNF precursor

Lower rows: base sequence endoding rabbit TNF precursor

## Table 5 (continued)

360
```
CGGGCCAATGCCCTCCTGGCCAATGGCGTG
CGTGCGAACGCCCTGCTGGCCAACGGCATG
```
390
```
GAGCTGAGAGATAACCAGCTGGTGGTGCCA
AAGCTCACGGACAACCAGCTGGTGGTGCCG
```
420
```
TCAGAGGGCCTGTACCTCATCTACTCCCAG
GCCGACGGGCTGTACCTCATCTACTCCCAG
```
450
```
GTCCTCTTCAAGGGCCAAGGCTGCCCCTCC
GTTCTCTTCAGCGGTCAAGGCTGCCGCTCC
```
480
```
ACCCATGTGCTCCTCACCCACACCATCAGC
---TACGTGCTCCTCACTCACACTGTCAGC
```
510
```
CGCATCGCCGTCTCCTACCAGACCAAGGTC
CGCTTCGCCGTCTCCTACCCGAACAAGGTC
```
540
```
AACCTCCTCTCTGCCATCAAGAGCCCCTGC
AACCTCCTCTCTGCCATCAAGAGCCCCTGC
```
570
```
CAGAGGGAGACCCCAGAGGGGGGCTGAGGCC
CACCGGGAGACCCCCGAGGAGGCTGAGCCC
```
600
```
AAGCCCTGGTATGAGCCCATCTATCTGGCA
ATGGCCTGGTACGAGCCCATCTACCTGGGC
```
630
```
GGGGTCTTCCAGCTGGAGAAGGGTGACCGA
GGCGTCTTCCAGTTGGAGAAGGGTGACCGG
```
660
```
CTCAGCGCTGAGATCAATCGGCCCGACTAT
CTCAGCACCGAGGTCAACCAGCCTGAGTAC
```
690
```
CTCGACTTTGCCGAGTCTGGGCAGGTCTAC
CTGGACCTTGCCGAGTCCGGGCAGGTCTAC
```
                              ***
```
TTTGGGATCATTGCCCTGTGA
TTTGGGATCATTGCCCTGTGA
```

Regions surrounded by a rectangle are a homologus region.

Mark "---" shows deletion of a codon.
Mark "***" shows a termination codon.

## Table 6

```
                                                            10
Met Ser Thr Glu Ser Met Ile Arg Asp Val
Met Ser Thr Glu Ser Met Ile Arg Asp Val
                                                            20
Glu Leu Ala Glu Glu Ala Leu Pro Lys Lys
Glu Leu Ala Glu Gly Pro Leu Pro Lys Lys
                                                            30
Thr Gly Gly Pro Gln Gly Ser Arg Arg Cys
Ala Gly Gly Pro Gln Gly Ser Lys Arg Cys
                                                            40
Leu Phe Leu Ser Leu Phe Ser Phe Leu Ile
Leu Cys Leu Ser Leu Phe Ser Phe Leu Ieu
                                                            50
Val Ala Gly Ala Thr Thr Leu Phe Cys Leu
Val Ala Gly Ala Thr Thr Leu Phe Cys Leu
                                                            60
Leu His Phe Gly Val Ile Gly Pro Gln Arg
Leu His Phe Arg Val Ile Gly Pro Gln Glu
                                                            70
Glu Glu --- Phe Pro Arg Asp Leu Ser Leu
Glu Glu Gln Ser Pro Asn Asn Leu His Leu
                                                            80
Ile Ser Pro Leu Ala Gln --- --- Ala Val
Val Asn Pro Val Ala Gln Met Val Thr Leu
                                                            90
Arg Ser Ser Ser Arg Thr Pro Ser Asp Lys
Arg Ser Ala Ser Arg Ala Leu Ser Asp Lys
                                                            100
Pro Val Ala His Val Val Ala Asn Pro Gln
Pro Leu Ala His Val Val Ala Asn Pro Gln
                                                            110
Ala Glu Gly Gln Leu Gln Trp Leu Asn Arg
Val Glu Gly Gln Leu Gln Trp Leu Ser Gln
                                                            120
Arg Ala Asn Ala Leu Leu Ala Asn Gly Val
Arg Ala Asn Ala Leu Leu Ala Asn Gly Met
```

Upper rows:   human TNF precursor

Lower rows:   rabbit TNF precursor

## Table 6 (continued)

```
                                           130
Glu Leu Arg Asp Asn Gln Leu Val Val Pro
Lys Leu Thr Asp Asn Gln Leu Val Val Pro
                                           140
Ser Glu Gly Leu Tyr Leu Ile Tyr Ser Gln
Ala Asp Gly Leu Tyr Leu Ile Tyr Ser Gln
                                           150
Val Leu Phe Lys Gly Gln Gly Cys Pro Ser
Val Leu Phe Ser Gly Gln Gly Cys Arg Ser
                                           160
Thr His Val Leu Leu Thr His Thr Ile Ser
--- Tyr Val Leu Leu Thr His Thr Val Ser
                                           170
Arg Ile Ala Val Ser Tyr Gln Thr Lys Val
Arg Phe Ala Val Ser Tyr Pro Asn Lys Val
                                           180
Asn Leu Leu Ser Ala Ile Lys Ser Pro Cys
Asn Leu Leu Ser Ala Ile Lys Ser Pro Cys
                                           190
Gln Arg Glu Thr Pro Glu Gly Ala Glu Ala
His Arg Glu Thr Pro Glu Glu Ala Glu Pro
                                           200
Lys Pro Trp Tyr Glu Pro Ile Tyr Leu Gly
Met Ala Trp Tyr Glu Pro Ile Tyr Leu Gly
                                           210
Gly Val Phe Gln Leu Glu Lys Gly Asp Arg
Gly Val Phe Gln Leu Glu Lys Gly Asp Arg
                                           220
Leu Ser Ala Glu Ile Asn Arg Pro Asp Tyr
Leu Ser Thr Glu Val Asn Gln Pro Glu Tyr
                                           230
Leu Asp Phe Ala Glu Ser Gly Gln Val Tyr
Leu Asp Leu Ala Glu Ser Gly Gln Val Tyr

Phe Gly Ile Ile Ala Leu
Phe Gly Ile Ile Ala Leu
```

Regions surrounded by a rectangle are
a homologus region.

Mark "---" shows deletion of an amino acid.

## Table 7

| Amino acid | Relative molar quantities |
|------------|---------------------------|
| Asp + Asn  | 12.1 |
| Thr        | 5.5 |
| Ser        | 12.4 |
| Glu + Gln  | 20.3 |
| Pro        | 10.3 |
| Gly        | 10.6 |
| Ala        | 13.0 |
| Cys        | 1.5 |
| Val        | 12.1 |
| Met        | <0.1 |
| Ile        | 8.0 |
| Leu        | 17.7 |
| Tyr        | 6.8 |
| Phe        | 3.9 |
| His        | 2.8 |
| Lys        | 6.1 |
| Arg        | 7.5 |
| Trp        | 1.6 |

## Table 8

| Human Cell | Origin | | Ratio of growth inhibition |
|---|---|---|---|
| **Normal cells:** | | | |
| WI-38 | (ATCC CCL 75) | lung diploid | not inhibited |
| MRC-5 | (ATCC CCL 171) | lung diploid | not inhibited |
| IMR-90 | (ATCC CCL 186) | lung diploid | not inhibited |
| **Tumor cells:** | | | |
| G-361 | (ATCC CRL 1424) | melanoma | 75% |
| HT-1376 | (ATCC CRL 1472) | bladder carcinoma | 49% |
| ZR-75-1 | (ATCC CRL 1500) | breast carcinoma | 97% |
| HOS | (ATCC CRL 1543) | osteogenic sarcoma | 47% |
| WiDr | (ATCC CCL 218) | colon adenocarcinoma | 37% |
| MCF7 | (ATCC HTB 22) | breast adenocarcinoma | 69% |
| G-402 | (ATCC CRL 1440) | renal leiomyoblastoma | 98% |
| PANC-1 | (ATCC CRL 1469) | epitheloid carcinoma | 59% |
| HeLa | (ATCC CCL 2) | epitheloid carcinoma | 31% |

EP 0 155 549 B1

## Table 9

```
         10        20        30
         |         |         |
GGGGGGGGGGGGGGGGGCCCTCTGGAGAGAGC

         40        50        60
         |         |         |
GCCATGAGCACTGAGAGTATGATCCGGGAC
    MetSerThrGluSerMetIleArgAsp

         70        80        90
         |         |         |
GTCGAGCTGGCGGAGGGGCCGCTCCCCAAG
ValGluLeuAlaGluGlyProLeuProLys

         100       110       120
         |         |         |
AAGGCAGGGGGGCCCCAGGGCTCCAAGCGC
LysAlaGlyGlyProGlnGlySerLysArg

         130       140       150
         |         |         |
TGCCTCTGCCTCAGCCTCTTCTCTTTCCTG
CysLeuCysLeuSerLeuPheSerPheLeu

         160       170       180
         |         |         |
CTCGTGGCTGGAGCCACCACGCTCTTCTGC
LeuValAlaGlyAlaThrThrLeuPheCys

         190       200       210
         |         |         |
CTGCTGCACTTCAGGGTGATCGGCCCTCAG
LeuLeuHisPheArgValIleGlyProGln

         220       230     . 240
         |         |         |
GAGGAAGAGCAGTCCCCAAACAACCTCCAT
GluGluGluGlnSerProAsnAsnLeuHis

         250       260       270
         |         |         |
CTAGTCAACCCTGTGGCCCAGATGGTCACC
LeuValAsnProValAlaGlnMetValThr

         280       290       300
         |         |         |
CTCAGATCAGCTTCTCGGGCCCTGAGTGAC
LeuArgSerAlaSerArgAlaLeuSerAsp

         310       320       330
         |         |         |
AAGCCTCTAGCCCACGTAGTAGCAAACCCG
LysProLeuAlaHisValValAlaAsnPro

         340       350       360
         |         |         |
CAAGTGGAGGGCCAGCTCCAGTGGCTGAGC
GlnValGluGlyGlnLeuGlnTrpLeuSer

         370       380       390
         |         |         |
CAGCGTGCGAACGCCCTGCTGGCCAACGGC
GlnArgAlaAsnAlaLeuLeuAlaAsnGly
```

- to be continued -

## Table 9 (continued)

```
           400        410        420
ATGAAGCTCACGGACAACCAGCTGGTGGTG
MetLysLeuThrAspAsnGlnLeuValVal

           430        440        450
CCGGCCGACGGGCTGTACCTCATCTACTCC
ProAlaAspGlyLeuTyrLeuIleTyrSer

           460        470        480
CAGGTTCTCTTCAGCGGTCAAGGCTGCCGC
GlnValLeuPheSerGlyGlnGlyCysArg

           490        500        510
TCCTACGTGCTCCTCACTCACACTGTCAGC
SerTyrValLeuLeuThrHisThrValSer

           520        530        540
CGCTTCGCCGTCTCCTACCCGAACAAGGTC
ArgPheAlaValSerTyrProAsnLysVal

           550        560        570
AACCTCCTCTCTGCCATCAAGAGCCCCTGC
AsnLeuLeuSerAlaIleLysSerProCys

           580        590        600
CACCGGGAGACCCCCGAGGAGGCTGAGCCC
HisArgGluThrProGluGluAlaGluPro

           610        620        630
ATGGCCTGGTACGAGCCCATCTACCTGGGC
MetAlaTrpTyrGluProIleTyrLeuGly

           640        650        660
GGCGTCTTCCAGTTGGAGAAGGGTGACCGG
GlyValPheGlnLeuGluLysGlyAspArg

           670        680        690
CTCAGCACCGAGGTCAACCAGCCTGAGTAC
LeuSerThrGluValAsnGlnProGluTyr

           700        710        720
CTGGACCTTGCCGAGTCCGGGCAGGTCTAC
LeuAspLeuAlaGluSerGlyGlnValTyr

           730        740        750
TTTGGGATCATTGCCCTGTGAGGGGACTGA
PheGlyIleIleAlaLeu

           760        770        780
CCACCACTCCTCCCCCTCTCCCACCCCAGC

           790        800
CCCCTCACTCTGGGCGCCCTCAG
```

[VI] The following Examples and Referential Examples illustrate this invention more specifically. It should be understood however that the invention is in no way limited to these examples.

For a better understanding of the following examples, Figures 1 to 5 are attached to the present specification.

Figure 1 shows the restriction endonuclease cleavage sites used for preparing DNA fragments, and the directions and extents of sequencing for determination of the base sequence of the cloned cDNA encoding human TNF polypeptide [Example 1-(9)];

Figure 2 shows a process of constructing an expression plasmid pHTT26 [Example 2-(1)];

Figure 3 shows a process of constructing an expression plasmid pHTR91 [Example 2-12)];

Figure 4 shows a process of constructing an expression plasmid pHTS115 [Example 2-(3)];

Figure 5 shows a process of constructing an expression plasmid pHTS37 [Example 2-(4)].

Example 1

(1) Preparation of TNF mRNA from Human Alveolar Macrophages

Human alveolar macrophages were collected by broncho-alveolar lavage with phosphate buffered saline. The alveolar macrophages, $6.3 \times 10^7$ cells, were suspended in RPMI-1640 medium containing 10% fetal bovine serum, and seeded in Petri dishes (8 cm in diameter) at a cell density of $9 \times 10^6$ cells per dish. They were pre-cultivated at 37°C in a fully humidified atmosphere containing 5% carbon dioxide. After 1 hour cultivation, endotoxin (lipopolysaccharide derived from E. coli ), TPA (phorbol-12-myristate-13-acetate) and cycloheximide (protein synthesis inhibitor) were added to the dishes so that their final concentrations became 10 micrograms ml, 10 ng/ml and 1 microgram ml, respectively. The cultivation was further continued for 4 to 4.5 hours (total of 5 to 5.5 hours). The culture medium was removed by suction, and the macrophages adhered to the dishes were lysed and homogenized in a 5M guanidyl thiocyanate solution containing 0.6% sodium N-lauroyl sarcosinate and 6mM sodium citrate. The homogenate was loaded on a 5.7M cesium chloride solution containing 0.1M EDTA, and centifuged for 20 hours at 26,500 rpm using an ultracentrifuge (RPS27-2 rotor, Hitachi Koki) to obtain a total RNA fraction as pellets. The pellets were dissolved in a small amount of 7M urea solution containing 0.35M NaCl, 20mM Tris-HCl (pH 7.4) and 20mM EDTA, and recovered by precipitation from ethanol. One hundred and fifty-nine micrograms of total RNA was obtained.

The total RNA fraction was dissolved in 1 ml of 10mM Tris-HCl (pH 7.4) buffer containing 1mM EDTA (to be referred to as TE solution), and the solution was heated at 65°C for 5 minutes. A NaCl solution was added to a final concentration of 0.5M, and the solution was applied onto a column of oligo(dT)-cellulose previously equilibrated with the TE solution containing 0.5M NaCl. Poly(A)mRNA was eluted from the column with the TE solution in an yield of 8 micrograms.

The poly(A)mRNA was dissolved to a concentration of 1.9 ng nl in distilled water, and the solution was injected into the oocytes of Xenopus laevis at a dose of about 50 nl per oocyte by a microinjection method. Ten oocytes were incubated in 100 microliters of the Barth's medium [J. B. Gurdon, J. Embryol, Exp. Morphol., 20 , 401 (1968)] at 22°C for 24 hours. The oocytes were homogenized, and centrifuged at 10.000 rpm for 10 minutes. The supernatant was subjected to assay of TNF activity by determining the cytotoxic activity against mouse L-929 cells.

The method of measuring the cytotoxic activity against L-929 cells was as follows:

A sample (0.1 ml) diluted serially with the below-mentioned a medium and 0.1 ml of a suspension of L-929 cells ($5 \times 10^5$ cells ml) containing actinomycin D (2 micrograms ml) were added into each well of a 96 well multi-well plate (Flow Labs.). The Eagle's minimum essential medium containing 1% fetal bovine serum was used. The plate was incubated at 38.5°C for 18 hours in a fully humidified atmosphere containing 5% carbon dioxide.

Procedures for determining the number of viable L-929 cells and estimating the biological activity were the same as those of the cytotoxic activity assay using mouse L-M cells as a target cell as mentioned in section [III-2-(1)].

The cytotoxic activity against L-929 cells determined under the above conditions was represented by "units (L-929)" to distinguish from the cytotoxic activity against mouse L-M cells.

The supernatant prepared as above had a cytotoxic activity of 6.6 units (L-929) ml. It indicates that the poly(A)mRNA preparation contains TNF mRNA.

(2) Synthesis of cDNA

Complementary DNA was synthesized according to the method of Gubler and Hoffman [(Gene, 25 , 263 (1983)] using the poly(A)mRNA obtained in section (1) as a template.

Six micrograms of the poly(A)mRNA was dissolved in 40 microliters of 50 mM Tris-HCl (pH 8.3) buffer containing 10mM $MgCl_2$, 10mM dithiothreitol, 4mM sodium pyrophosphate, 1.25mM of each of the three deoxyribonucleotide triphosphates, dGTP, dATP and dTTP, 0.5mM dCTP, 167nM alpha-$^{32}$P-dCTP (specific radioactivity, 3,000 Ci/mmole), 4 micrograms of oligo(dT)$_{12-18}$ and 120 units of reverse tran- scriptase derived from avian myeloblastosis virus (AMV), and incubated at 43°C for 30 minutes. Then, the reaction was stopped by adding EDTA. The reaction mixture was extracted with phenol/chloroform (1:1), and ammonium acetate was added to the aqueous phase to a final concentration of 2.5M. The resulting cDNA-mRNA hybrid was recovered from the aqueous phase by precipitation from ethanol. The cDNA-mRNA hybrid precipitate was dissolved in 100 microliters of 20mM Tris-HCl (pH 7.5) buffer containing 5mM $MgCl_2$, 10mM $(NH_4)_2SO_4$, 100mM KCl, 0.15mM betanicotinamide-adenine dinucleotide, 5 micrograms of bovine serum albumin, 0.04mM of each of four deoxyribonucleotide triphosphates, dGTP, dATP, dTTP and dCTP, 0.9 unit of E. coli ribonuclease H and 23 units of E. coli DNA polymerase I, and incubated at 12°C for 60 minutes and further at 22°C for 60 minutes to synthesize a dscDNA. The reaction was stopped by adding EDTA. The dscDNA was extracted with phenol/chloroform, and recovered by precipitation from ethanol as shown above.

(3) Preparation of oligo(dC)-tailed cDNA

The dscDNA obtained as above was dissolved in 100 microliters of 100mM sodium cacodylate (pH 7.2) buffer containing 2mM $CoCl_2$, 0.2mM dithiothreitol, 0.1mM alpha-$^{32}$P-dCTP (specific radioactivity, 3 Ci mmole) and 10 units of terminal deoxynucleotidyl transferase, and incubated at 37°C for 30 minutes to permit the addition of oligo(dC) tails to the 3'-termini of dscDNA.

The reaction was stopped by adding EDTA. The oligo(dC)-tailed dscDNA was extracted with phenol chloroform, and recovered by precipitation from ethanol. The oligo(dC)-tailed dscDNA was dissolved in 10mM Tris-HCl (pH 7.4) buffer containing 1mM EDTA and 100mM NaCl so that it contained 2 micrograms of the oligo(dC)-tailed dscDNA per ml.

(4) Preparation of oligo(dG)-tailed pBR322 DNA

Ten micrograms of pBR322 DNA was dissolved in 100 microliters of 20mM Tris-HCl (pH 7.4) buffer containing 10mM $MgCl_2$, 50mM $(NH_4)_2SO_4$ and 10 micrograms of bovine serum albumin, and 15 units of the restriction endonuclease Pst I was added. The mixture was incubated at 37°C for 1 hour. After the reaction was terminated, the reaction mixture was extracted with phenol chloroform, and the resulting DNA was recovered from the aqueous phase by precipitation from ethanol. The DNA obtained was dissolved in 200 microliters of the same reaction buffer as used for tailing of the dscDNA above (except that it contained 80 units of terminal deoxynucleotidyl transferase and $^3$H-dGTP instead of $^{32}$P-dCTP) and incubated at 37°C for 20 minutes to add about 10-15 deoxygnanylic acid (dG) residues to the 3'-termini. The reaction mixture was extracted with phenol chloroform, and the oligo(dG)-tailed pBR322 DNA was recovered from the aqueous phase by ethanol precipitation. The resulting tailed pBR322 DNA was dissolved in the same buffer as used for dissolving the oligo(dC)-tailed dscDNA so that it contained the tailed pBR322 DNA at a concentration of 20 micrograms per ml.

(5) Construction of recombinant plasmids

One hundred and twenty microliters of the oligo(dC)-tailed cDNA solution was mixed with an equal volume of the oligo(dG)-tailed pBR322 DNA solution, and the mixture was incubated sequentially at 65°C for 5 minutes and at 57°C for 120 minutes to perform annealing and to construct recombinant plasmids.

(6) Selection of transformants

E. coli x1776 strain was transformed with the recombinant plasmids obtained as above.

Specifically, E. coli x1776 was cultivated at 37°C in 20 ml of L broth (composition: 10 g of trypton, 5 g of yeast extract, 5 g of NaCl and 1 g of glucose per liter; pH 7.2) supplemented with 100 micrograms/ml of diaminopimelic acid and 40 micrograms/ml of thymidine until the turbidity at 600 nm reached 0.5. The cells were collected by centrifugation at 4°C, and washed with 10 ml of 10mM Tris-HCl (pH 7.3) buffer containing 50 mM CaCl₂. The cells were resuspended in 2 ml of the same buffer as used above, and left to stand at 0°C for 5 minutes. To 0.2 ml of the suspension was added 0.1 ml of the recombinant plasmids solution obtained as above. The mixture was left to stand at 0°C for 15 minutes and then maintained at 42°C for 2 minutes. Then, 0.5 ml of the supplemented L broth as used above was added, and cultivation was carried out with shaking for 1 hour. An aliquot of the culture was taken, spread on the supplemented L broth agar plate containing 15 micrograms/ml of tetracycline, and cultivated at 37°C for about 12 hours. A cDNA library was prepared by selecting transformants resistant to tetracycline.

(7) Cloning of human TNF cDNA

Transformants harboring the recombinant plasmids containing cDNAs encoding human TNF polypeptide were selected from the cDNA library obtained in section (6), by a colony hybridization assay using DNA fragments prepared from the cloned cDNA encoding rabbit TNF as probes.

Specifically, the cDNA encoding rabbit TNF was isolated from the recombinant plasmid pRTNF802 as shown in Referential Example 1. Its base sequence is shown in Table 3. The cDNA was digested with the restriction endonuclease Ava I or Hae II. The digested DNA fragments were recovered by precipitation from ethanol. They were subjected to polyacrylamide gel electrophoresis to isolate desired DNA fragments.

The DNA fragment (299 bp) corresponding to the 285th to the 583rd bases as shown in Table 3 was obtained by digestion with the restriction endonuclease Ava I (to be referred to as Ava I fragment). Another DNA fragment (88 bp) corresponding to the 33rd to the 120th bases as shown in Table 3 was obtained by digestion with the restriction endonuclease Hae II (to be referred to as Hae II fragment). The Ava I fragment and Hae II fragment were labelled with ³²P. These labelled DNA fragments were used as a probe for screening the cDNA library to select transformants having a plasmid containing cDNA encoding human TNF polypeptide by the colony hybridization assay according to the method of Hanahan and Meselson, 10 . 63 (1980)].

Out of about 20.000 clones. 43 clones were selected by the 1st screening using the ³²P-labelled Ava I fragment as a probe. Furthermore. these 43 selected clones were subjected to 2nd screening using the ³²P-labelled Hae II fragment as a probe.

Finally, 6 clones harboring the recombinant plasmids having cDNAs which strongly hybridized with both the rabbit TNF cDNA fragments were selected by these assays.

(8) Expression

Recombinant plasmid DNAs were isolated by the method of Wilkie et al. [Nucleic Acid Res., 7 . 859 (1979)] from the six transformants selected in section (7); named plasmid No. pHTNF1. pHTNF4. pHTNF5. pHTNF13. pHTNF22 and pHTNF26. respectively. Each of the recombinant plasmids was introduced into E. coli HB101 according to the same method as shown in section (6) to prepare transformants harboring the recombinant plasmids.

The transformants were cultivated in 50 ml of LB broth [composition: 10 g of trypton. 5 g of yeast extract and 10 g of NaCl per liter; pH 7.5] until the turbidity at 600 nm of the culture reached about 0.8. Then, about 3 to 5 x 10¹⁰ cells were collected. The cells were lysed by the method of Nagata et al. [Nature. 284. 316 (1980)] with slight modifications. The cells were resuspended in 1 ml of 50mM Tris-HCl (pH 8.0) buffer containing 0.1% lysozyme and 30mM NaCl. After standing for 30 minutes in ice water. the cells were lysed by repeating freezing-thawing 6 times. The cell debris was removed by centrifugation to give a clarified lysate. The lysate obtained from each transformant was subjected to assay of cytotoxic activity against L-929 cells.

It was consequently found that the lysate obtained from the transformant harboring plasmid pHTNF13 had a cytotoxic activity of 186.1 units (L-929) ml.

(9) Determination of the base sequence of the cloned cDNA

The recombinant plasmid pHTNF13 was isolated as above. The plasmid DNA was cleaved with the restriction endonuclease Pst I to isolate a cloned cDNA inserted into a vector. The cloned cDNA fragment was further cleaved with various restriction endonucleases, and the base sequences of the resulting 16 fragments were determined by the Maxam-Gilert method.

Figure 1 shows the restriction endonuclease cleavage sites used for preparing the fragments, and the directions of sequencing indicated by arrows. The rectangular area is a coding region of human TNF precursor polypeptide. Table 4 shows the base sequence determined and the amino acid sequence deduced from the base sequence. The region encoding human TNF polypeptide was determined on the basis of homology to the base sequence encoding rabbit TNF.

The DNA encoding human TNF polypeptide codes for its precursor polypeptide consisting of 233 amino acid residues. The mature human TNF polypeptide is a polypeptide corresponding to the 155 amino acid residues from the carboxy-terminus of its precursor, which is coded in the base sequence from base No. 235 to base No. 699 in Table 4 (the region bracketed in Table 4). A termination codon followed by the last codon encoding human TNF polypeptide is an opal codon TGA.

(10) Immunological property of human TNF polypeptide The immunological cross-reactivity of human TNF polypeptide in the lysate obtained above with anti-rabbit plasma TNF antibody was tested as follows:

The lysate was mixed with an equal volume of a 100-fold dilution of the purified anti-rabbit plasma TNF antibody obtained in Referential Example 4. After incubating at 37°C for 2 hours, the cytotoxic activity of the reaction mixture was measured by the method described above using L-929 cells as a target cell. Rabbit plasma TNF obtained in Referential Example 2 was previously diluted with phosphate buffered saline and the dilution was used as a control TNF preparation. As the result below shows, the cytotoxic activity of human TNF polypeptide was not neutralized with the antibody.

| Sample | Anti-rabbit plasma TNF antibody | Cytotoxic activity* units (L-929)/ml |
|---|---|---|
| Lysate from the transformant harboring plasmid pHTNF13 | not added | 186.1 |
| | added | 191.0 |
| Rabbit plasma TNF | not added | 572.3 |
| | added | <0.1 |

**\* Cytotoxic activity was shown as the activity in the original sample solution used for the test.**

Example 2

Production of human TNF polypeptide in Escherichia coli

(1) Expression under the control of tac promoter

The cloned cDNA was isolated from the recombinant plasmid pHTNF13 as mentioned in Example 1. The cDNA was further digested with the restriction endonuclease Eco RI to split off a part of the non-coding

36

region downstream of the TNF coding region. The resulting DNA fragment (about 1.1 kbp) was inserted into a larger DNA fragment prepared from plasmid pBR322 by digestion with the restriction endonucleases Pst I and Eco RI to construct a recombinant plasmid including TNF cDNA and a tetracycline-resistance gene, which was named pHT113.

An expression plasmid pHTT26 carrying a cDNA encoding the mature human TNF polypeptide was constructed by the procedure illustrarted in Fig. 2.

The cDNA isolated from pHT113 was digested with the restriction endonucleases Ava I and Hin dIII and the resulting DNA fragment (578 bp) including most of the coding region for the mature human TNF polypeptide (to be referred to as HTNF fragment) was isolated by polyacrylamide gel electrophoresis.

A DNA fragment including a tac promoter region was isolated as follows: Plasmid DNA (300 micrograms) of pDR540 [P-L Biochemicals; Russell, D.R., et al., Gene, 20 , 231 (1982)] was dissolved in 2 ml of 10mM Tris-HCl (pH 7.5) buffer containing 50mM NaCl, 6mM $MgCl_2$ and 6mM 2-mercaptoethanol and digested with the restriction endonucleases Eco RI and Bam HI by incubating at 37°C for 60 minutes. After adding NaCl to give a final concentration of 0.3M, the digested DNA fragments were recovered from ethanol. The DNA fragment including a tac promoter region was isolated by polyacrylamide gel electrophoresis in an yield of 8.3 micrograms.

The tac promoter fragment was ligated with a chemically synthesized oligodeoxyribonucleotide adaptor represented by the following formula:

$$5'\text{-GATCCATGTCATCTTCTCGAACC}$$
$$3'\text{-GTACAGTAGAAGAGCTTGGGGCT}$$

This adaptor includes an initiation codon ATG and a base sequence corresponding to the 5'-terminal portion of the base sequence encoding the mature human TNF polypeptide, and has the Bam HI and Ava I cohesive termini. The resulting DNA fragment is referred to as tac promoter-adaptor fragment.

Separately, a larger DNA fragment (about 4.3 kbp) including an ampicillin-resistance gene (to be referred to as pBR322-Amp$^r$ fragment) was cut out from plasmid pBR322 by digestion with the restriction endonucleases Hin dIII and Eco RI, and isolated by gel electrophoresis using a low melting agarose (0.7%).

One microgram of the HTNF fragment and 6 micrograms of the pB322-Amp$^r$ fragment were dissolved in 66mM Tris-HCl (pH 7.6) buffer containing 6.6mM $MgCl_2$ and incubated at 55°C for 10 minutes. Then ATP and dithiothreitol were added to 1mM and 10mM respectively, and 168 units of $T_4$ DNA ligase was added and further the mixture was incubated at 22°C for 120 minutes. The resulting DNA fragment was recovered by extraction with phenol in an yield of about 4 micrograms. The DNA fragment (0.8 microgram) was ligated with the tac promoter-adaptor fragment (0.3 microgram) under the same conditions as above except for using 63 units of $T_4$ DNA ligase. The reaction mixture was diluted to 6 fold with distilled water and mixed with an equal volume of the suspension of E. coli JM103 cells (P-L Biochemicals) treated with calcium as shown below. The mixture was sequentially incubated in ice water for 20 minutes, at 42°C for 1 minute and at room temperature for 10 minutes, and LB broth was added. The mixture was shaken at 37°C for 60 minutes. An aliquot of the resulting cell suspension was spread on LB agar plates containing 25 micrograms ml of ampicillin, and cultivated overnight at 37°C. The ampicillin-resistance colonies were selected.

One of the transformants capable of producing human TNF polypeptide was named JM103 pHTT26.

The calcium-treated E. coli JM103 cells were prepared as follows: E. coli JM103 cells were inoculated in 5 ml of L broth and cultivated overnight at 37°C. One ml of the resulting culture was inoculated in 100 ml of LB broth and further cultivated at 37°C until the turbidity at 650 nm of the culture reached 0.6. After standing for 30 minutes in ice water, the cells were collected by centrifugation and suspended in 50 ml of 50mM $CaCl_2$, followed by standing at 0°C for 60 minutes. The cells were collected by centrifugation and again suspended in 10 ml of 50mM $CaCl_2$ containing 20% glycerol, which was used as the calcium-treated E. coli JM103 cells.

The transformant JM103 pHTT26 was cultivated overnight in LB broth at 37°C. The culture (0.5 ml) was inoculated in 5 ml of fresh LB broth and further cultivated at 37°C for 1 hour. Then, isopropyl beta-D-thiogalactoside was added to a final concentration of 1mM, and the cultivation was continued further for 4 hours. The cells were collected and suspended in 1ml of 50mM Tris-HCl (pH 8.0) buffer containing 0.1% lysozyme and 30mM NaCl and left to stand at 0°C for 30 minutes. Then freezing on a dry ice ethanol bath and thawing at 37°C were repeated 6 times, and the cell debris was removed by centrifugation to give a clarified lysate.

The lysate had a cytotoxic activity of 9.9 x 10⁴ units (L-929)/ml. This cytotoxic activity was not neutralized with an antibody to rabbit plasma TNF as shown in Referential Example 4. It was confirmed that human TNF polypeptide did not cross-react with rabbit plasma TNF immunologically.

(2) Expression under the control of trp promoter An expression plasmid pHTR91 was constructed by the procedure illustrated in Fig. 3.

The recombinant plasmid pHT113 was digested with the restriction endonucleases Ava I and Sal I to be cut into 3 fragments (about 0.8 kbp, 1.3 kbp and 2.6 kbp in size). The 1.3 kbp-DNA fragment including most of the coding region for the mature human TNF polypeptide and a part of tetracycline-resistance gene was isolated (to be referred to as Ava I-Sal I fragment). The Ava I-Sal I fragment was ligated with the following chemically synthesized oligodeoxyribonucleotide adaptor. This adaptor was referred to as adaptor I.

**5-CGATATGTCATCTTCTCGAACC**
**3'-TATACAGTAGAAGAGCTTGGGGCT**

The resulting DNA fragment was referred to as HTNF-adaptor fragment.

Separately, the DNA fragment (35 bp) including a part of trp promoter region was cut out from a plasmid pDR720 [P-L Biochemicals; Russell, D.R., et al., Gene, 20 , 231 (1982)] by digesting with the restriction endonucleases Eco RI and Hpa I, and the DNA fragment was ligated with a chemically synthesized adaptor represented by the following formula:

**5'-AACTAGTACGCAAGTTCACGTAAAAAGGGTAAT**
**3'-TTGATCATGCGTTCAAGTGCATTTTTCCCATTAGC**

The ligated DNA fragment was referred to as trp promoter fragment.

Plasmid pBR322 was digested with the restriction endonucleases Eco RI and Sal I, and a larger DNA fragment (about 3.7 kbp) was isolated. By sequential ligation of these three DNA fragments, the HTNF-adaptor fragment, the trp promoter fragment and the larger pBR322 fragment, an expression plasmid pHTR91 was constructed. The expression plasmid was introduced into E. coli HB101 cells by the method described in section (1), and one of the transformants was named HB101 pHTR91.

The transformant HB101 pXTR91 was cultivated overnight at 30°C in the modified M-9 medium (composition: 0.7% Na₂HPO₄ 12H₂O, 0.3% KH₂PO₄, 0.05% NaCl, 0.1% NH₄Cl, 2 mg t of vitamine B·, 0.45% casamino acid, 1mM MgSO₄, 0.1mM CaCl₂ and 0.5% glucose). The culture (0.05 ml) was inoculated in 5 ml of the same medium, and cultivated at 37°C for 1 hour. Then, 3-beta-indoleacrylic acid was added to give a final concentration of 20 micrograms ml, and cultivation was continued for 4 hours. The cells were collected and treated by the same procedure as described in section (1) to obtain a clarified lysate.

The lysate had a cytotoxic activity of 1.01 x 10⁶ units (L-929) ml.

(3) Expression under the control of phos promoter

An expression plasmid pHTS115 capable of producing human TNF polypeptide fused with the signal peptide and the N-terminal portion of a phosphate binding protein, and secreting the fused protein into the periplasms of host cells was constructed by the procedure illustrated in Fig. 4.

The Ava I-Sal I fragment obtained in section (2) was ligated with a chemically synthesized oligodeoxyribonucleotide adaptor having the following sequence:

**5'-GATCCATGTCATCTTCTCGAACC**
**3'-GTACAGTAGAAGAGCTTGGGGCT**

The ligated DNA fragment was digested with the restriction endonuclease Bam HI to prepare a DNA fragment having Bam HI cohesive termini at both ends (to be referred to as HTNF-Tet'● Bam HI fragment).

Separately, plasmid pSN5182 [Morita, T., et al., Eur. J. Biochem., 130, 427 (1983)] containing a phos gene was digested with the restriction endonucleases Hpa I and Eco RI to prepare the DNA fragment (about 4 kbp) including the phos promoter region and Shine-Dalgarno sequence, the DNA sequence coding for the signal peptide and the N-terminal portion of a phosphate binding protein, and the tetracycline-resistance gene. This DNA fragment was referred to as Hpa I-Eco RI fragment. The Hpa I-Eco RI fragment was ligated with a chemically synthesized oligodeoxyribonucleotide linker having the following sequence:

$$5'-CCCGGATCCGGG$$
$$3'-GGGCCTAGGCCC$$

Then, the ligated DNA fragment was digested with the restriction endonuclease Bam HI. The resulting DNA fragment (about 3.6 kbp) having Bam HI cohesive termini at the both ends was referred to as phos promoter-Bam HI·Tet$^r$ fragment.

The HTNF-Tet $^r$·Bam HI fragment was ligated with the phos promoter-Bam HI·Tet$^r$ fragment to construct an expression plasmid for producing the human TNF polypeptide fused with a phosphate binding protein, which was named pHTS115. The expression plasmid was introduced into E. coli HB101 according to the method as shown above.

One of the transformants (HB101/pHTS115) was cultivated in 5 ml of TG medium [composition: 120mM Tris-HCl (pH 7.4) buffer containing 0.2% glucose, 80mM NaCl, 20mM KCl, 20mM NH₄Cl, 3mM Na₂SO₄, 1mM MgCl₂, 0.2mM CaCl₂, 200nM FeCl₃, 20 mg/ℓ of leucine, 20 mg/ℓ of proline and 10 mg/ℓ of vitamine B₁], supplemented with 0.64mM KH₂PO₄, at 37°C for 20 hours with shaking. The cells were collected by centrifugation, resuspended in 2 ml of TG medium supplemented with 0.064mM KH₂PO₄ and cultivated at 37°C for 6 hours. The cells were collected by centrifugation, and washed with 1 ml of 10mM Tris-HCl (pH 7.2) buffer containing 30mM NaCl. Then they were resuspended in 0.2 ml of 33mM Tris-HCl (pH 7.2) buffer and mixed with an equal volume of 33mM Tris-HCl (pH 7.2) buffer containing 0.1mM EDTA and 40% sucrose. After incubation at 37°C for 10 minutes, the cells were collected and resuspended in 0.4 ml of chilled 0.5mM MgCl₂ and left to stand in ice water for 10 minutes with occasional shaking. The cell debris was removed by centrifugation to obtain a clarified extract (to be referred to as periplasmic extract).

The periplasmic extract has a cytotoxic activity of $1.34 \times 10^5$ units (L-929) ml.

The molecular weight of the polypeptide having cytotoxic activity was determined to be 19.000 daltons by sodium dodecylsulfate (SDS)-polyacrylamide gel (12.5%) electrophoresis. which suggests that the polypeptide produced was a fused protein.

(4) Expression under the control of phos promoter

An expression plasmid pHTS37 capable of producing human TNF polypeptide fused with the signal peptide and the N-terminal portion of a phosphate binding protein and the mature human TNF polypeptide with a part of its precursor portion. and secreting the fused protein into the periplasm of host cells was constructed by the procedure illustrated in Fig. 5.

Recombinant plasmid (pHTNF13) DNA was digested with the restriction endonuclease Pst I to cut out the DNA fragment (about 1.2 kbp) including human TNF cDNA. The DNA fragment was further digested with the restriction endonuclease Bbe I to obtain a DNA fragment (about 0.9 kbp; to be referred to as Bbe I-Pst I fragment). Six micrograms of the Bbe I-Pst I fragment was dissolved in 80 microliters of 20mM Tris-HCl (pH 8.0) buffer containing 12mM CaCl₂. 12mM MgCl₂. 0.2M NaCl. 1mM EDTA and 0.02 unit of the exonuclease Bal 31. and incubated at 30°C for 30 minutes to chew away about 50 to 150 base pairs from the both ends of the DNA fragment. Then the termini of the fragment were repaired by incubating at 37°C for 60 minutes with 10 units of E. coli DNA polymerase I (large fragment) in the presence of 0.1mM of each of four deoxyribonucleotide triphosphates. dGTP. dATP. dCTP and dTTP. and 50 micrograms ml of bovine serum albumin. The repaired DNA fragment was digested with the restriction endonuclease Eco RI and resulting DNA fragment (about 750 bp) having a blunt end and a Eco RI cohesive end was isolated in a yield of about 3 micrograms (to be referred to as Bal 31-Eco RI fragment).

The Bal 31-Eco RI fragment was ligated with the Hpa I-Eco RI fragment (about 4 kbp) obtained from pSN5182 as described in section (3) to construct an expression plasmid containing the phos promoter region, Shine-Dalgarno sequence, the DNA sequence coding for the signal peptide and the N-terminal portion of a phosphate binding protein and the DNA sequence coding for the mature human TNF polypeptide and a part of its precursor polypeptide.

The expression plasmid was named pHTS37 and it was introduced into E. coli HB101 to obtain a transformant which was named HB10l/pHTS37. The transformant HB101/ pHTS37 was cultivated and the periplasmic extract was obtained under the same conditions as shown in section (3).

The periplasmic extract obtained had a cytotoxic activity of $4.93 \times 10^5$ units (L-929)/ml.

The extract was subjected to SDS-polyacrylamide gel (12.5%) electrophoresis. The electrophoresis was carried out at 35 V for 12 hours using Tris-glycine (pH 8.3) buffer containing 0.1% SDS. Protein was stained with Coomassie brilliant blue. Two protein bands, which were not observed in the extract of E. coli HB101, were detected at the positions corresponding to molecular weights of about 22,000 daltons and 16,500 daltons. Separately, the gel was sliced into 2 mm width and each gel was shaken in 1 ml of Eagle's minimum essential medium containing 1% fetal bovine serum at 4° C overnight to elute proteins from the gel. Each eluate was used for determining the cytotoxic activity against mouse L-929 cells. As a result, a strong cytotoxic activity was detected in the eluate from the gel sliced from the position corresponding to the protein stained with Coomassie brilliant blue, which was determined to have a molecular weight of 16,500 daltons.

It was supposed from the structure of the expression plasmid pHTS37 that human TNF polypeptide produced in the transformant should be a fused protein of a part of phosphate binding protein and human TNF polypeptide with a part of its precursor portion. The theoretical molecular weight of the fused protein was calculated as about 23,000 daltons. On the other hand, the molecular weight of the mature human TNF polypeptide is 17,097 daltons. This investigation suggested that the fused protein produced could be converted to the mature human TNT polypeptide in the host cells by limited hydrolysis probably at a specific site (Arg-Ser) joining the mature human TNF polypeptide to its precursor portion.

When the periplasmic extract was incubated with 5 micrograms ml of trypsin (from bovine pancreas, Sigma Chemical Co.) at 37° C for 1 hour and the reaction mixture was subjected to SDS-polyacrylamide gel electrophoresis under the same conditions as above, a protein band detected at the position corresponding to a molecular weight of about 22,000 daltons, which might be the fused protein, disappeared by the trypsin digestion.

Example 3

Production of modified human TNF polypeptide

An expression plasmid pHTRD4 carrying a DNA encodng a polypeptide resulting from deletion of four amino acids from the N-terminus of mature human TNF polypeptide was constructed according to the same strategy as that of the expression plasmid pHTR91 shown in Example 2-(2), except that the following synthetic adaptor was used instead of the adaptor l:

**5'-CGATATGACC**
**3'-TATACTGGGGCT**

By using the above synthetic adaptor, the base sequence followed by an initiation codon (ATG) is coding for an amino acid sequence consisting of 151 amino acid residues which results from deletion of 4 amino acids (Ser-Ser-Ser-Arg) from the N-terminal of the mature human TNF polypeptide.

The expression plasmid pHTRD4 constructed by the above procedure was introduced into E. coli HB101, and the transformant was cultivated overnight at 37° C in the modified M-9 medium. The culture (0.05 ml) was inoculated in 5 ml of the same medium, and cultivated at 37° C for 1 hour. Then, 3-beta-indoleacrylic acid was added to give a final concentration of 20 micrograms ml and the cultivation was continued overnight. The cells were collected by centrifugation and treated by the same methods as described in Example 2-(1) to obtain a clarified lysate.

The lysate had a cytotoxic activity of $1.1 \times 10^5$ units (L-929) ml.

Example 4

Production of human TNF precursor polypeptide in Escherichia coli

An expression plasmid pHTRP3 carrying a cDNA encoding a human TNF precursor polypeptide consisting of 233 amino acids was constructed as follows: The cloned cDNA was isolated from the recombinant plasmid pHT113 by double digestion with the restriction endonucleases Pst I and Hin dIII and the cDNA fragment was further digested partially with the restriction endonuclease Hgi AI to obtain the DNA fragment (about 820 bp) including the base seqnence encoding most of the precursor polypeptide. The resulting DNA fragment was ligated with a chemically synthesized oligodeoxyribonucleotide fragment represented by the following formula:

**5'-CGATATGAGCA**
**3'-TATAC**

The ligated DNA fragment was referred to as Pre-TNF fragment.

Separately, the DNA fragment containing a part of trp promoter region was cut out from plasmid pDR720 by digesting with the restriction endonucleases Eco RI and Hpa I, and the DNA fragment was ligated with the following chemically synthesized adaptor.

**5'-AACTAGTACGCAAGTTCACGTAAAAAGGGTAAT**
**3'-TTGATCATGCGTTCAAGTGCATTTTTCCCATTAGC**

The resulting DNA fragment was ligated with the Pre-TNF fragment and the ligated DNA fragment was combined with the DNA fragment (about 4.3 kbp) having an ampicillin-resistance gene isolated from plasmid pBR322 by digesting with the restriction endonucleases Eco RI and Hin dIII.

The expression plasmid pHTRP3 constructed by the above procedure was introduced into E. coli HB101, and the transformant was cultivated overnight at 37°C in the modified M-9 medium. The culture (0.05 ml) was inoculated in 5 ml of the same medium, and cultivated at 37°C for 1 hour. Then, 3-beta-indoleacrylic acid was added to give a final concentration of 20 micrograms/ml, and the cultivation was continued overnight. The cells were collected by centrifugation and treated by the same methods as described in Example 2-(1) to obtain a clarified lysate.

The lysate had a cytotoxic activity of $1.8 \times 10^4$ units (L-929)/ml.

Example 5

Production and purification of human TNF polypeptide

(1) Production in Escherichia coli

The transformant HB101 pHTR91 obtained in Example 2-(2) was used for the production of human TNF polypeptide. The transformant was cultivated overnight at 37°C in LB broth supplemented with 12.5 micrograms/ml of tetracycline. The culture was inoculated into 10 volumes of the modified M-9 medium used in Example 2-(2), and cultivated at 37°C for 1 hour. After adding 3-beta-indoleacrylic acid to give a final concentration of 20 micrograms/ml, the cultivation was continued for 4 hours. The cells were collected and treated to obtain a clarified lysate by the same method as shown in Example 2-(1). The lysate was used for the purification of human TNF polypeptide.

(2) Purification of human TNF polypeptide

Human TNF polypeptide was purified from the lysate obtained in section (1) by the following procedure. To a column (5 x 20 cm) packed with DEAE-Sepharose CL-6B (Pharmacia) previously equilibrated with 20mM Tris-HCl (pH 7.8) buffer, 1030 ml of the lysate was applied. The column was washed with 3 liters of the same buffer; and then eluted with a linear gradient of NaCl from zero to 0.3M in the same buffer at a flow rate of 133 ml/hour. The eluate was fractionated into 10 ml-fractions. The fractions having cytotoxic activity were collected and pooled.

A recovery of a cytotoxic activity was 53% in this step, and a specific activity was increased up to about 9 times.

The pooled active fractions were desalted and concentrated to one-tenth volume by ultrafiltration with Diaflo using a YM10 membrane (Amicon).

The concentrate was subjected to preparative isoelectrofocusing gel electrophoresis. An aliquot of the concentrate was loaded onto a polyacrylamide gel plate (0.2 x 10 x 10 cm) with a pH gradient ranging from pH 5.6 to pH 6.1 created with Immobiline (LKB). The electrophoresis was carried out at 2,400 V for 16 hours at 15°C. The gel was sliced into 10-mm width and the protein was eluted with 20mM Tris-HCl (pH 7.8) buffer. The above procedure was repeated. The eluates having cytotoxic activity were pooled and concentrated by ultrafiltration as above. Finally, the concentrate was applied onto a column (0.7 x 25 cm) of Bio-Gel P-6 (BIO-RAD) and desalted.

The desalted final preparation having cytotoxic activity was homogeneous on the basis of SDS-polyacrylamide gel electrophoretic analysis, and it was used as a purified human TNF polypeptide for analyses as mentioned in section [III].

Example 6

Process for preparation of lyophilized human TNF polypeptide

The purified human TNF polypeptide solution obtained by the method described in Example 5 was used for preparation of a lyophilized human TNF polypeptide.

Ten milliliters of the purified human TNF polypeptide solution containing a cytotoxic activity of $2.2 \times 10^7$ units (LM) was mixed with one-tenth volume of 8% NaCl containing 10% human serum albumin and 20% D-mannitol. After adjusting pH of the solution to 6.8, the resulting solution was sterilized by filtration through Microflow membrane (Flow Labs., pore size 0.2 micron). Five milliliters of the sterile solution was dispensed into glass vials and then lyophilized. Each vial contains $10^7$ units (LM) of purified human TNF polypeptide.

Referential Example 1

Preparation of Rabbit TNF cDNA

(1) Preparation of TNF mRNA from macrophages of rabbit alveolus

Rabbits (weighing about 2.5 kg) were intravenously injected with killed dried cells of Propionibacterium acnes at a dose of 100 mg per rabbit, and sacrificed 8 days later. The lungs were repeatedly washed with phosphate buffered saline through a tube inserted into the trachea of the animals, and alveolar macrophages were collected. About $3 \times 10^9$ alveolar macrophages were obtained from 12 rabbits.

The alveolar macrophages were suspended in RPMI-1640 medium containing 10% fetal bovine serum, and seeded in Petri dishes (8 cm in diameter) at a cell density of $2 \times 10^7$ cells per dish. They were pre-cultivated at 37°C in a fully humidified atmosphere containing 5% carbon dioxide. After pre-cultivation for one hour, endotoxin (lipopolysaccharide derived from E. coli ), TPA (phorbol-12-myristate-13-acetate) and cycloheximide (protein synthesis inhibitor) were added so that their final concentrations became 10 micrograms ml, 10 ng ml and 1 microgram ml, respectively. The cultivation was further continued for 4 to 4.5 hours (total of 5 to 5.5 hours), the culture medium was removed by suction, and the macrophages adhered to the dishes were lysed and homogenized in a 5M guanidyl thio cyanate solution containing 0.6% sodium N-lauroyl sarcosinate and 6mM sodium citrate. The homogenate was loaded on a 5.7M cesium chloride solution containing 0.1M EDTA, and centrifuged for 20 hours at 26,500 rpm using an ultracentrifuge (RPS27-2 rotor, Hitachi Koki) to obtain a total RNA fraction as pellets. The pellets were dissolved in a small amount of 7M urea solution containing 0.35M NaCl, 20mM Tris-HCl (pH 7.4) and 20mM EDTA and recovered by precipitation from ethanol. From 12 rabbits, 5.2 mg of total RNA were obtained.

The total RNA fraction was dissolved in 2 ml of TE solution used in Example 1-(1), and the solution was heated at 65°C for 5 minutes. A NaCl solution was added to a final concentration of 0.5M, and the solution was applied onto a column of oligo(dT)-cellulose previously equilibrated with the TE solution containing 0.5M NaCl. Poly-(A)mRNA was eluted from the column with the TE solution in an yield of 314 micrograms. Two hundred micrograms of the resulting poly(A)mRNA was subjected to agarose gel electrophoresis (gel concentration 1%, in the presence of 6M urea, pH 4), and fractionated into 7 fractions according to molecular sizes. Poly(A)mRNA was isolated from each gel fractionated by melting at 70°C for 10 minutes followed by successive extraction with phenol and chloroform, and by precipitation from ethanol. The content of rabbit TNF mRNA in poly(A)mRNA recovered from each fraction was determined by a mRNA translation assay using oocytes of Xenopus laevis.

Rabbit TNF mRNA was recovered at a higher concentration from a fraction corresponding to a molecular size of 1.6 to 2.7 kb (to be abbreviated as an enriched rabbit TNF mRNA).

The enriched rabbit TNF mRNA fraction obtained herein was used in the following experiment.

(2) Synthesis of cDNA

cDNA was synthesized under the following conditions. Four micrograms of the enriched TNF mRNA fraction was dissolved in 100 microliters of 50mM Tris-HCl (pH 8.3) buffer containing 10mM $MgCl_2$, 70mM KCl, 1mM dithiothreitol, 0.5mM of each of the four deoxyribonucleotide triphosphates, dTTP, dCTP, dATP and dGTP (dCTP was labelled with $^{32}P$; specific activity, 4.4 x $10^6$ cpm/nmole), 3 micrograms of oligo(dT)-$_{12-18}$ and 80 units of reverse transcriptase derived from avian myeloblastosis virus and incubated at 43°C for 90 minutes. Then the reaction was stopped by adding EDTA. The resulting cDNA-mRNA hybrid was extracted with phenol/chloroform (1:1), and recovered from the aqueous phase by precipitation from ethanol. The mRNA template was removed by treating with an alkali at 65 to 70°C. The synthesized sscDNA was recovered by precipitation from ethanol.

The sscDNA precipitate was dissolved in 40 microliters of a 0.1M Hepes (pH 6.9) buffer containing 0.5mM of each of the four deoxyribonucleotide triphosphates, dATP, dTTP, dGTP and dCTP, 5mM $MgCl_2$, 70mM KCl, 1.5mM 2-mercaptoethanol, and 8 units of E. coli DNA polymerase I (large fragment), and incubated at 15°C for 20 hours to synthesize dscDNA. The reaction was stopped by adding sodium dodecylsulfate. The dscDNA was extracted with phenol/chloroform, and recovered by precipitation from ethanol.

The resulting dscDNA was dissolved in 100 microliters of 50mM sodium acetate (pH 4.5) containing 1mM $ZnSO_4$, 200mM NaCl, 0.5% glycerol and 0.5 unit of S1 nuclease and incubated at 37°C for 20 minutes to cleave the hairpin structure. The reaction was stopped by adding EDTA. The reaction mixture was extracted with phenol/chloroform and then with diethyl ether. dscDNA was recovered by precipitation from ethanol.

(3) Preparation of oligo(dC)-tailed cDNA

The dscDNA obtained as above was dissolved in 100 microliters of 130mM sodium cacodylate-30mM Tris-HCl (pH 6.8) buffer containing 1mM $CoCl_2$, 0.1mM dithiothreitol, 0.2 microgram of poly(A), 0.1mM H-dCTP (specific activity, 5400 cpm/pmole) and 10 units of terminal deoxynucleotidyl transferase and incubated at 37°C for 20 minutes to permit the addition of oligo(dC) tails to the 3'-termini of dscDNA.

The reaction was stopped by adding EDTA. The reaction mixture was extracted with phenol/chloroform and then with diethyl ether, and the oligo(dC)-tailed dscDNA was recovered by precipitation from ethanol. The oligo-(dC)-tailed dscDNA was dissolved in 10mM Tris-HCl (pH 7.4) buffer containing 1mM EDTA and 100mM NaCl so that it contained 0.2 microgram of the oligo(dC)-tailed dscDNA per ml.

(4) Preparation of oligo(dG)-tailed plasmid pBR322 DNA

pBR322 (10 micrograms) was dissolved in 100 microliters of 20mM Tris-HCl (pH 7.4) buffer containing 10mM $MgCl_2$, 50mM $(NH_4)_2SO_4$ and 10 micrograms of bovine serum albumin, and 15 units of restriction endonuclease Pst I was added. The mixture was incubated at 37°C for 1 hour. After the reaction was terminated, the reaction mixture was extracted with phenol/chloroform, and the resulting DNA was recovered from the aqueous phase by precipitation from ethanol. The DNA obtained was dissolved in 200 microliters of the same reaction buffer as used in tailing of the dscDNA above (except that it contained 80 units of terminal deoxynucleotidyl transferase and $^3H$-dGTP instead of $^3H$-dCTP) and incubated at 37°C for 20 minutes to add about 10-15 dG residues per end. The reaction mixture was extracted with phenol/chloroform, and the oligo(dG)-tailed plasmid pBR322 DNA was recovered from the aqueous phase by ethanol precipitation. The resulting tailed plasmid DNA was dissolved in the same buffer as used in dissolving the oligo(dC)-tailed dscDNA so that it contained the tailed plasmid DNA in a concentration of 2 micrograms per ml.

(5) Construction of a recombinant plasmid

Fifty microliters of the oligo(dC)-tailed cDNA solution was mixed with 50 microliters of the oligo(dG)-tailed pBR322 DNA solution, and the mixture was incubated sequentially at 65°C for 10 minutes, at 57°C for 120 minutes, at 45°C for 60 minutes, at 35°C for 60 minutes and at room temperature for 60 minutes to perform annealing and to construct recombinant plasmids.

(6) Selection of transformants

E. coli x1776 strain was transformed with the recombinant plasmids obtained as above.
Specifically, E. coli x1776 was cultivated at 37°C in 20 ml of L broth supplemented with 100 micrograms/ml of diaminopimelic acid and 40 micrograms/ml of thymidine until the turbidity at 600 nm reached 0.5. The cells were collected by centrifugation at 0°C, and washed with 10mM Tris-HCl (pH 7.3) buffer containing 50 mM CaCl₂. The cells were resuspended in 2 ml of the same buffer as used above, and left to stand at 0°C for 5 minutes. To 0.2 ml of the suspension was added 0.1 ml of the recombinant plasmid solution obtained as above. The mixture was left to stand at 0°C for 15 minutes and then maintained at 42°C for 2 minutes. Then, 0.5 ml of the supplemented L broth used above was added, and cultivation was carried out with shaking for 1 hour. An aliquot of the culture was taken, spread on the supplemented L broth agar plate containing 15 micrograms/ml of tetracycline, and cultivated at 37°C for about 12 hours. Transformants resistant to tetracycline were selected, which were used as a cDNA library.

(7) Hybridization assay

Colony hybridization assay was conducted using a $^{32}$P-labelled cDNA probe by the method of Hanahan and Meselson in order to screen the cDNA library for transformants which had a plasmid containing cDNA encoding rabbit TNF. Induction plus and induction minus $^{32}$P-labelled sscDNA probes were synthesized by the method described in section (2) above using mRNAs obtained from induction plus and minus alveolar macrophages by the method described in section (1) except that $^{32}$P-dCTP with high specific radioactivity was used. By this test, there were selected colonies of transformants harboring the recombinant plasmids which strongly hybridized with the induction plus probe but did not hybridize with the induction minus probe. Fifty colonies were selected from about 20,000 colonies.
Twenty out of the selected 50 colonies were then subjected to a mRNA hybridization-translation assay by the method described in T. Maniatis et al. (ed) "Molecular Cloning", 329 (1980). Cold Spring Harbor Lab. The plasmid DNA was extracted from each of the transformants and fixed to nitrocellulose filters after heat denaturation. The poly(A)mRNA fraction containing rabbit TNF mRNA obtained in section (1) above was added to the filter and incubated at 50°C for 3 hours to perform hybridization. The hybridized mRNA was recovered and injected into the oocytes to determine whether the recovered mRNA was rabbit TNF mRNA. As a result of this test, three colonies were found to have plasmids containing cDNAs that strongly hybridized with the rabbit TNF mRNA. cDNA fragments were obtained from the plasmid having cDNA of the largest size (about 750 bp) by digestion with the restriction endonuclease Dde I. and used as a probe for further screening. These DNA fragments were labelled with [32]P. By using these probes. the cDNA library obtained in section (6) above was screened by a colony hybridization assay, and colonies of transformants having plasmids containing cDNAs which strongly hybridized with the labelled probes were selected. Ninety-eight colonies out of about 60,000 colonies of the cDNA library were found to be positive in this test. The recombinant plasmid DNA was isolated and cDNA inserts were cut out from these recombinant plasmids by digestion with the restriction endonuclease Pst I. and their sizes were measured by polyacrylamide gel electrophoresis. seventeen clones of transformants having cDNA inserts of at least 1 kbp were selected. From a transformant containing cDNA of the largest size (transformant No: x1776 pRTNF802; plasmid No.: pRTNF802), the cloned cDNA was isolated, and its base sequence was determined by the following method.

44

(8) Determination of the base sequence of the cloned cDNA

The tranformant X1776/pRTNF802 selected in section (7) above was cultivated in L broth supplemented with diaminopimelic acid and thymidine. The cells were treated in accordance with the method of Wilkie et al. to obtain a plasmid DNA. The plasmid DNA was digested with the restriction endonuclease Pst I, and purified to obtain a cloned cDNA. The cloned cDNA fragment was further digested with various restriction endonucleases, and the base sequences of suitable restriction endonuclease-cleaved fragments were determined by Maxam-Gilbert method.

The base sequence determined is shown in Table 9 below. The base sequence of the 277th to 738th bases were assigned to the coding region for mature rabbit TNF according to the N-terminal and C-terminal amino acid sequences of TNF purified from rabbit plasma elucidated in Referential Example 2. The 34th to 276th bases constitute a base sequence which is presumed to encode a polypeptide required for constituting a precursor of rabbit TNF.

Referential Example 2

Isolation and purification of rabbit plasma TNF

Rabbits (body weighing 2. 5 to 3.0 kg) were injected intravenously with 50 mg of killed dried cells of Propionibacterium acnes. Eight days later, the rabbits were intravenously injected with 100 micrograms of endotoxin (lipopolysaccharide derived from E. coli). Two hours later, blood was taken from each rabbit by cardiac puncture. The blood was mixed with 100 units of sodium heparin per 100 ml, and then centrifuged at 5,000 rpm for 30 minutes under cooling to remove blood cells and insoluble matters. Twenty-four liters of the plasma was obtained from 400 rabbits.

EDTA (24 g) and 240 g of celite were added to 24 liters of the plasma, and the mixture was stirred for 1 hour and then filtered successively through filters having a pore size of 3 microns, 1 micron and 0.2 micron.

To 24 liters of the filtrate was added 12 liters of 0.04M Tris-HCl (pH 7.8) buffer, and the mixture was applied onto a column (27 x 45 cm) of DEAE-Sepharose CL-6B (Pharmacia) equilibrated with 0.04M Tris-HCl (pH 7.8) buffer containing 0.1M NaCl. The column was then washed with 75 liters of 0.04M Tris-HCl (pH 7.8) buffer containing 0.1M NaCl and then with 50 liters of 0.04M Tris-HCl (pH 7.8) buffer containing 0.15M NaCl, and then eluted with 0.04M Tris-HCl (pH 7.2) buffer containing 0.18M NaCl. The eluate was fractionated into 8-liter fractions, and active fractions having cytotoxic activity were collected. The active fractions were pooled and diluted with an equal volume of 0.04M Tris-HCl (pH 7.8) buffer. The diluted solution was applied onto a column (10 x 13 cm) of DEAE-Sepharose CL-6B. The column was washed with 1 liter of 0.04M Tris-HCl (pH 7.8) buffer containing 0.1M NaCl and then eluted with 5 liters of 0.04M Tris-HCl (pH 7.2) buffer containing 0.18M NaCl. The eluate was fractionated into 250 ml fractions and fractions having cytotoxic activity were collected and pooled.

The active fraction was heated at 60° C for 30 minutes and rapidly cooled to 4° C. The cooled solution was concentrated by ultrafiltration.

The resulting concentrate was applied onto a column (5 x 80 cm) of Sephacryl S-200 (Pharmacia) equilibrated with 0.005M phosphate (pH 7.4) buffer containing 0.1M NaCl. and eluted with the same buffer. The eluate was fractionated into 40 ml fractions. and active fractions were collected. pooled and concentrated by ultrafiltration.

The concentrate of the active fraction obtained by gel filtration was applied onto a column of $Zn^{2+}$ chelate Sepharose as shown below. A column (1.6 x 20 cm) filled with chelate Sepharose (iminodiacetic acid fixed resin) prepared by the method of J. Porath et al., [Nature. 258. 598 (1975)] was washed with 120 ml of zinc chloride solution (1 mg ml) and then equilibrated with 0.05M phosphate (pH 7.4) buffer containing 0.1M NaCl. Then, the concentrate obtained in the previous step was applied onto the column. and eluted with the same buffer. Fractions not adsorbed on the column were collected. Cytotoxic activity was almost completely recovered in these fractions.

The active fractions obtained in the previous step were concentrated and applied onto a column (1.5 x 90 cm) of Toyopearl HW-55 (Toyo Soda Co., Ltd.) fully equilibrated with 0.005M phosphate (pH 7.4) buffer containing 0.15m NaCl. The column was eluted with the same buffer. and active fractions were pooled. This preparation was a purified rabbit plasma TNF.

This purified rabbit plasma TNF obtained herein was used in Referential Examples 3 and 4.

Referential Example 3

Determination of N-terminal and C-terminal amino acid sequences of rabbit plasma TNF

The purified rabbit plasma TNF obtained in Referential Example 2 was used for the determination of the N-terminal and C-terminal amino acid sequences.

The N-terminal amino acid sequence was determined by the Edman degradation method. The resulting phenylthiohydantoin-amino acids were identified by high-performance liquid chromatography using a Zorbax ODS column (Du Pont).

The C-terminal amino acid sequence was determined by the enzymatic method using carboxypeptidases. The purified rabbit plasma TNF was digested with the carboxypeptidases A and Y, at molar ratios of enzyme to substrate of 1:25 and 1:1,000 respectively. The free amino acids released from the C-terminus of rabbit plasma TNF by the double digestion were identified by a micro-amino acid analyzer (Shimadzu Seisakusho) at appropriate intervals from 2 minutes to 180 minutes after the digestion.

It was consequently found that the N-terminal and C-terminal amino acid sequences of rabbit plasma TNF were as follows:

N-terminal: Ser-Ala-Ser-Arg-Ala- .....
C-terminal: ..... Val-Tyr-Phe-Gly-Ile-Ile-Ala-Leu

Referential Example 4

Preparation of an anti-rabbit plasma TNF antibody

TNF solution containing $1.9 \times 10^5$ units (L-929) of the purified rabbit plasma TNF obtained in Referential Example 2 was emulsified with an equal volume of the Freund's complete adjuvant, and the emulsion was injected subcutaneously into the back of guinea pigs at several parts. Then, the animals were immunized by the same method 1, 3 and 6 weeks later. Furthermore, 8 weeks later, the same amount of the purified rabbit plasma TNF was intraperitoneally injected together with aluminum hydroxide gel. The whole blood was taken by cardiac puncture 9 weeks after the first immunization, and centrifuged to obtain an antiserum containing an anti-rabbit plasma TNF antibody.

The antiserum was passed through a column of Sepharose 4B coupled with serum protein components of normal rabbits. By repeating it 3 times a purified antibody specific for the rabbit plasma TNF was obtained. It was confirmed by immunoelectrophoresis and gel double diffusion methods that this antibody formed a single precipitation line only with the purified rabbit plasma TNF.

An about 60,000-fold dilution of this purified antibody has the ability of neutralizing 50% of 500 units (L-929) of the cytotoxic activity of the rabbit plasma TNF, and an about 1,000-fold dilution of the antibody can completely neutralize 50 units (LM) of the cytotoxic activity of the rabbit plasma TNF.

## Claims

1. A DNA having or containing a base sequence corresponding to the human tumor necrosis factor polypeptide, its precursor polypeptide or its modified or allelic mutant polypeptide consisting of an amino acid sequence represented by the following formula [I]:

$$(Y)_p - (X)_n - (B)_m - A \qquad\qquad [I]$$

wherein
A is a polypeptide of the formula [Ia] below in which one to twelve amino acids at the N-terminus may be deleted or one or two amino acids may be replaced by other amino acid(s),
B is a peptide of the formula [Ib] below in which one to three amino acids may be deleted or

46

replaced by other amino acid(s),
X is a polypeptide,
Y is Met, and
m, n and p are 1 or 0;
the formula [Ia] being as follows:

```
Thr Pro Ser Asp Lys Pro Val Ala His Val
Val Ala Asn Pro Gln Ala Glu Gly Gln Leu
Gln Trp Leu Asn Arg Arg Ala Asn Ala Leu
Leu Ala Asn Gly Val Glu Leu Arg Asp Asn
Gln Leu Val Val Pro Ser Glu Gly Leu Tyr
Leu Ile Tyr Ser Gln Val Leu Phe Lys Gly
Gln Gly Cys Pro Ser Thr His Val Leu Leu
Thr His Thr Ile Ser Arg Ile Ala Val Ser

Tyr Gln Thr Lys Val Asn Leu Leu Ser Ala
Ile Lys Ser Pro Cys Gln Arg Glu Thr Pro
Glu Gly Ala Glu Ala Lys Pro Trp Tyr Glu
Pro Ile Tyr Leu Gly Gly Val Phe Gln Leu
Glu Lys Gly Asp Arg Leu Ser Ala Glu Ile
Asn Arg Pro Asp Tyr Leu Asp Phe Ala Glu
Ser Gly Gln Val Tyr Phe Gly Ile Ile Ala
Leu                              ... [Ia];
```

and the formula [Ib] being as follows:

```
Ser-Ser-Ser-Arg            ... [Ib];
```

and the polypeptide represented by the above formula B-A having the functional characteristics as a mature human necrosis factor.

2. A DNA claimed in claim 1 having or containing a base sequence corresponding to an amino acid sequence represented by the following formula [I]:

$$(Y)_p - (X)_n - (B)_m - A \qquad [I]$$

wherein
A. B. Y. m. n and p are the same as defined in claim 1, and X is a polypeptide of the formula [Ic]:

```
Ser Thr Glu Ser Met Ile Arg Asp Val Glu
Leu Ala Glu Glu Ala Leu Pro Lys Lys Thr
Gly Gly Pro Gln Gly Ser Arg Arg Cys Leu
Phe Leu Ser Leu Phe Ser Phe Leu Ile Val
Ala Gly Ala Thr Thr Leu Phe Cys Leu Leu
His Phe Gly Val Ile Gly Pro Gln Arg Glu
Glu Phe Pro Arg Asp Leu Ser Leu Ile Ser
Pro Leu Ala Gln Ala Val Arg    ... [Ic];
```

and the polypeptide represented by the above formula Y-X-B-A having the functional characteristics of precursor polypeptide of human tumor necrosis factor.

3. A DNA according to claim 1 having or containing a base sequence corresponding to the human tumor necrosis factor precursor polypeptide consisting of an amino acid sequence represented by the following formula [I]:

$$(Y)_p - (X)_n - (B)_m - A \qquad\qquad [I]$$

or its allelic mutant polypeptide, wherein
A is a polypeptide of the formula [Ia] shown in claim 1,
B is a peptide of the formula [Ib] shown in claim 1,
Y is the same as defined in claim 1,
X is a polypeptide of the formula [Ic] shown in claim 2, and
m, n and p are 1.

4. A DNA having or containing a base sequence corresponding to the mature human tumor necrosis factor polypeptide consisting of an amino acid sequence represented by the following formula [I]:

$$(Y)_p - (X)_n - (B)_m - A \qquad\qquad [I]$$

or its allelic mutant polypeptide, wherein
A is a polypeptide of the formula [Ia] shown in claim 1,
B is a peptide of the formula [Ib] shown in claim 1,
m is 1, n is 0, and
Y and p are the same as defined in claim 1.

5. A DNA having or containing a base sequence corresponding to the modified human tumor necrosis factor polypeptide consisting of an amino acid sequence represented by the following formula [I]:

$$(Y)_p - (X)_n - (B)_m - A \qquad\qquad [I]$$

wherein
A is a polypeptide of the formula [Ia] shown in claim 1,
n and m are 0, and
Y and p are the same as defined in claim 1.

48

6. A DNA having or containing a base sequence corresponding to the modified human tumor necrosis factor-polypeptide consisting of an amino acid sequence represented by the following formula [I]:

$$(Y)_p - (X)_n - (B)_m - A \qquad [I]$$

wherein

A is a polypeptide of the formula [Ia] in which one to twelve amino acids are deleted or one to two amino acids are replaced by other amino acid(s),

m and n are 0, and

y and p are the same as defined in claim 1.

7. A DNA having or containing a base sequence corresponding to the modified human tumor necrosis factor polypeptide consisting of an amino acid sequence represented by the following formula [I]

$$(Y)_p - (X)_n - (B)_m - A \qquad [I]$$

wherein

A is a polypeptide of the formula [Ia] in which Thr in the first position from the N-terminus, amino acids from the above Thr in the first position to Pro in the 6th position, amino acids from the above Thr in the first position to His in the 9th position, or amino acids from the above Thr in the first position to Ala in the 12th position are deleted,

m and n are 0, and

Y and p are the same as defined in claim 1.

8. A DNA having or containing a base sequence corresponding to the modified human tumor necrosis factor polypeptide consisting of an amino acid sequence represented by the following formula [I]:

$$-(Y)_p - (X)_n - (B)_m - A \qquad [I]$$

wherein

A is a polypeptide of the formula [Ia] in which Thr and His in the 66th and 67th positions from the N-terminus are replaced by His, Thr or Tyr,

m and n are 0, and

Y and p are the same as defined in claim 1.

9. A DNA claimed in claim 2 having or containing a base sequence represented by the following formula [II]:

$$(Y')_p - (X')_n - (B')_m - A' \qquad [II]$$

wherein

A' is a base sequence of the formula [IIa] below in which one to twelve codons at the 5'-terminus may be deleted or one or two amino acids may be replaced by other codon(s),

B' is a base sequence of the formula [IIb] below in which one to three codons may be deleted or replaced by other codon(s),

X' is a base sequence of the formula [IIc] below,

Y' is ATG, and

m, n and p are 1 or 0: the formula [IIa] being as follows:

49

```
(5')-ACC CCG AGT GAC AAG CCT GTA GCC
CAT GTT GTA GCA AAC CCT CAA GCT GAG GGG
CAG CTC CAG TGG CTG AAC CGC CGG GCC AAT
GCC CTC CTG GCC AAT GGC GTG GAG CTG AGA
GAT AAC CAG CTG GTG GTG CCA TCA GAG GGC

CTG TAC CTC ATC TAC TCC CAG GTC CTC TTC
AAG GGC CAA GGC TGC CCC TCC ACC CAT GTG
CTC CTC ACC CAC ACC ATC AGC CGC ATC GCC
GTC TCC TAC CAG ACC AAG GTC AAC CTC CTC
TCT GCC ATC AAG AGC CCC TGC CAG AGG GAG
ACC CCA GAG GGG GCT GAG GCC AAG CCC TGG
TAT GAG CCC ATC TAT CTG GGA GGG GTC TTC
CAG CTG GAG AAG GGT GAC CGA CTC AGC GCT
GAG ATC AAT CGG CCC GAC TAT CTC GAC TTT
GCC GAG TCT GGG CAG GTC TAC TTT GGG ATC
ATT GCC CTG-(3')                  ... [IIa],
```

the formula [IIb] being as follows:

```
(5')-TCA-TCT-TCT-CGA-(3')                  [IIb],
```

the formula [IIc] being as follows:

```
(5')-AGC ACT GAA AGC ATG ATC CGG GAC
GTG GAG CTG GCC GAG GAG GCG CTC CCC AAG
AAG ACA GGG GGG CCC CAG GGC TCC AGG CGG
TGC TTG TTC CTC AGC CTC TTC TCC TTC CTG
ATC GTG GCA GGC GCC ACC ACG CTC TTC TGC
CTG CTG CAC TTT GGA GTG ATC GGC CCC CAG
AGG GAA GAG TTC CCC AGG GAC CTC TCT CTA
ATC AGC CCT CTG GCC CAG GCA GTC AGA-(3')
                                  ... [IIc].
```

10. A DNA claimed in claim 3 having or containing a base sequence represented by the following formula [II]:

$$(Y')_p - (X')_n - (B')_m - A' \qquad [II]$$

wherein
A' is a base sequence of the formula [IIa] shown in claim 9,
B' is a base sequence of the formula [IIb] shown in claim 9,
m, n and p are 1, and
X' and Y' are the same as defined in claim 9.

11. A DNA claimed in claim 4 having or containing a base sequence represented by the following formula [II]:

$$(Y')_p - (X')_n - (B')_m - A' \qquad [II]$$

wherein
A' is a base sequence of the formula [IIa] shown in claim 9,
B' is a base sequence of the formula [IIb] shown in claim 9,
m is 1,
n is 0, and
Y' and p are the same as defined in claim 9.

12. A DNA claimed in claim 5 having or containing a base sequence represented by the following formula [II]:

$$(Y')_p - (X')_n - (B')_m - A' \qquad [II]$$

wherein
A' is a base sequence of the formula [IIa] shown in claim 9,
m and n are 0, and
Y' and p are the same as defined in claim 9.

13. A DNA claimed in claim 6 having or containing a base sequence represented by the following formula [II]:

$$(Y')_p - (X')_n - (B')_m - A' \qquad [II]$$

wherein
A' is a base sequence of the formula [IIa] in which one to twelve codons at the 5'-terminus are deleted or one or two are replaced by other codon(s),
m and n are 0, and
Y' and p are the same as defined in claim 9.

14. A DNA claimed in claim 7 having or containing a base sequence represented by the following formula [II]:

$$(Y')_p - (X')_n - (B')_m - A' \qquad [II]$$

wherein
A' is a base sequence of the formula [IIa] in which ACC in the first codon from the 5'-terminus, codons from the above ACC in the first codon to CCT in the 6th codon, codons from the above ACC in the first codon to CAT in the 9th codon, or codons from the above ACC in the first codon to GCA in the 12th codon are deleted,
m and n are 0, and
Y' and p are the same as defined in claim 9.

51

15. A DNA claimed in claim 9 having or containing a base sequence represented by the following formula [II]:

$$(Y')_p - (X')_n - (B')_m - A' \qquad\qquad [II]$$

wherein
A' is a base sequence of the formula [IIa] in which ACC and CAT in the 66th and 67th codons from the 5'-terminus are replaced by CAT, ACC or TAC,
m and n are 0, and
Y' and p are the same as defined in claim 9.

16. A vector into which the DNA of anyone of the preceding claims is inserted.

17. The vector of claim 16 which is an expression vector.

18. An expression vector of claim 17 which comprises a base sequence corresponding to an amino acid sequence represented by the following formula [I]:

$$(Y)_p - (X)_n - (B)_m - A \qquad\qquad [I]$$

wherein
p is 1, and
A, B, X, Y, m and n are the same as defined in claim 1, and having a termination codon at the 3'-terminus of said base sequence, wherein said base sequence is under the control of a transcriptional promoter.

19. An expression vector of claim 18 wherein the transcriptional promoter is a trp promoter or a tac promoter.

20. An expression vector of claim 18 wherein the base sequence corresponding to the amino acid sequence of formula [I] as defined in claim 18 having a termination codon at the 3'-terminus of said base sequence is connected in the correct translational reading frame to a second base sequence encoding a protein to be fused.

21. An expression vector of claim 20 wherein the second base sequence encoding a protein to be fused is a phoS gene.

22. A vector of claims 16 to 21 which can proliferate in Escherichia coli.

23. A vector of claim 22 which is Escherichia coli plasmid.

24. A vector of claim 23 which is pBR322.

25. A host transformed with a vector of claims 16 to 24.

26. A host of claim 25 which is a microorganisms

27. A host of claim 26 which is Escherichia coli.

28. A polypeptide having or containing a human tumor necrosis factor polypeptide, its precursor polypeptide or its modified or allelic mutant polypeptide consisting of an amino acid sequence represented by the following formula [I]:

52

EP 0 155 549 B1

$$(Y)_p - (X)_n - (B)_m - A \qquad [I]$$

or its derivative or a salt thereof, wherein

A is a polypeptide of the formula [Ia] below in which one to twelve amino acids may be deleted or one or two amino acids may be replaced by other amino acid(s),

B is a peptide of the formula [Ib] below in which one to three amino acids may be deleted or replaced by other amino acid(s),

X is a polypeptide,

Y is Met, and

m, n and p are respectively 1 or 0;

the formula [Ia] being as follows:

```
Thr Pro Ser Asp Lys Pro Val Ala His Val

Val Ala Asn Pro Gln Ala Glu Gly Gln Leu

Gln Trp Leu Asn Arg Arg Ala Asn Ala Leu

Leu Ala Asn Gly Val Glu Leu Arg Asp Asn

Gln Leu Val Val Pro Ser Glu Gly Leu Tyr

Leu Ile Tyr Ser Gln Val Leu Phe Lys Gly

Gln Gly Cys Pro Ser Thr His Val Leu Leu

Thr His Thr Ile Ser Arg Ile Ala Val Ser

Tyr Gln Thr Lys Val Asn Leu Leu Ser Ala


Ile Lys Ser Pro Cys Gln Arg Glu Thr Pro

Glu Gly Ala Glu Ala Lys Pro Trp Tyr Glu

Pro Ile Tyr Leu Gly Gly Val Phe Gln Leu

Glu Lys Gly Asp Arg Leu Ser Ala Glu Ile

Asn Arg Pro Asp Tyr Leu Asp Phe Ala Glu

Ser Gly Gln Val Tyr Phe Gly Ile Ile Ala

Leu                                ... [Ia];
```

the formula [Ib] being as follows:

```
Ser-Ser-Ser-Arg                    ... [Ib];
```

and the polypeptide represented by the above formula B-A having the functional characteristics as a mature human tumor necrosis factor.

**29.** A polypeptide claimed in claim 28 represented by the following formula [I]:

53

$$(Y)_p - (X)_n - (B)_m - A \qquad\qquad [I]$$

or its derivative or a salt thereof, wherein

A, B, Y, m, n and p are the same as defined in claim 28, and x is a polypeptide of the formula [Ic] being as follows:

```
Ser Thr Glu Ser Met Ile Arg Asp Val Glu

Leu Ala Glu Glu Ala Leu Pro Lys Lys Thr

Gly Gly Pro Gln Gly Ser Arg Arg Cys Leu

Phe Leu Ser Leu Phe Ser Phe Leu Ile Val

Ala Gly Ala Thr Thr Leu Phe Cys Leu Leu


His Phe Gly Val Ile Gly Pro Gln Arg Glu

Glu Phe Pro Arg Asp Leu Ser Leu Ile Ser

Pro Leu Ala Gln Ala Val Arg    ... [Ic];
```

and the polypeptide represented by the above formula Y-X-B-A having the functional characteristics of precursor polypeptide of human tumor necrosis factor

**30.** A polypeptide having or containing a human tumor necrosis factor precursor polypeptide consisting of an amino acid sequence represented by the following formula [I]:

$$(Y)_p - (X)_n - (B)_m - A \qquad\qquad [I]$$

wherein

A is a polypeptide of the formula [Ia] shown in claim 28,

B is a peptide of the formula [Ib] shown in claim, 28,

X is a polypeptide of the formula [Ic] shown in claim 29,

m and n are 1. and

Y and p are the same as defined in claim 28.

**31.** A polypeptide having or containing a mature human tumor necrosis factor polypeptide consisting of an amino acid sequence represented by the following formula [I]:

$$(Y)_p - (X)_n - (B)_m - A \qquad\qquad [I]$$

wherein

A is a polypeptide of the formula [Ia] shown in claim 28,

B is a peptide of the formula [Ib] shown in claim 28,

m is 1,

n is 0. and

Y and p are the same as defined in claim 28.

**32.** A polypeptide having or containing a modified human tumor necrosis factor polypeptide consisting of

54

an amino acid sequence represented by the following formula [I]:

$$(Y)_p - (X)_n - (B)_m - A \qquad [I]$$

wherein
A is a polypeptide of the formula [Ia] shown in claim 28,
m and n are 0, and
Y and p are the same as defined in claim 28.

33. A polypeptide claimed in claim 31 represented by the following formula [I]:

$$(Y)_p - (X)_n - (B)_m - A \qquad [I]$$

wherein
A is a polypeptide of the formula [Ia] shown in claim 28,
B is a peptide of the formula [Ib] shown in claim 28,
m is 1, and
n and p are 0.

34. A polypeptide claimed in claim 32 represented by the following formula [I]:

$$(Y)_p - (X)_n - (B)_m - A \qquad [I]$$

wherein
A is a polypeptide of formula [Ia] shown in claim 28, and
m, n and p are 0.

35. A polypeptide having or containing a modified human tumor necrosis factor polypeptide consisting of an amino acid sequence represented by the following formula [I]:

$$(Y)_p - (X)_n - (B)_m - A \qquad [I]$$

wherein
A is a polypeptide of the formula [Ia] in which one to twelve amino acids are deleted or one to two amino acids are replaced by other amino acid(s),
m and n are 0, and 2 -
Y and p are the same as defined in claim 28.

36. A polypeptide claimed in claim 35 represented by the following formula [I]:

$$(Y)_p - (X)_n - (B)_m - A \qquad [I]$$

wherein
A is a polypeptide of the formula [Ia] in which Thr in the first position from the N-terminus, amino acids from the above Thr in the first position to Pro in the 6th position, amino acids from the above Thr in the first position to His in the 9th position, or amino acids from the above Thr in the first position to Ala in the 12th position are deleted,
m and n are 0, and
Y and p are the same as defined in claim 28.

37. A polypeptide claimed in claim 35 represented by the following formula [I]:

$$(Y)_p - (X)_n - (B)_m - A \qquad [I]$$

wherein

A is a polypeptide of the formula [Ia] in which Thr and His in the 66th and 67th positions from the N-terminus are replaced by His, Thr or Tyr,

m and n are 0, and

Y and p are the same as defined in claim 28.

38. A substance resulting from chemical or enzymatic modification of a polypeptide claimed in anyone of claims 30 to 37.

39. A pharmaceutically acceptable salt of a polypeptide claimed in anyone of claims 30 to 38.

40. An aggregate consisting of a polypeptide claimed in anyone of claims 30 to 38 as a subunit.

41. An aggregate claimed in claim 40 which is a trimer.

42. A trimer claimed in claim 41 which consists of a polypeptide claimed in claim 33 as a subunit.

43. A pharmaceutical preparation containing a substance claimed in anyone of claims 29 to 42 as an active ingredient.

44. Use of anyone of the substances claimed in claims 29 to 43 as an antitumor agent.

45. A process for the production of a DNA having or containing a base sequence corresponding to an amino acid sequence represented by the following formula [I]:

$$(Y)_p - (X)_n - (B)_m - A \qquad [I]$$

wherein

A, b, X, Y, m, n and p are the same as defined in claim 1, which comprises

(1) cultivating human macrophages together with inducer(s),

(2) separating a fraction containing a human tumor necrosis factor mRNA from the macrophages,

(3) preparing a single-stranded cDNA from the mRNA by using reverse transcriptase and then converting it to a double-stranded cDNA,

(4) inserting the double-stranded cDNA into a vector,

(5) introducing the recombinant vector in to a host to transform it and construct a cDNA library,

(6) cloning cDNA encoding the human tumor necrosis factor polypeptide or its allelic mutant polypeptide from the library, and

(7) as desired, modifying the cloned cDNA.

46. A process of claim 45 which is for the production of a DNA having or containing a base sequence represented by the following formula [II]:

$$(Y')_p - (X')_n - (B')_m - A' \qquad [II]$$

wherein

A', B', X', Y', m, n and p are the same as defined in claim 9.

47. A process for the production of a polypeptide represented by the following formula [I]:

$$(Y)_p - (X)_n - (B)_m - A \qquad [I]$$

or its derivative or a salt thereof, wherein

A, B, X, Y, m, n and p are the same as defined in claim 28, which comprises inserting a DNA having or containing a base sequence enooding the above polypeptide into an expression vector, transforming a host with the recombinant vector, cultivating the host and collecting the resulting polypeptide, and if desired, modifying the polypeptide.

Claims for the following Contracting State: AT

1. A process for the production of a DNA having or containing a base sequence corresponding to the human tumor necrosis factor polypeptide, its precursor polypeptide or its modified or allelic mutant polypeptide consisting of an amino acid sequence represented by the following formula [I]:

$$(Y)_p - (X)_n - (B)_m - A \qquad [I]$$

wherein

A is a polypeptide of the formula [Ia] below in which one to twelve amino acids at the N-terminus may be deleted or one or two amino acids may be replaced by other amino acid(s),

B is a peptide of the formula [Ib] below in which one to three amino acids may be deleted or replaced by other amino acid(s),

X is a polypeptide,

y is Met, and

m, n and p are 1 or 0;

the formula [Ia] being as follows:

```
Thr Pro Ser Asp Lys Pro Val Ala His Val
Val Ala Asn Pro Gln Ala Glu Gly Gln Leu
Gln Trp Leu Asn Arg Arg Ala Asn Ala Leu
Leu Ala Asn Gly Val Glu Leu Arg Asp Asn
Gln Leu Val Val Pro Ser Glu Gly Leu Tyr
Leu Ile Tyr Ser Gln Val Leu Phe Lys Gly
Gln Gly Cys Pro Ser Thr His Val Leu Leu
Thr His Thr Ile Ser Arg Ile Ala Val Ser

Tyr Gln Thr Lys Val Asn Leu Leu Ser Ala
Ile Lys Ser Pro Cys Gln Arg Glu Thr Pro
Glu Gly Ala Glu Ala Lys Pro Trp Tyr Glu
Pro Ile Tyr Leu Gly Gly Val Phe Gln Leu
Glu Lys Gly Asp Arg Leu Ser Ala Glu Ile
Asn Arg Pro Asp Tyr Leu Asp Phe Ala Glu
Ser Gly Gln Val Tyr Phe Gly Ile Ile Ala
Leu                          ... [Ia];
```

and the formula [Ib] being as follows:

$$Ser-Ser-Ser-Arg \qquad \ldots [Ib];$$

and the polypeptide represented by the above formula B-A having the functional characteristics as a mature human necrosis factors, which comprises

(1) cultivating human macrophages together with inducer(s),

(2) separating a fraction containing a human tumor necrosis factor mRNA from the macrophages,

(3) preparing a single-stranded cDNA from the mRNA by using reverse transcriptase and then converting it to a double-stranded cDNA,

(4) inserting the double-stranded cDNA into a vector,

(5) introducing the recombinant vector into a host to transform it and construct a cDNA library,

(6) cloning cDNA encoding the human tumor necrosis factor polypeptide or its allelic mutant polypeptide from the library, and

(7) as desired, modifying the cloned cDNA.

2. A process of claim 1 which is for the production of a DNA having or containing a base sequence corresponding to an amino acid sequence represented by the following formula [I]:

$$(Y)_{p} - (X)_{n} - (B)_{m} - A \qquad [I]$$

wherein

A, B, Y, m, n and p are the same as defined in claim 1, and X is a polypeptide of the formula [Ic]:

Ser Thr Glu Ser Met Ile Arg Asp Val Glu

Leu Ala Glu Glu Ala Leu Pro Lys Lys Thr

Gly Gly Pro Gln Gly Ser Arg Arg Cys Leu

Phe Leu Ser Leu Phe Ser Phe Leu Ile Val

Ala Gly Ala Thr Thr Leu Phe Cys Leu Leu

His Phe Gly Val Ile Gly Pro Gln Arg Glu

Glu Phe Pro Arg Asp Leu Ser Leu Ile Ser

Pro Leu Ala Gln Ala Val Arg     ... [Ic];

and the polypeptide represented by the above formula Y-X-B-A having the functional characteristics of precursor polypeptide of human tumor necrosis factor.

3. A process of claim 1 which is for the production of a DNA having or containing a base sequence corresponding to the human tumor necrosis factor precursor polypeptide consisting of an amino acid sequence represented by the following formula [I]:

$$(Y)_{p} - (X)_{n} - (B)_{m} - A \qquad [I]$$

or its allelic mutant polypeptide, wherein

A is a polypeptide of the formula [Ia] shown in claim 1,

B is a peptide of the formula [Ib] shown in claim 1,

y is the same as defined in claim 1,

EP 0 155 549 B1

X is a polypeptide of the formula [lc] shown in claim 2, and
m, n and p are 1.

4. A process of claim 1 which is for the production of a DNA having or containing a base sequence corresponding to the mature human tumor necrosis factor polypeptide consisting of an amino acid sequence represented by the following formula [I]:

$$(Y)_p - (X)_n - (B)_m - A \qquad [I]$$

or its allelic mutant polypeptide, wherein
A is a polypeptide of the formula [la] shown in claim 1,
B is a peptide of the formula [lb] shown in claim 1,
m is 1, n is 0, and
y and p are the same as defined in claim 1.

5. A process of claim 1 which is for the production of a DNA having or containing a base sequence corresponding to the modified human tumor necrosis factor polypeptide consisting of an amino acid sequence represented by the following formula [I]:

$$(Y)_p - (X)_n - (B)_m - A \qquad [I]$$

wherein
A is a polypeptide of the formula [la] shown in claim 1,
n and m are 0, and
Y and p are the same as defined in claim 1.

6. A process of claim 1 which is the production of a DNA having or containing a base sequence corresponding to the modified human tumor necrosis factor polypeptide consisting of an amino acid sequence represented by the following formula [I]:

$$(Y)_p - (X)_n - (B)_m - A \qquad [I]$$

wherein
A is a polypeptide of the formula [la] in which one to twelve amino acids are deleted or one to two amino acids are replaced by other amino acid(s),
m and n are 0, and
Y and p are the same as defined in claim 1.

7. A process of claim 1 which is for the production of a DNA having or containing a base sequence corresponding to the modified human tumor necrosis factor polypeptide consisting of an amino acid sequence represented by the following formula [I]

$$(Y)_p - (X)_n - (B)_m - A \qquad [I]$$

wherein
A is a polypeptide of the formula [la] in which Thr in the first position from the N-terminus, amino acids from the above Thr in the first position to Pro in the 6th position, amino acids from the above Thr in the first position to His in the 9th position, or amino acids from the above Thr in the first position to Ala in the 12th position are deleted,
m and n are 0, and
Y and p are the same as defined in claim 1.

8. A process of claims 1 which is for the production of a DNA having or containing a base sequence corresponding to the modified human tumor necrosis factor polypeptide consisting of an amino acid sequence represented by the following formula [I]:

$$(Y)_p - (X)_n - (B)_m - A \qquad [I]$$

wherein

A is a polypeptide of the formula [Ia] in which Thr and His in the 66th and 67th positions from the N-terminus are replaced by His, Thr or Tyr,

m and n are 0, and

y and p are the same as defined in claim 1.

9. A process of claim 1 which is for the production of a DNA having or containing a base sequence represented by the following formula [II] :

$$(Y')_p - (X')_n - (B')_m - A' \qquad [II]$$

wherein

A' is a base sequence of the formula [IIa] below in which one to twelve codons at the 5'-terminus may be deleted or one or two amino acids may be replaced by other codon(s),

B' is a base sequence of the formula [IIb] below in which one to three codons may be deleted or replaced by other codon(s),

X' is a base sequence of the formula [IIc] below,

Y' is ATG, and

m, n and p are 1 or 0; the formula [IIa] being as follows:

```
(5')-ACC-CCG AGT GAC AAG CCT GTA GCC

CAT GTT GTA GCA AAC CCT CAA GCT GAG GGG

CAG CTC CAG TGG CTG AAC CGC CGG GCC AAT

GCC CTC CTG GCC AAT GGC GTG GAG CTG AGA

GAT AAC CAG CTG GTG GTG CCA TCA GAG GGC
```

```
CTG TAC CTC ATC TAC TCC CAG GTC CTC TTC

AAG GGC CAA GGC TGC CCC TCC ACC CAT GTG

CTC CTC ACC CAC ACC ATC AGC CGC ATC GCC

GTC TCC TAC CAG ACC AAG GTC AAC CTC CTC

TCT GCC ATC AAG AGC CCC TGC CAG AGG GAG

ACC CCA GAG GGG GCT GAG GCC AAG CCC TGG

TAT GAG CCC ATC TAT CTG GGA GGG GTC TTC

CAG CTG GAG AAG GGT GAC CGA CTC AGC GCT

GAG ATC AAT CGG CCC GAC TAT CTC GAC TTT

GCC GAG TCT GGG CAG GTC TAC TTT GGG ATC

ATT GCC CTG-(3')                    ...: [IIa],
```

the formula [IIb] being as follows:

$$(5')-TCA-TCT-TCT-CGA-(3')\qquad [IIb],$$

the formula [IIc] being as follows:

```
(5')-AGC ACT GAA AGC ATG ATC CGG GAC
GTG GAG CTG GCC GAG GAG GCG CTC CCC AAG
AAG ACA GGG GGG CCC CAG GGC TCC AGG CGG
TGC TTG TTC CTC AGC CTC TTC TCC TTC CTG
ATC GTG GCA GGC GCC ACC ACG CTC TTC TGC
CTG CTG CAC TTT GGA GTG ATC GGC CCC CAG
AGG GAA GAG TTC CCC AGG GAC CTC TCT CTA
ATC AGC CCT CTG GCC CAG GCA GTC AGA-(3')
                                    ... [IIc].
```

**10.** A process for the production of a polypeptide having or containing a human tumor necrosis factor polypeptide, its precursor polypeptide or its modified or allelic mutant polypeptide consisting of an amino acid sequence represented by the following formula [I]:

$$(Y)_p-(X)_n-(B)_m-A\qquad [I]$$

or its derivative or a salt thereof, wherein

A is a polypeptide of the formula [Ia] below in which one to twelve amino acids may be deleted or one or two amino acids may be replaced by other amino acid(s),

B is a peptide of the formula [Ib] below in which one to three amino acids may be deleted or replaced by other amino acid(s),

X is a polypeptide,

Y is Met, and

m, n and p are respectively 1 or 0:

the formula [Ia] being as follows:

```
Thr Pro Ser Asp Lys Pro Val Ala His Val

Val Ala Asn Pro Gln Ala Glu Gly Gln Leu

Gln Trp Leu Asn Arg Arg Ala Asn Ala Leu

Leu Ala Asn Gly Val Glu Leu Arg Asp Asn

Gln Leu Val Val Pro Ser Glu Gly Leu Tyr

Leu Ile Tyr Ser Gln Val Leu Phe Lys Gly

Gln Gly Cys Pro Ser Thr His Val Leu Leu

Thr His Thr Ile Ser Arg Ile Ala Val Ser

Tyr Gln Thr Lys Val Asn Leu Leu Ser Ala


Ile Lys Ser Pro Cys Gln Arg Glu Thr Pro

Glu Gly Ala Glu Ala Lys Pro Trp Tyr Glu

Pro Ile Tyr Leu Gly Gly Val Phe Gln Leu

Glu Lys Gly Asp Arg Leu Ser Ala Glu Ile

Asn Arg Pro Asp Tyr Leu Asp Phe Ala Glu

Ser Gly Gln Val Tyr Phe Gly Ile Ile Ala

Leu                                  ... [Ia];
```

the formula [Ib] being as follows:

$$Ser-Ser-Ser-Arg \qquad \qquad ... \ [Ib];$$

and the polypeptide represented by the above formula B-A having the functional characteristics as a mature human tumor necrosis factor, which comprises inserting a DNA having or containing a base sequence encoding the above polypeptide into an expression vector, transforming a host with the recombinant vector, cultivating the host and collecting the resulting polypeptide, and if desired, modifying the polypeptide.

11. A process of claim 10 which is for the production of a polypeptide having or containing a polypeptide represented by the following formula [I] :

$$(Y)_p-(X)_n-(B)_m-A \qquad \qquad [I]$$

or its derivative or a salt thereof, wherein
A, B, Y, m, n and p are the same as defined in claim 10. and X is a polypeptide of the formula [Ic] being as follows:

```
Ser Thr Glu Ser Met Ile Arg Asp Val Glu

Leu Ala Glu Glu Ala Leu Pro Lys Lys Thr

Gly Gly Pro Gln Gly Ser Arg Arg Cys Leu

Phe Leu Ser Leu Phe Ser Phe Leu Ile Val

Ala Gly Ala Thr Thr Leu Phe Cys Leu Leu


His Phe Gly Val Ile Gly Pro Gln Arg Glu

Glu Phe Pro Arg Asp Leu Ser Leu Ile Ser

Pro Leu Ala Gln Ala Val Arg    ... [Ic];
```

and the polypeptide represented by the above formula Y-X-B-A having the functional characteristics of precursor polypeptide of human tumor necrosis factor.

12. A process of claim 10 which is for the production of a polypeptide having or containing a human tumor necrosis factor precursor polypeptide consisting of an amino acid sequence represented by the following formula [I]:

$$(Y)_p-(X)_n-(B)_m-A \qquad [I]$$

wherein
A is a polypeptide of the formula [Ia] shown in claim 10,
B is a peptide of the formula [Ib] shown in claim 10,
X is a polypeptide of the formula [Ic] shown in claim 11,
m and n are 1, and
Y and p are the same as defined in claim 10.

13. A process of claim 10 which is for the production of a polypeptide having or containing a mature human tumor necrosis factor polypeptide consisting of an amino acid sequence represented by the following formula [I]:

$$(Y)_p-(X)_n-(B)_m-A \qquad [I]$$

wherein
A is a polypeptide of the formula [Ia] shown in claim 10,
B is a peptide of the formula [Ib] shown in claim 10,
m is 1,
n is 0, and
Y and p are the same as defined in claim 10.

14. A process of claim 10 which is for the production of a polypeptide having or containing a modified human tumor necrosis factor polypeptide consisting of an amino acid sequence represented by the following formula [I] :

$$(Y)_p-(X)_n-(B)_m-A \qquad [I]$$

wherein

A is a polypeptide of the formula [Ia] shown in claim 10,
m and n are 0, and
Y and p are the same as defined in claim 10.

15. A process of claim 10 which is for the production of a polypeptide claimed in claim 13 represented by the following formula [I] :

$$(Y)_p - (X)_n - (B)_m - A \qquad [I]$$

wherein
A is a polypeptide of the formula [Ia] shown in claim 10,
B is a peptide of the formula (Ib) shown in claim 10,
m is 1, and
n and p are 0.

16. A process of claim 10 which is for the production of a polypeptide claimed in claims 14 represented by the following formula [I]:

$$(Y)_p - (X)_n - (B)_m - A \qquad [I]$$

wherein
A is a polypeptide of formula [Ia] shown in claim 10, and
m, n and p are 0.

17. A process of claim 10 which is for the production of a polypeptide having or containing a modified human tumor necrosis factor polypeptide consisting of an amino acid sequence represented by the following formula [I] :

$$(Y)_p - (X)_n - (B)_m - A \qquad [I]$$

wherein
A is a polypeptide of the formula [Ia] in which one to twelve amino acids are deleted or one to two amino acids are replaced by other amino acid(s).
m and n are 0, and
Y and p are the same as defined in claim 10,

18. A process of claim 10 which is for the production of a polypeptide claimed in claim 17 represented by the following formula [I] :

$$(Y)_p - (X)_n - (B)_m - A \qquad [I]$$

wherein
A is a polypeptide of the formula [Ia] in which Thr in the first position from the N-terminus, amino acids from the above Thr in the first position to Pro in the 6th position, amino acids from the above Thr in the first position to His in the 9th position, or amino acids from the above Thr in the first position to Ala in the 12th position are deleted,
m and n are 0, and
y and p are the same as defined in claims 10.

19. A process of claim 10 which is for the production of a polypeptide claimed in claim 17 represented by the following formula [I]:

64

$$(Y)_p - (X)_n - (B)_m - A \qquad\qquad [I]$$

wherein
A is a polypeptide of the formula [Ia] in which Thr and His in the 66th and 67th positions from the N-terminus are replaced by His, Thr or Tyr,
m and n are 0, and
Y and p are the same as defined in claim 10.

## Revendications

1. ADN ayant ou contenant une séquence de bases correspondant au polypeptide de facteur humain de nécrose tumorale, à son polypeptide précurseur ou à son polypeptide modifié ou de mutant allélomorphe qui consiste en une séquence d'acides aminés représentée par la formule [I] suivante :

$$(Y)_p - (X)_n - (B)_m - A \qquad\qquad [I]$$

dans laquelle
A est un polypeptide de la formule [Ia] ci-dessous dans laquelle un à douze acides aminés situés à l'extrémité N-terminale peuvent être supprimés, ou bien un ou deux acides aminés peuvent être remplacés par un ou des acides aminés différents,
B est un peptide de la formule [Ib] ci-dessous dans laquelle un à trois acides aminés peuvent être supprimés ou remplacés par un ou des acides aminés différents,
X est un polypeptide,
Y est Met, et
m, n et p sont chacun 1 ou 0 ; la formule [Ia] étant comme suit :

```
Thr Pro Ser Asp Lys Pro Val Ala His Val
Val Ala Asn Pro Gln Ala Glu Gly Gln Leu
Gln Trp Leu Asn Arg Arg Ala Asn Ala Leu
Leu Ala Asn Gly Val Glu Leu Arg Asp Asn
Gln Leu Val Val Pro Ser Glu Gly Leu Tyr
Leu Ile Tyr Ser Gln Val Leu Phe Lys Gly
Gln Gly Cys Pro Ser Thr His Val Leu Leu
Thr His Thr Ile Ser Arg Ile Ala Val Ser
Tyr Gln Thr Lys Val Asn Leu Leu Ser Ala
Ile Lys Ser Pro Cys Gln Arg Glu Thr Pro
Glu Gly Ala Glu Ala Lys Pro Trp Tyr Glu


Pro Ile Tyr Leu Gly Gly Val Phe Gln Leu
Glu Lys Gly Asp Arg Leu Ser Ala Glu Ile
Asn Arg Pro Asp Tyr Leu Asp Phe Ala Glu
Ser Gly Gln Val Tyr Phe Gly Ile Ile Ala
Leu                              ... [Ia] ;
```

et la formule [Ib] étant comme suit :

```
Ser-Ser-Ser-Arg        ... [Ib] ;
```

et le polypeptide représenté par la formule B-A ci-dessus ayant les caractéristiques fonctionnelles d'un facteur de nécrose humain mature.

2. ADN selon la revendication 1 ayant ou contenant une séquence de bases correspondant à une séquence d'acides aminés représentée par la formule [I] suivante :

$$(Y)_p-(X)_n-(B)_m-A \qquad [I]$$

dans laquelle A, B, Y, m, n et p sont tels que définis dans la revendication 1, et X est un polypeptide de formule [Ic] :

```
Ser Thr Glu Ser Met Ile Arg Asp Val Glu
Leu Ala Glu Glu Ala Leu Pro Lys Lys Thr
Gly Gly Pro Gln Gly Ser Arg Arg Cys Leu
Phe Leu Ser Leu Phe Ser Phe Leu Ile Val
Ala Gly Ala Thr Thr Leu Phe Cys Leu Leu
His Phe Gly Val Ile Gly Pro Gln Arg Glu
Glu Phe Pro Arg Asp Leu Ser Leu Ile Ser
Pro Leu Ala Gln Ala Val Arg        ... [Ic] ;
```

et le polypeptide représenté par la formule Y-X-B-A ci-dessus ayant les caractéristiques fonctionnelles d'un polypeptide précurseur de facteur humain de nécrose tumorale.

3. ADN selon la revendication 1 ayant ou contenant une séquence de bases correspondant au polypeptide précurseur de facteur humain de nécrose tumorale qui consiste en une séquence d'acides aminés représentée par la formule [I] suivante :

$$(Y)_p-(X)_n-(B)_m-A \qquad [I]$$

ou à son polypeptide de mutant allélomorphe, formule dans laquelle
A est un polypeptide de la formule [Ia] donnée dans la revendication 1,
B est un peptide de la formule [Ib] donnée dans la revendication 1,
Y est tel que défini dans la revendication 1,
X est un polypeptide de la formule [Ic] donnée dans la revendication 2, et
m, n et p sont chacun 1.

4. ADN ayant ou contenant une séquence de bases correspondant au polyptide de facteur humain de nécrose tumorale mature qui consiste en une séquence d'acides aminés représentée par la formule [I] suivante :

$$(Y)_p-(X)_n-(B)_m-A \qquad [I]$$

ou à son polypeptide de mutant allélomorphe, formule dans laquelle
A est un polypeptide de la formule [Ia] donnée dans la revendication 1,
B est un peptide de la formule [Ib] donnée dans la revendication 1,
m est 1, n est 0, et
Y et p sont tels que définis dans la revendication 1.

5. ADN ayant ou contenant une séquence de bases correspondant au polypeptide modifié de facteur humain de nécrose tumorale qui consiste en une séquence d'acides aminés représentée par la formule [I] suivante :

$$(Y)_p-(X)_n-(B)_m-A \qquad [I]$$

dans laquelle

A est un polypeptide de la formule [Ia] donnée dans la revendication 1,

n et m sont chacun 0, et

Y et p sont tels que définis dans la revendication 1.

6. ADN ayant ou contenant une séquence de bases correspondant au polypeptide modifié de facteur humain de nécrose tumorale qui consiste en une séquence d'acides aminés représentée par la formule [II suivante :

$$(Y)_p-(X)_n-(B)_m-A \qquad [I]$$

dans laquelle

A est un polypeptide de la formule [Ia] dans laquelle un à douze acides aminés sont supprimés, ou bien un ou deux acides aminés sont remplacés par un ou des acides aminés différents,

m et n sont chacun 0, et

Y et p sont tels que définis dans la revendication 1.

7. ADN ayant ou contenant une séquence de bases correspondant au polypeptide modifié de facteur humain de nécrose tumorale qui consiste en une séquence d'acides aminés représentée par la formule [I] suivante :

$$(Y)_p-(X)_n-(B)_m-A \qquad [I]$$

dans laquelle

A est un polypeptide de la formule [Ia] dans laquelle sont supprimés : le Thr situé à la première position de l'extrémité N-terminale, les acides aminés allant du Thr situé à la première position ci-dessus au Pro situé à la 6ème position, les acides aminés allant du Thr situé à la première position ci-dessus au His situé à la 9ème position, ou les acides aminés allant du Thr situé à la première position ci-dessus à l'Ala situé à la 12ème position,

m et n sont chacun 0, et

Y et p sont tels que définis dans la revendication 1.

8. ADN ayant ou contenant une séquence de bases correspondant au polypeptide modifié de facteur humain de nécrose tumorale qui consiste en une séquence d'acides aminés représentée par la formule [I] suivante :

$$(Y)_p-(X)_n-(B)_m-A \qquad [I]$$

dans laquelle

A est un polypeptide de la formule [Ia] dans laquelle Thr et His situés aux 66ème et 67ème positions à partir de l'extrémité N-terminale sont remplacés par His. Thr ou Tyr,

m et n sont chacun 0, et

Y et p sont tels que définis dans la revendication 1.

9. ADN selon la revendication 2 ayant ou contenant une séquence de bases représentée par la formule [II] suivante :

$$(Y')_p-(X')_n-(B')_m-A' \qquad [II]$$

dans laquelle

A' est une séquence de bases de la formule [IIa] ci-dessous dans laquelle un à douze codons situés à l'extrémité 5' peuvent être supprimes, ou bien un ou deux codons peuvent être remplacés par un ou des codons différents,

B' est une séquence de bases de la formule [IIb] ci-dessous dans laquelle un à trois codons peuvent être supprimés ou remplacés par un ou des codons différents,

X' est un séquence de bases de la formule [IIc] ci-dessous,

Y' est ATG, et

m, n et p sont chacun 1 ou 0 ; la formule [IIa] étant comme suit :

```
(5')-ACC CCG AGT GAC AAG CCT GTA GCC

CAT GTT GTA GCA AAC CCT CAA GCT GAG GGG

CAG CTC CAG TGG CTG AAC CGC CGG GCC AAT


GCC CTC CTG GCC AAT GGC GTG GAG CTG AGA

GAT AAC CAG CTG GTG GTG CCA TCA GAG GGC

CTG TAC CTC ATC TAC TCC CAG GTC CTC TTC

AAG GGC CAA GGC TGC CCC TCC ACC CAT GTG

CTC CTC ACC CAC ACC ATC AGC CGC ATC GCC

GTC TCC TAC CAG ACC AAG GTC AAC CTC CTC

TCT GCC ATC AAG AGC CCC TGC CAG AGG GAG

ACC CCA GAG GGG GCT GAG GCC AAG CCC TGG

TAT GAG CCC ATC TAT CTG GGA GGG GTC TTC

CAG CTG GAG AAG GGT GAC CGA CTC AGC GCT

GAG ATC AAT CGG CCC GAC TAT CTC GAC TTT

GCC GAG TCT GGG CAG GTC TAC TTT GGG ATC

ATT GCC CTG-(3')                    ... [IIa],
```

la formule [IIb] étant comme suit :

```
(5')-TCA-TCT-TCT-CGA-(3')      [IIb],
```

la formule [IIc] étant comme suit :

```
(5')-AGC ACT GAA AGC ATG ATC CGG GAC

GTG GAG CTG GCC GAG GAG GCG CTC CCC AAG

AAG ACA GGG GGG CCC CAG GGC TCC AGG CGG

TGC TTG TTC CTC AGC CTC TTC TCC TTC CTG

ATC GTG GCA GGC GCC ACC ACG CTC TTC TGC

CTG CTG CAC TTT GGA GTG ATC GGC CCC CAG

AGG GAA GAG TTC CCC AGG GAC CTC TCT CTA

ATC AGC CCT CTG GCC CAG GCA GTC AGA-(3')

                                    ... [IIc].
```

**10.** ADN selon la revendication 3 ayant ou contenant une séquence de bases représentée par la formule [II] suivante

$$(Y')_p - (X')_n - (B')_m - A' \qquad [II]$$

dans laquelle

A' est une séquence de bases de la formule [IIa] donnée dans la revendication 9,

B' est une séquence de bases de la formule [IIb] donnée dans la revendication 9,

m, n et p sont chacun 1, et

X' et Y' sont tels que définis dans la revendication 9.

**11.** ADN selon la revendication 4 ayant ou contenant une séquence de bases représentée par la formule [II] suivante :

$$(Y')_p - (X')_n - (B')_m - A' \qquad [II]$$

dans laquelle

A' est une séquence de bases de la formule [IIa] donnée dans la revendication 9.

B' est une séquence de bases de la formule [IIb] donnée dans la revendication 9.

m est 1

n est 0, et

Y' et p sont tels que définis dans la revendication 9.

**12.** ADN selon la revendication 5 ayant ou contenant une séquence de bases représentée par la formule [II] suivante :

$$(Y')_p - (X')_n - (B')_m - A' \qquad [II]$$

dans laquelle

A' est une séquence de bases de la formule [IIa] donnée dans la revendication 9.

m et n sont chacun 0, et

Y' et p sont tels que définis dans la revendication 9.

**13.** ADN selon la revendication 6 ayant ou contenant une séquence de bases représentée par la formule [II] suivante :

69

EP 0 155 549 B1

$$(Y')_p-(X')_n-(B')_m-A' \qquad [II]$$

dans laquelle
A' est une séquence de bases de la formule [IIa] dans laquelle un à douze codons situés à l'extré mité 5' sont supprimés, ou bien un ou deux codons sont remplacés par un ou des codons différents, m et n sont chacun 0, et
Y' et p sont tels que définis dans la revendication 9.

14. ADN selon la revendication 7 ayant ou contenant une séquence de bases représentée par la formule [II] suivante :

$$(Y')_p-(X')_n-(B')_m-A' \qquad [II]$$

dans laquelle
A' est une séquence de bases de la formule [IIa] dans laquelle sont supprimés : l'ACC du premier codon de l'extrémité 5', les codons allant de l'ACC du premier codon ci-dessus au CCT du 6ème codon, les codons allant de l'ACC du premier codon ci-dessus au CAT du 9ème codon, ou les codons allant de l'ACC du premier codon ci-dessus au GCA du 12ème codon,
m et n sont chacun 0, et
Y' et p sont tels que définis dans la revendication 9.

15. ADN selon la revendication 9 ayant ou contenant une séquence de bases représentée par la formule [II] suivante :

$$(Y')_p-(X')_n-(B')_m-A' \qquad [II]$$

dans laquelle
A' est une séquence de bases de la formule [IIa] dans laquelle l'ACC et le CAT des 66ème et 67ème codons à partir de l'extrémité 5' sont remplacés par CAT, ACC ou TAC,
m et n sont chacun 0, et
Y' et p sont tels que définis dans la revendication 9.

16. Vecteur dans lequel est inséré l'ADN de l'une quelconque des revendications précédentes.

17. Vecteur selon la revendication 16, qui est un vecteur d'expression.

18. Vecteur d'expression selon la revendication 17, qui comprend une séquence de bases correspondant à une séquence d'acides aminés représentée par la formule [I] suivante :

$$(Y)_p-(X)_n-(B)_m-A \qquad [I]$$

dans laquelle
p est 1, et
A, B, X, Y, m et n sont tels que définis dans la revendication 1, et comportant un codon de terminaison situé à l'extrémité 3' de ladite séquence de bases, dans lequel ladite séquence de bases est sous le contrôle d'un promoteur de transcription.

19. Vecteur d'expression selon la revendication 18, dans lequel le promoteur de transcription est un promoteur trp ou un promoteur tac.

70

**20.** Vecteur d'expression selon la revendication 18, dans lequel la séquence de bases correspondant à la séquence d'acides aminés de formule [I] telle que définie dans la revendication 18 et comportant un codon de terminaison situé à l'extrémité 3' de ladite séquence de bases est reliée dans le cadre de lecture de traduction correct à une seconde séquence de bases codant pour une protéine devant être fusionnée.

**21.** Vecteur d'expression selon la revendication 20, dans lequel la seconde séquence de bases codant pour une protéine devant être fusionnée est un gène phoS .

**22.** Vecteur selon les revendications 16 à 21, qui peut proliférer chez Escherichia coli.

**23.** Vecteur selon la revendication 22, qui est un plasmide d'Escherichia coli.

**24.** Vecteur selon la revendication 23, qui est pBR322.

**25.** Hôte transformé par un vecteur des revendications 16 à 24.

**26.** Hôte selon la revendication 25, qui est un micro-organisme.

**27.** Hôte selon la revendication 26, qui est Escherichia coli.

**28.** Polypeptide ayant ou contenant un polypeptide de facteur humain de nécrose tumorale, son polypeptide précurseur ou son polypeptide modifié ou de mutant allélomorphe qui consiste en une séquence d'acides aminés représentée par la formule [I] suivante :

$$(Y)_p - (X)_n - (B)_m - A \qquad\qquad [I]$$

ou l'un de ses dérivés ou sels, formule dans laquelle
A est un polypeptide de la formule [Ia] ci-dessous dans laquelle un à douze acides aminés peuvent être supprimés, ou bien un ou deux acides aminés peuvent être remplacés par un ou des acides aminés différents,
B est un peptide de la formule [Ib] ci-dessous dans laquelle un à trois acides aminés peuvent être supprimés ou remplacés par un ou des acides aminés différents,
X est un polypeptide,
Y est Met, et
m, n et p sont respectivement 1 ou 0 : la formule [Ia] étant comme suit :

```
Thr Pro Ser Asp Lys Pro Val Ala His Val
Val Ala Asn Pro Gln Ala Glu Gly Gln Leu
Gln Trp Leu Asn Arg Arg Ala Asn Ala Leu
Leu Ala Asn Gly Val Glu Leu Arg Asp Asn
Gln Leu Val Val Pro Ser Glu Gly Leu Tyr
Leu Ile Tyr Ser Gln Val Leu Phe Lys Gly
```

```
Gln Gly Cys Pro Ser Thr His Val Leu Leu
Thr His Thr Ile Ser Arg Ile Ala Val Ser
Tyr Gln Thr Lys Val Asn Leu Leu Ser Ala
Ile Lys Ser Pro Cys Gln Arg Glu Thr Pro
Glu Gly Ala Glu Ala Lys Pro Trp Tyr Glu
Pro Ile Tyr Leu Gly Gly Val Phe Gln Leu
Glu Lys Gly Asp Arg Leu Ser Ala Glu Ile
Asn Arg Pro Asp Tyr Leu Asp Phe Ala Glu
Ser Gly Gln Val Tyr Phe Gly Ile Ile Ala
Leu                               ... [Ia] ;
```

et la formule [Ib] étant comme suit :

$$Ser-Ser-Ser-Arg \qquad ... [Ib] ;$$

et le polypeptide représenté par la formule B-A ci-dessus ayant les caractéristiques fonctionnelles d'un facteur de nécrose humain mature.

**29.** Polypeptide selon la revendication 28, representé par la formule [I] suivante :

$$(Y)_p - (X)_n - (B)_m - A \qquad [I]$$

ou l'un de ses dérivés ou sels, formule dans laquelle

A, B, Y, m, n et p sont tels que définis dans la revendication 28, et X est un polypeptide de la formule [Ic] suivante :

```
Ser Thr Glu Ser Met Ile Arg Asp Val Glu
Leu Ala Glu Glu Ala Leu Pro Lys Lys Thr
Gly Gly Pro Gln Gly Ser Arg Arg Cys Leu
Phe Leu Ser Leu Phe Ser Phe Leu Ile Val
Ala Gly Ala Thr Thr Leu Phe Cys Leu Leu
His Phe Gly Val Ile Gly Pro Gln Arg Glu
Glu Phe Pro Arg Asp Leu Ser Leu Ile Ser
Pro Leu Ala Gln Ala Val Arg      ... [Ic] ;
```

et le polypeptide représenté par la formule Y-X-B-A ci-dessus ayant les caractéristiques fonctionnelles d'un polypeptide précurseur de facteur humain de nécrose tumorale.

**30.** Polypeptide ayant ou contenant un polypeptide précurseur de facteur humain de nécrose tumorale qui consiste en une séquence d'acides aminés représentée par la formule [II suivante :

$$(Y)_p - (X)_n - (B)_m - A \qquad [I]$$

dans laquelle

72

A est un polypeptide de la formule [Ia] donnée dans la revendication 28,
B est un peptide de la formule [Ib] donnée dans la revendication 28,
X est un polypeptide de la formule [Ic] donnée dans la revendication 29,
m et n sont chacun 1, et
Y et p sont tels que définis dans la revendication 28.

**31.** Polypeptide ayant ou contenant un polypeptide de facteur humain de nécrose tumorale mature qui consiste en une séquence d'acides aminés représentée par la formule [I] suivante :

$$(Y)_p - (X)_n - (B)_m - A \qquad [I]$$

dans laquelle
A est un polypeptide de la formule [Ia] donnée dans la revendication 28,
B est un peptide de la formule [Ib] donnée dans la revendication 28,
m est 1,
n est 0, et
Y et p sont tels que définis dans la revendication 28.

**32.** Polypeptide ayant ou contenant un polypeptide modifié de facteur humain de nécrose tumorale qui consiste en une séquence d'acides aminés représentée par la formule [I] suivante :

$$(Y)_p - (X)_n - (B)_m - A \qquad [I]$$

dans laquelle
A est un polypeptide de la formule [Ia] donnée dans la revendication 28,
m et n sont chacun 0, et
Y et p sont tels que définis dans la revendication 28.

**33.** Polypeptide selon la revendication 31, représenté par la formule [I] suivante :

$$(Y)_p - (X)_n - (B)_m - A \qquad [I]$$

dans laquelle
A est un polypeptide de la formule [Ia] donnée dans la revendication 28,
B est un peptide de la formule [Ib] donné dans la revendication 28,
m est 1, et
n et p sont 0.

**34.** Polypeptide selon la revendication 32, representé par la formule [I] suivante :

$$(Y)_p - (X)_n - (B)_m - A \qquad [I]$$

dans laquelle
A est un polypeptide de la formule [Ia] donnée dans la revendication 28, et
m, n et p sont chacun 0.

**35.** Polypeptide ayant ou contenant un polypeptide modifié de facteur humain de nécrose tumorale qui consiste en une séquence d'acides aminés représentée par la formule [I] suivante :

$$(Y)_p - (X)_n - (B)_m - A \qquad [I]$$

dans laquelle

A est un polypeptide de la formule [Ia] dans laquelle un à douze acides aminés sont supprimés, ou bien un ou deux acides aminés sont remplacés par un ou des acides aminés différents,

m et n sont chacun 0, et

Y et p sont tels que définis dans la revendication 28.

36. Polypeptide selon la revendication 35, représenté par la formule [I] suivante :

$$(Y)_p - (X)_n - (B)_m - A \qquad [I]$$

dans laquelle

A est un polypeptide de la formule [Ia] dans laquelle sont supprimés : le Thr situé à la première position de l'extrémité N-terminale, les acides aminés allant du Thr situé à la première position ci-dessus au Pro situé à la 6ème position, les acides aminés allant du Thr situé à la première position ci-dessus au His situé à la 9ème position, ou les acides aminés allant du Thr situé à la première position ci-dessus à l'Ala situé à la 12ème position,

m et n sont chacun 0, et

Y et p sont tels que définis dans la revendication 28.

37. Polypeptide selon la revendication 35, représenté par la formule [I] suivante :

$$(Y)_p - (X)_n - (B)_m - A \qquad [I]$$

dans laquelle

A est un polypeptide de la formule [Ia] dans laquelle Thr et His situés aux 66ème et 67ème positions à partir de l'extrémité N-terminale sont remplacés par His, Thr ou Tyr,

m et n sont chacun 0, et

Y et p sont tels que définis dans la revendication 28.

38. Substance résultant de la modifiation chimique ou enzymatique d'un polypeptide revendiqué dans l'une quelconque des revendications 30 à 37.

39. Sel pharmaceutiquement acceptable d'un polypeptide revendiqué dans l'une quelconque des revendications 30 à 38.

40. Agrégat constitué d'un polypeptide revendiqué dans l'une quelconque des revendications 30 à 38 formant un sous-unité.

41. Agrégat selon la revendication 40, qui est un trimère.

42. Trimère selon la revendication 41, qui est constitué d'un polypeptide revendiqué dans la revendication 33 formant une sous-unité.

43. Préparation pharmaceutique contenant une substance revendiquée dans l'une quelconque des revendications 29 à 42 en tant qu'ingrédient actif.

44. Utilisation de l'une quelconque des substances revendiquées dans les revendications 29 à 43 en tant qu'agent antitumoral.

45. Procédé pour la production d'un ADN ayant ou contenant une séquence de bases correspondant à une séquence d'acides aminés représentée par la formule [I] suivante :

$$(Y)_p - (X)_n - (B)_m - A \qquad [I]$$

dans laquelle

A, B, X, Y, m, n et p sont tels que définis dans la revendication 1, qui comprend

(1) la culture de macrophages humains avec un ou plusieurs inducteurs,

(2) la séparation d'une fraction contenant un ARNm de facteur humain de nécrose tumorale à partir des macrophages,

(3) la préparation d'un ADNc monocaténaire à partir de l'ARNm en utilisant la transcriptase inverse, puis sa conversion en un ADNc bicaténaire,

(4) l'insertion de l'ADNc bicaténaire dans un vecteur,

(5) l'introduction du vecteur recombinant dans un hôte pour transformer celui-ci et construire une librairie d'ADNc,

(6) le clonage de l'ADNc codant pour le polypeptide de facteur humain de nécrose tumorale ou son polypeptide de mutant allélomorphe à partir de la librairie, et

(7) si cela est souhaité, la modification de l' ADNc cloné.

**46.** Procédé selon la revendication 45, qui est destiné à la production d'un ADN ayant ou contenant une séquence de bases représentée par la formule [II] suivante :

$$(Y')_p - (X')_n - (B')_m - A' \qquad [II]$$

dans laquelle

A' , B', X', Y', m, n et p sont tels que définis dans la revendication 9.

**47.** Procédé pour la production d'un polypeptide représenté par la formule [I] suivante :

$$(Y)_p - (X)_n - (B)_m - A \qquad [I]$$

ou un dérivé ou sel de celui-ci, formule dans laquelle

A, B, X, Y, m, n et p sont tels que définis dans la revendication 28, qui comprend l'insertion, dans un vecteur d'expression, d'un ADN ayant ou contenant une séquence de bases codant pour le polypeptide ci-dessus, la transformation d'un hôte avec le vecteur recombinant, la culture de l'hôte et la collecte du polypeptide résultant et, si cela est souhaité, la modification du polypeptide.

Revendications pour l'Etat Contractant suivant: AT

**1.** Procédé pour la production d'un ADN ayant ou contenant une séquence de bases correspondant au polypeptide de facteur humain de nécrose tumorale, à son polypeptide précurseur ou à son polypeptide modifié ou de mutant allélomorphe qui consiste en une séquence d'acides aminés représentée par la formule [I] suivante :

$$(Y)_p - (X)_n - (B)_m - A \qquad [I]$$

dans laquelle

A est un polypeptide de la formule [Ia] ci-dessous dans laquelle un à douze acides aminés situés à l'extrémité N-terminale peuvent être supprimés, ou bien un ou deux acides aminés peuvent être remplacés par un ou des acides aminés différents,

B est un peptide de la formule [Ib] ci-dessous dans laquelle un à trois acides aminés peuvent être supprimés ou remplacés par un ou des acides aminés différents,

X est un polypeptide,

Y est Met, et m, n et p sont chacun I ou 0 ; la formule [Ia] étant comme suit :

```
Thr Pro Ser Asp Lys Pro Val Ala His Val
Val Ala Asn Pro Gln Ala Glu Gly Gln Leu
Gln Trp Leu Asn Arg Arg Ala Asn Ala Leu
Leu Ala Asn Gly Val Glu Leu Arg Asp Asn
Gln Leu Val Val Pro Ser Glu Gly Leu Tyr
Leu Ile Tyr Ser Gln Val Leu Phe Lys Gly
Gln Gly Cys Pro Ser Thr His Val Leu Leu
Thr His Thr Ile Ser Arg Ile Ala Val Ser
Tyr Gln Thr Lys Val Asn Leu Leu Ser Ala
Ile Lys Ser Pro Cys Gln Arg Glu Thr Pro
Glu Gly Ala Glu Ala Lys Pro Trp Tyr Glu
```

```
Pro Ile Tyr Leu Gly Gly Val Phe Gln Leu
Glu Lys Gly Asp Arg Leu Ser Ala Glu Ile
Asn Arg Pro Asp Tyr Leu Asp Phe Ala Glu
Ser Gly Gln Val Tyr Phe Gly Ile Ile Ala
Leu                             ... [Ia] ;
```

et la formule [Ib] étant comme suit :

```
Ser-Ser-Ser-Arg         ... [Ib] ;
```

et le polypeptide représenté par la formule B-A ci-dessus ayant les caractéristiques fonctionnelles d'un facteur de nécrose humain mature. qui comprend

(1) la culture de macrophages humains avec un ou plusieurs inducteurs,

(2) la séparation d'une fraction contenant un ARNm de facteur humain de nécrose tumorale à partir des macrophages,

(3) la préparation d'un ADNc monocaténaire à partir de l'ARNm en utilisant la transcriptase inverse, puis sa conversion en un ADNc bicaténaire,

(4) l'insertion de l'ADNc bicaténaire dans un vecteur,

(5) l'introduction du vecteur recombinant dans un hôte pour transformer celui-ci et construire une librairie d'ADNc,

(6) le clonage de l'ADNc codant pour le polypeptide de facteur humain de nécrose tumorale ou son polypeptide de mutant allélomorphe à partir de la librairie. et

(7) si cela est souhaité, la modification de l'ADNc cloné.

2. Procédé selon la revendication 1. qui est destiné à la production d'un ADN ayant ou contenant une séquence de bases correspondant à une séquence d'acides aminés représentée par la formule [I] suivante :

$$(Y)_p-(X)_n-(B)_m-A \qquad [I]$$

dans laquelle

A. B, Y, m, n et p sont tels que définis dans la revendication 1. et X est un polypeptide de formule [Ic] :

76

```
Ser Thr Glu Ser Met Ile Arg Asp Val Glu
Leu Ala Glu Glu Ala Leu Pro Lys Lys Thr
Gly Gly Pro Gln Gly Ser Arg Arg Cys Leu
Phe Leu Ser Leu Phe Ser Phe Leu Ile Val
Ala Gly Ala Thr Thr Leu Phe Cys Leu Leu
His Phe Gly Val Ile Gly Pro Gln Arg Glu
Glu Phe Pro Arg Asp Leu Ser Leu Ile Ser
Pro Leu Ala Gln Ala Val Arg        ... [Ic] ;
```

et le polypeptide représenté par la formule Y-X-B-A ci-dessus ayant les caractéristiques fonctionnelles d'un polypeptide précurseur de facteur humain de nécrose tumorale.

3. Procédé selon la revendication 1, qui est destiné à la production d'un ADN ayant ou contenant une séquence de bases correspondant au polypeptide précurseur de facteur humain de nécrose tumorale qui consiste en une séquence d'acides aminés représentée par la formule [I] suivante :

$$(Y)_p - (X)_n - (B)_m - A \qquad [I]$$

ou à son polypeptide de mutant allélomorphe, formule dans laquelle
  A est un polypeptide de la formule [Ia] donnée dans la revendication 1,
  B est un peptide de la formule [Ib] donnée dans la revendication 1,
  Y est tel que défini dans la revendication 1,
  X est un polypeptide de la formule [Ic] donnée dans la revendication 2, et
  m, n et p sont chacun 1.

4. Procédé selon la revendication 1, qui est destiné à la production d'un ADN ayant ou contenant une séquence de bases correspondant au polyptide de facteur humain de nécrose tumorale mature qui consiste en une séquence d'acides aminés représentée par la formule [I] suivante :

$$(Y)_p - (X)_n - (B)_m - A \qquad [I]$$

ou à son polypeptide de mutant allélomorphe, formule dans laquelle
  A est un polypeptide de la formule [Ia] donnée dans la revendication 1.
  B est un peptide de la formule [Ib] donnée dans la revendication 1,
  m est 1, n est 0, et
  Y et p sont tels que définis dans la revendication 1.

5. Procédé selon la revendication 1, qui est destiné à la production d'un ADN ayant ou contenant une séquence de bases correspondant au polypeptide modifié de facteur humain de nécrose tumorale qui consiste en une séquence d'acides aminés représentée par la formule [I] suivante :

$$(Y)_p - (X)_n - (B)_m - A \qquad [I]$$

dans laquelle
  A est un polypeptide de la formule [Ia] donnée dans la revendication 1.
  n et m sont chacun 0, et
  Y et p sont tels que définis dans la revendication 1.

6. Procédé selon la revendication 1. qui est destiné à la production d'un ADN ayant ou contenant une

77

séquence de bases correspondant au polypeptide modifié de facteur humain de nécrose tumorale qui consiste en une séquence d'acides aminés représentée par la formule [I] suivante :

$$(Y)_p - (X)_n - (B)_m - A \qquad [I]$$

dans laquelle

A est un polypeptide de la formule [Ia] dans laquelle un à douze acides aminés sont supprimés, ou bien un ou deux acides aminés sont remplacés par un ou des acides aminés différents,

m et n sont chacun 0, et

Y et p sont tels que définis dans la revendication 1.

7. Procédé selon la revendication 1, qui est destiné à la production d'un ADN ayant ou contenant une séquence de bases correspondant au polypeptide modifié de facteur humain de nécrose tumorale qui consiste en une séquence d'acides aminés représentée par la formule [I] suivante :

$$(Y)_p - (X)_n - (B)_m - A \qquad [I]$$

dans laquelle

A est un polypeptide de la formule [Ia] dans laquelle sont supprimés : le Thr situé à la première position de l'extrémité N-terminale, les acides aminés allant du Thr situé à la première position ci-dessus au Pro situé à la 6ème position, les acides aminés allant du Thr situé à la première position ci-dessus au His situé à la 9ème position, ou les acides aminés allant du Thr situé à la première position ci-dessus à l'Ala situé à la 12ème position,

m et n sont chacun 0, et

Y et p sont tels que définis dans la revendication 1.

8. Procédé selon la revendication 1, qui est destiné à la production d'un ADN ayant ou contenant une séquence de bases correspondant au polypeptide modifié de facteur humain de nécrose tumorale qui consiste en une séquence d'acides aminés représentée par la formule [I] suivante :

$$(Y)_p - (X)_n - (B)_m - A \qquad [I]$$

dans laquelle

A est un polypeptide de la formule [Ia] dans laquelle Thr et His situés aux 66ème et 67ème positions à partir de l'extrémité N-terminale sont remplacés par His, Thr ou Tyr,

m et n sont chacun 0, et

Y et p sont tels que définis dans la revendication 1.

9. Procédé selon la revendication 1, qui est destiné à la production d'un ADN selon la revendication 2 ayant ou contenant une séquence de bases représentée par la formule [II] suivante :

$$(Y')_p - (X')_n - (B')_m - A' \qquad [II]$$

dans laquelle

A' est une séquence de bases de la formule [IIa] ci-dessous dans laquelle un à douze codons situés à l'extrémité 5' peuvent être supprimés, ou bien un ou deux codons peuvent être remplacés par un ou des codons différents,

B' est une séquence de bases de la formule [IIb] ci-dessous dans laquelle un à trois codons peuvent être supprimés ou remplacés par un ou des codons différents,

X' est un séquence de bases de la formule [IIc] ci-dessous,

Y' est ATG, et

78

m, n et p sont chacun 1 ou 0 ; la formule [IIa] étant comme suit :

```
(5')-ACC CCG AGT GAC AAG CCT GTA GCC
     CAT GTT GTA GCA AAC CCT CAA GCT GAG GGG
     CAG CTC CAG TGG CTG AAC CGC CGG GCC AAT
     GCC CTC CTG GCC AAT GGC GTG GAG CTG AGA
     GAT AAC CAG CTG GTG GTG CCA TCA GAG GGC

     CTG TAC CTC ATC TAC TCC CAG GTC CTC TTC
     AAG GGC CAA GGC TGC CCC TCC ACC CAT GTG
     CTC CTC ACC CAC ACC ATC AGC CGC ATC GCC
     GTC TCC TAC CAG ACC AAG GTC AAC CTC CTC
     TCT GCC ATC AAG AGC CCC TGC CAG AGG GAG
     ACC CCA GAG GGG GCT GAG GCC AAG CCC TGG
     TAT GAG CCC ATC TAT CTG GGA GGG GTC TTC
     CAG CTG GAG AAG GGT GAC CGA CTC AGC GCT
     GAG ATC AAT CGG CCC GAC TAT CTC GAC TTT
     GCC GAG TCT GGG CAG GTC TAC TTT GGG ATC
     ATT GCC CTG-(3')              ... [IIa],
```

la formule [IIb] étant comme suit :

```
     (5')-TCA-TCT-TCT-CGA-(3')        [IIb],
```

la formule [IIc] étant comme suit :

```
(5')-AGC ACT GAA AGC ATG ATC CGG GAC
     GTG GAG CTG GCC GAG GAG GCG CTC CCC AAG
     AAG ACA GGG GGG CCC CAG GGC TCC AGG CGG
     TGC TTG TTC CTC AGC CTC TTC TCC TTC CTG
     ATC GTG GCA GGC GCC ACC ACG CTC TTC TGC
     CTG CTG CAC TTT GGA GTG ATC GGC CCC CAG
     AGG GAA GAG TTC CCC AGG GAC CTC TCT CTA
     ATC AGC CCT CTG GCC CAG GCA GTC AGA-(3')
                                  ... [IIc].
```

10. Procédé pour la production d'un polypeptide ayant ou contenant un polypeptide de facteur humain de nécrose tumorale, son polypeptide précurseur ou son polypeptide modifié ou de mutant allélomorphe qui consiste en une séquence d'acides aminés représentée par la formule [I] suivante :

$$(Y)_p-(X)_n-(B)_m-A \qquad\qquad [I]$$

ou l'un de ses dérivés ou sels, formule dans laquelle

A est un polypeptide de la formule [Ia] ci-dessous dans laquelle un à douze acides aminés peuvent être supprimés, ou bien un ou deux acides aminés peuvent être remplacés par un ou des acides aminés différents,

B est un peptide de la formule [Ib] ci-dessous dans laquelle un à trois acides aminés peuvent être supprimés ou remplacés par un ou des acides aminés différents,

X est un polypeptide,

Y est Met, et

m, n et p sont respectivement 1 ou 0 ; la formule [Ia] étant comme suit :

```
Thr Pro Ser Asp Lys Pro Val Ala His Val
Val Ala Asn Pro Gln Ala Glu Gly Gln Leu
Gln Trp Leu Asn Arg Arg Ala Asn Ala Leu
Leu Ala Asn Gly Val Glu Leu Arg Asp Asn
Gln Leu Val Val Pro Ser Glu Gly Leu Tyr
Leu Ile Tyr Ser Gln Val Leu Phe Lys Gly
Gln Gly Cys Pro Ser Thr His Val Leu Leu
Thr His Thr Ile Ser Arg Ile Ala Val Ser
Tyr Gln Thr Lys Val Asn Leu Leu Ser Ala
Ile Lys Ser Pro Cys Gln Arg Glu Thr Pro
Glu Gly Ala Glu Ala Lys Pro Trp Tyr Glu
Pro Ile Tyr Leu Gly Gly Val Phe Gln Leu
Glu Lys Gly Asp Arg Leu Ser Ala Glu Ile
Asn Arg Pro Asp Tyr Leu Asp Phe Ala Glu
Ser Gly Gln Val Tyr Phe Gly Ile Ile Ala
Leu                                     ... [Ia] ;
```

et la formule [Ib] étant comme suit :

```
Ser-Ser-Ser-Arg        ... [Ib] ;
```

et le polypeptide représenté par la formule B-A ci-dessus ayant les caractéristiques fonctionnelles d'un facteur de nécrose humain mature.

qui comprend l'insertion, dans un vecteur d'expression, d'un ADN ayant ou contenant une séquence de bases codant pour le polypeptide ci-dessus, la transformation d'un hôte avec le vecteur recombinant, la culture de l'hôte et la collecte du polypeptide résultant et, si cela est souhaité, modifier le polypeptide.

11. Procédé selon la revendication 10, qui est destiné à la production d'un polypeptide ayant ou contenant un polypeptide représenté par la formule [I] suivante :

$$(Y)_p-(X)_n-(B)_m-A \qquad [I]$$

ou l'un de ses dérivés ou sels, formule dans laquelle

A, B, Y, m, n et p sont tels que définis dans la revendication 10, et X est un polypeptide de la formule [Ic] suivante :

```
Ser Thr Glu Ser Met Ile Arg Asp Val Glu
Leu Ala Glu Glu Ala Leu Pro Lys Lys Thr
Gly Gly Pro Gln Gly Ser Arg Arg Cys Leu
Phe Leu Ser Leu Phe Ser Phe Leu Ile Val
Ala Gly Ala Thr Thr Leu Phe Cys Leu Leu
His Phe Gly Val Ile Gly Pro Gln Arg Glu
Glu Phe Pro Arg Asp Leu Ser Leu Ile Ser
Pro Leu Ala Gln Ala Val Arg          ... [Ic] ;
```

et le polypeptide représenté par la formule Y-X-B-A ci-dessus ayant les caractéristiques fonctionnelles d'un polypeptide précurseur de facteur humain de nécrose tumorale.

12. Procédé selon la revendication 10, qui est destiné à la production d'un polypeptide ayant ou contenant un polypeptide précurseur de facteur humain de nécrose tumorale qui consiste en une séquence d'acides aminés représentée par la formule [I] suivante :

$$(Y)_p - (X)_n - (B)_m - A \qquad\qquad [I]$$

dans laquelle

A est un polypeptide de la formule [Ia] donnée dans la revendication 10,
B est un peptide de la formule [Ib] donnée dans la revendication 10,
X est un polypeptide de la formule [Ic] donnée dans la revendication 11,
m et n sont chacun 1, et
Y et p sont tels que définis dans la revendication 10.

13. Procédé selon la revendication 10, qui est destiné à la production d'un polypeptide ayant ou contenant un polypeptide de facteur humain de nécrose tumorale mature qui consiste en une séquence d'acides aminés représentée par la formule [I] suivante :

$$(Y)_p - (X)_n - (B)_m - A \qquad\qquad [I]$$

dans laquelle

A est un polypeptide de la formule [Ia] donnée dans la revendication 10,
B est un peptide de la formule [Ib] donnée dans la revendication 10,
m est 1,
n est 0, et
Y et p sont tels que définis dans la revendication 10.

14. Procédé selon la revendication 10, qui est destiné à la production d'un polypeptide ayant ou contenant un polypeptide modifié de facteur humain de nécrose tumorale qui consiste en une séquence d'acides aminés représentée par la formule [I] suivante :

$$(Y)_p - (X)_n - (B)_m - A \qquad\qquad [I]$$

dans laquelle

A est un polypeptide de la formule [Ia] donnée dans la revendication 10,
m et n sont chacun 0, et
Y et p sont tels que définis dans la revendication 10.

15. Procédé selon la revendication 10, qui est destiné à la production d'un polypeptide révendiqué dans la revendication13 représenté par la formule [I] suivante :

$$(Y)_p - (X)_n - (B)_m - A \qquad [I]$$

dans laquelle
  A est un polypeptide de la formule [Ia] donnée dans la revendication 10,
  B est un peptide de la formule [Ib] donné dans la revendication 10,
  m est 1, et
  n et p sont 0.

16. Procédé selon la revendication 10, qui est destiné à la production d'un polypeptide revendiqué dans la revendication 14 représenté par la formule [I] suivante :

$$(Y)_p - (X)_n - (B)_m - A \qquad [I]$$

dans laquelle
  A est un polypeptide de la formule [Ia] donnée dans la revendication 10, et
  m, n et p sont chacun 0.

17. Procédé selon la revendication 10, qui est destiné à la production d'un polypeptide ayant ou contenant un polypeptide modifié de facteur humain de nécrose tumorale qui consiste en une séquence d'acides aminés représentée par la formule [I] suivante :

$$(Y)_p - (X)_n - (B)_m - A \qquad [I]$$

dans laquelle
  A est un polypeptide de la formule [Ia] dans laquelle un à douze acides aminés sont supprimés, ou bien un ou deux acides aminés sont remplacés par un ou des acides aminés différents,
  m et n sont chacun 0, et
  Y et p sont tels que définis dans la revendication 10.

18. Procédé selon la revendication 10, qui est destiné à la production d'un polypeptide selon la revendication 17 représenté par la formule [I] suivante :

$$(Y)_p - (X)_n - (B)_m - A \qquad [I]$$

dans laquelle
  A est un polypeptide de la formule [Ia] dans laquelle sont supprimés : le Thr situé à la première position de l'extrémité N-terminale, les acides aminés allant du Thr situé à la première position ci-dessus au Pro situé à la 6ème position, les acides aminés allant du Thr situé à la première position ci-dessus au His situé à la 9ème position, ou les acides aminés allant du Thr situé à la première position ci-dessus à l'Ala situé à la 12ème position,
  m et n sont chacun 0, et
  Y et p sont tels que définis dans la revendication 10.

19. Procédé selon la revendication 10, qui est destiné à la production d'un polypeptide revendiqué dans la revendication 17 représenté par la formule [I] suivante :

$$(Y)_p - (X)_n - (B)_m - A \qquad [I]$$

82

dans laquelle
A est un polypeptide de la formule [Ia] dans laquelle Thr et His situés aux 66ème et 67ème positions à partir de l'extrémité N-terminale sont remplacés par His, Thr ou Tyr,
m et n sont chacun 0, et
Y et p sont tels que définis dans la revendication 10.

## Ansprüche

1. DNA, die eine Basensequenz hat oder enthält, die dem humanen Tumor-Nekrosefaktor-Polypeptid, seinem Vorstufen-Polypeptid oder seinem modifizierten oder allelisch mutierten Polypeptid entspricht und eine Aminosäuresequenz enthält, welche durch die folgende Formel [I]:

$$(Y)_p - (X)_n - (B)_m - A \qquad [I]$$

dargestellt wird, worin
A ein Polypeptid der folgenden Formel [Ia] bedeutet, worin eine bis zwölf Aminosäuren an dem N-Terminus nicht vorhanden sein können oder worin eine oder zwei Aminosäuren durch eine andere Aminosäure(n) ersetzt sein kann bzw. können,
B ein Peptid der folgenden Formel [Ib] bedeutet, worin eine bis drei Aminosäuren nicht vorhanden sein kann bzw. können oder durch andere Aminosäure(n) ersetzt sein kann bzw. können,
X ein Polypeptid bedeutet,
Y Met bedeutet und
m, n und p 1 oder 0 bedeuten;
die Formel [Ia] wie folgt ist:

```
Thr Pro Ser Asp Lys Pro Val Ala His Val
Val Ala Asn Pro Gln Ala Glu Gly Gln Leu


Gln Trp Leu Asn Arg Arg Ala Asn Ala Leu
Leu Ala Asn Gly Val Glu Leu Arg Asp Asn
Gln Leu Val Val Pro Ser Glu Gly Leu Tyr
Leu Ile Tyr Ser Gln Val Leu Phe Lys Gly
Gln Gly Cys Pro Ser Thr His Val Leu Leu

Thr His Thr Ile Ser Arg Ile Ala Val Ser
Tyr Gln Thr Lys Val Asn Leu Leu Ser Ala

Ile Lys Ser Pro Cys Gln Arg Glu Thr Pro

Glu Gly Ala Glu Ala Lys Pro Trp Tyr Glu

Pro Ile Tyr Leu Gly Gly Val Phe Gln Leu

Glu Lys Gly Asp Arg Leu Ser Ala Glu Ile

Asn Arg Pro Asp Tyr Leu Asp Phe Ala Glu

Ser Gly Gln Val Tyr Phe Gly Ile Ile Ala

Leu                                 ... [Ia];
```

und die Formel [Ib] wie folgt ist:

$$Ser-Ser-Ser-Arg \qquad \ldots [Ib];$$

und wobei das Polypeptid, das durch die obige Formel B-A dargestellt wird, die funktionellen Eigenschaften als entwickelter humaner Nekrosefaktor aufweist.

2. DNA nach Anspruch 1, die eine Basensequenz hat oder enthält, welche einer Aminosäuresequenz entspricht, die durch die folgende Formel [I]:

$$(Y)_p-(X)_n-(B)_m-A \qquad [I]$$

dargestellt wird, worin

A, B, Y, m, n und p die in Anspruch 1 gegebenen Definitionen besitzen und x ein Polypeptid der Formel [Ic] bedeutet:

```
Ser Thr Glu Ser Met Ile Arg Asp Val Glu

Leu Ala Glu Glu Ala Leu Pro Lys Lys Thr

Gly Gly Pro Gln Gly Ser Arg Arg Cys Leu

Phe Leu Ser Leu Phe Ser Phe Leu Ile Val

Ala Gly Ala Thr Thr Leu Phe Cys Leu Leu

His Phe Gly Val Ile Gly Pro Gln Arg Glu

Glu Phe Pro Arg Asp Leu Ser Leu Ile Ser

Pro Leu Ala Gln Ala Val Arg     ... [Ic],
```

und wobei das Polypeptid, das durch die obige Formel Y-X-B-A dargestellt ist, die funktionellen Eigenschaften eines Vorstufen-Polypeptids des humanen Tumor-Nekrose-faktors aufweist.

3. DNA nach Anspruch 1, die eine Basensequenz hat oder enthält, welche dem humanen Tumor-Nekrosefaktor-Vorstufenpolypeptid, das eine Aminosäuresequenz enthält, welche durch die folgende Formel [I]:

$$(Y)_p-(X)_n-(B)_m-A \qquad [I]$$

dargestellt wird, oder seinem allelisch mutierten Polypeptid entspricht, worin

A ein Polypeptid der Formel [Ia], wie sie in Anspruch 1 dargestellt ist, bedeutet,

B ein Peptid der Formel [Ib], wie sie in Anspruch 1 dargestellt ist, bedeutet,

Y die gleiche Definition wie in Anspruch 1 besitzt,

X ein Polypeptid der Formel [Ic], wie sie in Anspruch 2 dargestellt ist, bedeutet und

m, n und p 1 bedeuten.

4. DNA, die eine Basensequenz hat oder enthält, welche dem ausgereiften humanen Tumor-Nekrosefaktor-Polypeptid, das eine Aminosäuresequenz, welche durch die folgende Formel [I]:

$$(Y)_p - (X)_n - (B)_m - A \qquad [I]$$

dargestellt wird, enthält oder seinem allelisch mutierten Polypeptid entspricht, worin
A ein Polypeptid der Formel [Ia], wie sie in Anspruch 1 dargestellt wird, bedeutet,
B ein Peptid der Formel [Ib], wie sie in Anspruch 1 dargestellt wird, bedeutet,
m 1, n 0 bedeuten und
Y und p die gleichen Definitionen wie in Anspruch 1 besitzen.

5. DNA, die eine Basensequenz hat oder enthält, welche dem modifizierten humanen Tumor-Nekrosefaktor-Polypeptid entspricht, das eine Aminosäuresequenz enthält, die durch die folgende Formel [I]:

$$(Y)_p - (X)_n - (B)_m - A \qquad [I]$$

dargestellt wird, worin
A ein Polypeptid der Formel [Ia], wie sie in Anspruch 1 dargestellt wird, bedeutet,
n und m 0 bedeuten und
Y und p die gleichen Definitionen wie in Anspruch 1 besitzen.

6. DNA, die eine Basensequenz hat oder enthält, die dem modifizierten humanen Tumor-Nekrosefaktor-Polypeptid entspricht, das eine Aminosäuresequenz enthält, welche durch die folgende Formel [I]:

$$(Y)_p - (X)_n - (B)_m - A \qquad [I]$$

dargestellt wird, worin
A ein Polypeptid der Formel [Ia] bedeutet, worin eine bis zwölf Aminosäuren weggelassen sein können oder eine oder zwei Aminosäuren durch eine andere Aminosäure(n) ersetzt sein kann bzw. können,
m und n 0 bedeuten und
Y und p die gleichen Definitionen wie in Anspruch 1 besitzen.

7. DNA, die eine Basensequenz hat oder enthält, die dem modifizierten humanen Tumor-Nekrosefaktor-Polypeptid entspricht, welches eine Aminosäuresequenz enthält, die durch die folgende Formel [I]:

$$(Y)_p - (X)_n - (B)_m - A \qquad [I]$$

dargestellt wird, worin
A ein Polypeptid der Formel [Ia] bedeutet, worin Thr in der ersten Stellung von dem N-Terminus, Aminosäuren von dem obigen Thr in der ersten Stellung zu Pro in der 6. Stellung, Aminosäuren von dem obigen Thr in der ersten Stellung zu His in der 9. Stellung oder Aminosäuren von dem obigen Thr in der ersten Stellung zu Ala in der 12. Stellung nicht vorhanden sind.
m und n 0 bedeuten und
y und p die gleichen Definitionen wie in Anspruch 1 besitzen.

8. DNA, die eine Basensequenz hat oder enthält, die dem modifizierten humanen Tumor-Nekrosefaktor-Polypeptid entspricht, das eine Aminosäuresequenz enthält, die durch die folgende Formel [I]:

$$(Y)_p - (X)_n - (B)_m - A \qquad [I]$$

dargestellt wird, worin

A ein Polypeptid der Formel [Ia] bedeutet, worin Thr und His in den 66. und 67. Stellungen von dem N-Terminus durch His, Thr oder Tyr ersetzt sind,

m und n 0 bedeuten und

Y und p die gleichen Definitionen wie in Anspruch 1 besitzen.

9. DNA nach Anspruch 2, die eine Basensequenz hat oder enthält, welche durch die folgende Formel [II]:

$$(Y')_p - (X')_n - (B')_m - A' \qquad [II]$$

dargestellt wird, worin

A' eine Basensequenz der folgenden Formel [IIa] bedeutet, worin ein bis zwölf Codon(s) an dem 5'-Terminus weggelassen sein kann bzw. können oder worin eine oder zwei Aminosäuren durch einen anderen Codon bzw. andere Codons ersetzt sein kann bzw. können,

B' eine Basensequenz der folgenden Formel [IIb] bedeutet, worin ein bis drei Codon(s) nicht vorhanden sein können oder durch einen anderen Codon bzw. andere Codons ersetzt sein kann bzw. können,

X' eine Basensequenz der folgenden Formel [IIc] bedeutet,

Y' ATG bedeutet und

m, n und p 1 oder 0 bedeuten, wobei die Formel [IIa] wie folgt ist:

```
(5')-ACC CCG AGT GAC AAG CCT GTA GCC

CAT GTT GTA GCA AAC CCT CAA GCT GAG GGG

CAG CTC CAG TGG CTG AAC CGC CGG GCC AAT

GCC CTC CTG GCC AAT GGC GTG GAG CTG AGA

GAT AAC CAG CTG GTG GTG CCA TCA GAG GGC

CTG TAC CTC ATC TAC TCC CAG GTC CTC TTC

AAG GGC CAA GGC TGC CCC TCC ACC CAT GTG

CTC CTC ACC CAC ACC ATC AGC CGC ATC GCC

GTC TCC TAC CAG ACC AAG GTC AAC CTC CTC

TCT GCC ATC AAG AGC CCC TGC CAG AGG GAG

ACC CCA GAG GGG GCT GAG GCC AAG CCC TGG

TAT GAG CCC ATC TAT CTG GGA GGG GTC TTC

CAG CTG GAG AAG GGT GAC CGA CTC AGC GCT

GAG ATC AAT CGG CCC GAC TAT CTC GAC TTT

GCC GAG TCT GGG CAG GTC TAC TTT GGG ATC

ATT GCC CTG-(3')           ... [IIa],
```

die Formel [IIb] wie folgt ist:

```
(5')-TCA-TCT-TCT-CGA-(3')           [IIb],
```

die Formel [IIc] wie folgt ist:

```
(5')-AGC ACT GAA AGC ATG ATC CGG GAC
GTG GAG CTG GCC GAG GAG GCG CTC CCC AAG
AAG ACA GGG GGG CCC CAG GGC TCC AGG CGG
TGC TTG TTC CTC AGC CTC TTC TCC TTC CTG
ATC GTG GCA GGC GCC ACC ACG CTC TTC TGC
CTG CTG CAC TTT GGA GTG ATC GGC CCC CAG
AGG GAA GAG TTC CCC AGG GAC CTC TCT CTA
ATC AGC CCT CTG GCC CAG GCA GTC AGA-(3')
                         ... [IIc].
```

**10.** DNA nach Anspruch 3, die eine Basensequenz hat oder enthält, die durch die folgende Formel [II]:

$$(Y')_p-(X')_n-(B')_m-A' \qquad [II]$$

dargestellt wird, worin
   A' eine Basensequenz der Formel [IIa], die in Anspruch 9 dargestellt ist, bedeutet,
   B' eine Basensequenz der Formel [IIb], die in Anspruch 9 dargestellt ist, bedeutet,
   m, n und p 1 bedeuten und
   X' und Y' die gleichen Definitionen wie in Anspruch 9 besitzen.

**11.** DNA nach Anspruch 4, die eine Basensequenz hat oder enthält, die durch die folgende Formel [II]:

$$(Y')_p-(X')_n-(B')_m-A' \qquad [II]$$

dargestellt wird, worin
   A' eine Basensequenz der Formel [IIa], die in Anspruch 9 dargestellt ist, bedeutet,
   B' eine Basensequenz der Formel [IIb], die in Anspruch 9 dargestellt ist, bedeutet,
   m 1 bedeutet,
   n 0 bedeutet und
   Y' und p die gleichen Definitionen wie in Anspruch 9 besitzen.

**12.** DNA nach Anspruch 5, die eine Basensequenz hat oder enthält, die durch die folgende Formel [II]

$$(Y')_p-(X')_n-(B')_m-A' \qquad [II]$$

dargestellt wird, worin
   A' eine Basensequenz der Formel [IIa], wie sie in Anspruch 9 angegeben wurde, bedeutet,
   m und n 0 bedeuten und
   Y' und p die gleichen Definitionen wie in Anspruch 9 besitzen.

**13.** DNA nach Anspruch 6, die eine Basensequenz hat oder enthält, die durch die folgende Formel [II]:

$$(Y')_p-(X')_n-(B')_m-A' \qquad [II]$$

dargestellt wird, worin

A' eine Basensequenz der Formel [IIa] bedeutet, in der ein bis zwölf Codons an dem 5'-Terminus nicht vorhanden sein können oder worin ein oder zwei Codons durch einen anderen Codon bzw. andere Codons ersetzt sein kann bzw. können,

m und n 0 bedeuten und

Y' und p die gleichen Definitionen wie in Anspruch 9 besitzen.

14. DNA nach Anspruch 7, die eine Basensequenz hat oder enthält, welche durch die folgende Formel [II]:

$$(Y')_p - (X')_n - (B')_m - A' \qquad [II]$$

dargestellt wird, worin

A' eine Basensequenz der Formel [IIa] bedeutet, worin ACC in dem ersten Codon von dem 5'-Terminus, Codons von dem obigen ACC in dem ersten Codon zu CCT in dem 6. Codon, Codons von dem obigen ACC in dem ersten Codon zu CAT in dem 9. Codon oder Codons von dem obigen ACC in dem ersten Codon zu GCA in dem 12. Codon weggelassen sein können,

m und n 0 bedeuten und

Y' und p die gleichen Definitionen wie in Anspruch 9 besitzen.

15. DNA nach Anspruch 9, die eine Basensequenz hat oder enthält, welche durch die folgende Formel [II]:

$$(Y')_p - (X')_n - (B')_m - A' \qquad [II]$$

dargestellt wird, worin

A' eine Basensequenz der Formel [IIa] bedeutet, worin ACC und CAT in den 66. und 67. Codons von dem 5'-Terminus durch CAT, ACC oder TAC ersetzt sind,

m und n 0 bedeuten und

Y' und p die gleichen Definitionen wie in Anspruch 9 besitzen.

16. Vektor, in den eine DNA nach irgendeinem der vorhergehenden Ansprüche eingeführt wurde.

17. Vektor nach Anspruch 16, dadurch **gekennzeichnet**, daß er ein Expressionsvektor ist.

18. Expressionsvektor nach Anspruch 17, der eine Basensequenz enthält, die einer Aminosäuresequenz entspricht, welche durch die folgende Formel [I]:

$$(Y)_p - (X)_n - (B)_m - A \qquad [I]$$

dargestellt wird, worin

p 1 bedeutet und

A, B, X, Y, m und n die gleichen Definitionen wie in Anspruch 1 besitzen, und der ein Terminationscodon an dem 3'-Terminus der Basensequenz hat, wobei die Basensequenz unter Kontrolle eines Transcriptions-Promotors ist.

19. Expressionsvektor nach Anspruch 18, worin der Transcriptions-Promotor ein trp -Promotor oder ein tac -Promotor ist.

20. Expressionsvektor nach Anspruch 18, worin die Basensequenz, die der Aminosäuresequenz der Formel [I] wie in Anspruch 18 definiert, entspricht, die einen Terminationscodon an dem 3'-Terminus der Basensequenz enthält, in dem korrekten Translations-Leseraster mit einer zweiten Basensequenz verbunden ist, die das Protein, das fusioniert werden soll, codiert.

21. Expressionsvektor nach Anspruch 20, worin die zweite Basensequenz, die ein Protein, das fusioniert werden soll, codiert, ein phoS -Gen ist.

22. Vektor nach Anspruch 16 oder 21, der in Escherichia coli proliferieren kann.

23. Vektor nach Anspruch 22, der ein Escherichia coli -Plasmid ist.

24. Vektor nach Anspruch 23, der pBR322 ist.

25. Wirt, der mit einem Vektor der Ansprüche 16 bis 24 transformiert ist.

26. Wirt nach Anspruch 25, der ein Mikroorganismus ist.

27. Wirt nach Anspruch 26, der Escherichia coli ist.

28. Polypeptid, das ein humanes Tumor-Nekrosefaktor-Polypeptid hat oder enthält, sein Vorstufen-Polypeptid oder sein modifiziertes oder allelisch mutiertes Polypeptid, das eine Aminosäuresequenz enthält, die durch die folgende Formel [I] :

$$(Y)_p - (X)_n - (B)_m - A \qquad [I]$$

dargestellt wird, oder ein Derivat oder Salz davon, worin
A ein Polypeptid der folgenden Formel [Ia] bedeutet, worin ein bis zwölf Aminosäuren nicht vorhanden sein können oder worin eine oder zwei Aminosäuren durch eine andere Aminosäure bzw. andere Aminosäuren ersetzt sein kann bzw. können,
B ein Peptid der folgenden Formel [Ib] bedeutet, worin ein bis drei Aminosäuren nicht vorhanden sein können oder durch eine andere Aminosäure bzw. andere Aminosäuren ersetzt sein kann bzw. können,
X ein Polypeptid bedeutet,
Y Met bedeutet und
m, n und p 1 bzw. 0 bedeuten,
wobei die Formel [Ia] wie folgt ist:

```
Thr Pro Ser Asp Lys Pro Val Ala His Val

Val Ala Asn Pro Gln Ala Glu Gly Gln Leu

Gln Trp Leu Asn Arg Arg Ala Asn Ala Leu

Leu Ala Asn Gly Val Glu Leu Arg Asp Asn

Gln Leu Val Val Pro Ser Glu Gly Leu Tyr

Leu Ile Tyr Ser Gln Val Leu Phe Lys Gly

Gln Gly Cys Pro Ser Thr His Val Leu Leu

Thr His Thr Ile Ser Arg Ile Ala Val Ser

Tyr Gln Thr Lys Val Asn Leu Leu Ser Ala

Ile Lys Ser Pro Cys Gln Arg Glu Thr Pro

Glu Gly Ala Glu Ala Lys Pro Trp Tyr Glu

Pro Ile Tyr Leu Gly Gly Val Phe Gln Leu
```

```
Glu Lys Gly Asp Arg Leu Ser Ala Glu Ile

Asn Arg Pro Asp Tyr Leu Asp Phe Ala Glu

Ser Gly Gln Val Tyr Phe Gly Ile Ile Ala

Leu                              ... [Ia],
```

die Formel [Ib] wie folgt ist:

```
Ser-Ser-Ser-Arg                  ... [Ib]
```

und das Polypeptid, das durch die obige Formel B-A dargestellt wird, die funktionellen Eigenschaften wie ein entwickelter humaner Tumor-Nekrosefaktor aufweist.

29. Polypeptid nach Anspruch 28, das durch die folgende Formel [I]:

$$(Y)_p - (X)_n - (B)_m - A \qquad [I]$$

dargestellt wird, oder ein Derivat oder Salz davon, worin
A, B, Y, m, n und p die gleichen Bedeutungen wie in Anspruch 28 besitzen und X ein Polypeptid der Formel [Ic] wie folgt bedeutet:

```
Ser Thr Glu Ser Met Ile Arg Asp Val Glu

Leu Ala Glu Glu Ala Leu Pro Lys Lys Thr

Gly Gly Pro Gln Gly Ser Arg Arg Cys Leu

Phe Leu Ser Leu Phe Ser Phe Leu Ile Val

Ala Gly Ala Thr Thr Leu Phe Cys Leu Leu

His Phe Gly Val Ile Gly Pro Gln Arg Glu

Glu Phe Pro Arg Asp Leu Ser Leu Ile Ser

Pro Leu Ala Gln Ala Val Arg     ... [Ic]
```

und das Polypeptid, das durch die obige Formel Y-X-B-A dargestellt ist, die funktionellen Eigenschaften des Vorstufen-Polypeptids von humanem Tumor-Nekrosefaktor aufweist.

30. Polypeptid, das ein humanes Tumor-Nekrosefaktor-Vorstufenpolypeptid hat oder enthält, das eine Aminosäuresequenz, die durch die folgende Formel [I]:

$$(Y)_p - (X)_n - (B)_m - A \qquad [I]$$

dargestellt wird, enthält, worin
A ein Polypeptid der Formel [Ia], wie sie in Anspruch 28 gezeigt ist, bedeutet,
B ein Peptid der Formel [Ib], wie sie in Anspruch 28 gezeigt ist, bedeutet,
X ein Polypeptid der Formel [Ic], wie sie in Anspruch 29 gezeigt ist, bedeutet,
m und n 1 bedeuten und

Y und p die gleichen Definitionen wie in Anspruch 28 besitzen.

31. Polypeptid, das ein entwickeltes humanes Tumor-Nekrosefaktor-Polypeptid hat oder enthält, wobei das Polypeptid eine Aminosäuresequenz enthält, die durch die folgende Formel [I]:

$$(Y)_p - (X)_n - (B)_m - A \qquad [I]$$

dargestellt wird, worin
   A ein Polypeptid der Formel [Ia], wie sie in Anspruch 28 gezeigt ist, bedeutet,
   B ein Peptid der Formel [Ib], wie sie in Anspruch 28 gezeigt ist, bedeutet,
   m 1 bedeutet,
   n 0 bedeutet und
   Y und p die gleichen Definitionen wie in Anspruch 28 besitzen.

32. Polypeptid, das ein modifiziertes humanes Tumor-Nekrosefaktor-Polypeptid hat oder enthält, wobei das Polypeptid eine Aminosäuresequenz enthält, die durch die folgende Formel [I]:

$$(Y)_p - (X)_n - (B)_m - A \qquad [I]$$

dargestellt ist, worin
   A ein Polypeptid der Formel [Ia], wie sie in Anspruch 28 gezeigt ist, bedeutet,
   m und n 0 bedeuten und
   Y und p die gleichen Definitionen wie in Anspruch 28 besitzen.

33. Polypeptid nach Anspruch 31, das durch die folgende Formel [I]:

$$(Y)_p - (X)_n - (B)_m - A \qquad [I]$$

dargestellt wird, worin
   A ein Polypeptid der Formel [Ia], wie sie in Anspruch 28 angegeben ist, bedeutet,
   B ein Peptid der Formel [Ib], wie sie in Anspruch 28 angegeben ist, bedeutet,
   m 1 bedeutet und
   n und p 0 bedeuten.

34. Polypeptid nach Anspruch 32, das durch die folgende Formel [I]:

$$(Y)_p - (X)_n - (B)_m - A \qquad [I]$$

dargestellt wird, worin
   A ein Polypeptid der Formel [Ia], wie sie in Anspruch 28 angegeben ist, bedeutet und
   m, n und p 0 bedeuten.

35. Polypeptid, das ein modifiziertes humanes Tumor-Nekrosefaktor-Polypeptid hat oder enthält, wobei das Polypeptid eine Aminosäuresequenz enthält, die durch die folgende Formel [I]:

$$(Y)_p - (X)_n - (B)_m - A \qquad [I]$$

dargestellt wird, worin
   A ein Polypeptid der Formel [Ia] bedeutet, worin ein bis zwölf Aminosäuren nicht vorhanden sein

91

können oder worin ein bis zwei Aminosäuren durch eine andere Aminosäure bzw. andere Aminosäuren ersetzt sein kann bzw. können,

m und n 0 bedeuten und

Y und p die gleichen Definitionen wie in Anspruch 28 besitzen.

**36.** Polypeptid nach Anspruch 35, das durch die folgende Formel [1]:

$$(Y)_p - (X)_n - (B)_m - A \qquad [I]$$

dargestellt wird, worin

A ein Polypeptid der Formel [Ia] bedeutet, worin Thr in der ersten Stellung von dem N-Terminus, Aminosäuren von dem obigen Thr in der ersten Stellung zu Pro in der 6. Stellung, Aminosäuren von dem obigen Thr in der ersten Stellung zu His in der 9. Stellung oder Aminosäuren von dem obigen Thr in der ersten Stellung zu Ala in der 12. Stellung nicht vorhanden sein können,

m und n 0 bedeuten und

Y und p die gleichen Definitionen wie in Anspruch 28 besitzen.

**37.** Polypeptid nach Anspruch 35, das durch die folgende Formel [I]:

$$(Y)_p - (X)_n - (B)_m - A \qquad [I]$$

dargestellt wird, worin

A ein Polypeptid der Formel [Ia] bedeutet, worin Thr und His in den 66. und 67. Stellungen von dem N-Terminus durch His, Thr oder Tyr ersetzt sind,

m und n 0 bedeuten und

Y und p die gleichen Definitionen wie in Anspruch 28 besitzen.

**38.** Substanz, die durch chemische oder enzymatische Modifizierung eines Polypeptids nach einem der Ansprüche 30 bis 37 erhalten worden ist.

**39.** Pharmazeutisch annehmbares Salz eines Polypeptids nach einem der Ansprüche 30 bis 38.

**40.** Aggregat, das ein Polypeptid nach irgendeinem der Ansprüche 30 bis 38 als Untereinheit enthält oder daraus besteht.

**41.** Aggregat nach Anspruch 40, welches ein Trimer ist.

**42.** Trimer nach Anspruch 41, welcher ein Polypeptid nach Anspruch 33 als Untereinheit enthält oder daraus besteht.

**43.** Pharmazeutisches Präparat, das eine Substanz nach irgendeinem der Ansprüche 29 bis 42 als aktiven Bestandteil enthält.

**44.** Verwendung von irgendeiner der Substanzen nach den Ansprüchen 29 bis 43 als Anti-Tumormittel.

**45.** Verfahren zur Herstellung einer DNA, die eine Basensequenz hat oder enthält, die einer Aminosäuresequenz entspricht, welche durch die folgende Formel [I]:

$$(Y)_p - (X)_n - (B)_m - A \qquad [I]$$

dargestellt wird, worin

A, B, X,Y, m, n und p die gleichen Definitionen wie in Anspruch 1 besitzen, dadurch **gekennzeichnet**, daß

(1) humane Macrophagen zusammen mit einem oder mehreren Induktoren kultiviert werden,

(2) eine Fraktion, die einen humanen Tumor-Nekrosefaktor mRNA enthält, aus den Macrophagen abgetrennt wird,

(3) eine einstrangige cDNA aus der mRNA unter Verwendung der Umkehr-Transcriptase und dann Umwandlung in doppelstrangige cDNA hergestellt wird,

(4) die doppelstrangige cDNA in einen Vektor eingesetzt wird,

(5) der rekombinante Vektor in einen Wirt eingeführt wird, um ihn zu transformieren und eine cDNA-Library zu bilden,

(6) die cDNA, die das humane Tumor-Nekrosefaktor-Polypeptid oder sein allelisch mutiertes Polypeptid codiert, aus der Library cloniert und

(7) gegebenenfalls die clonierte cDNA modifiziert.

**46.** Verfahren nach Anspruch 45 für die Herstellung einer DNA, die eine Basensequenz hat oder enthält, welche durch die folgende Formel [II]:

$$(Y')_p - (X')_n - (B')_m - A' \qquad [II]$$

dargestellt wird, worin

A', B', X', Y', m, n und p die gleichen Definitionen wie in Anspruch 9 besitzen.

**47.** Verfahren zur Herstellung eines Polypeptids, das durch die folgende Formel [I]:

$$(Y)_p - (X)_n - (B)_m - A \qquad [I]$$

dargestellt wird, oder eines Derivates oder Salzes davon, worin

A, B, X, Y, m, n und p die gleichen Definitionen wie in Anspruch 28 besitzen, dadurch **gekennzeichnet**, daß eine DNA, die eine Basensequenz, welche das obige Polypeptid codiert, hat oder enthält, in einen Expressionsvektor eingesetzt wird, ein Wirt mit dem rekombinanten Vektor transformiert wird, der Wirt kultiviert wird und das entstehende Polypeptid gesammelt wird und gegebenenfalls das Polypeptid modifiziert wird.

Patentansprüche für folgenden Vertragsstaat: AT

**1.** Verfahren zur Herstellung einer DNA, die eine Basensequenz hat oder enthält, welche dem humanen Tumor-Nekrosefaktor-Polypeptid, seinem Vorstufen-Polypeptid oder seinem modifizierten oder allelisch mutierten Polypeptid entspricht und eine Aminosäuresequenz enthält, welche durch die folgende Formel [I] :

$$(Y)_p - (X)_n - (B)_m - A \qquad [I]$$

dargestellt wird, worin

A ein Polypeptid der folgenden Formel [Ia] bedeutet, worin eine bis zwölf Aminosäuren an dem N-Terminus nicht vorhanden sein können oder worin eine oder zwei Aminosäuren durch eine andere Aminosäure(n) ersetzt sein kann bzw. können.

B ein Peptid der folgenden Formel [Ib] bedeutet, worin eine bis drei Aminosäuren nicht vorhanden sein kann bzw. können oder durch andere Aminosäure(n) ersetzt sein kann bzw. können.

X ein Polypeptid bedeutet,

Y Met bedeutet und

m, n und p 1 oder 0 bedeuten;

die Formel [Ia] wie folgt ist:

```
Thr Pro Ser Asp Lys Pro Val Ala His Val
Val Ala Asn Pro Gln Ala Glu Gly Gln Leu


Gln Trp Leu Asn Arg Arg Ala Asn Ala Leu
Leu Ala Asn Gly Val Glu Leu Arg Asp Asn
Gln Leu Val Val Pro Ser Glu Gly Leu Tyr
Leu Ile Tyr Ser Gln Val Leu Phe Lys Gly
Gln Gly Cys Pro Ser Thr His Val Leu Leu
Thr His Thr Ile Ser Arg Ile Ala Val Ser
Tyr Gln Thr Lys Val Asn Leu Leu Ser Ala
Ile Lys Ser Pro Cys Gln Arg Glu Thr Pro
Glu Gly Ala Glu Ala Lys Pro Trp Tyr Glu
Pro Ile Tyr Leu Gly Gly Val Phe Gln Leu
Glu Lys Gly Asp Arg Leu Ser Ala Glu Ile
Asn Arg Pro Asp Tyr Leu Asp Phe Ala Glu
Ser Gly Gln Val Tyr Phe Gly Ile Ile Ala
Leu                             ... [Ia];
```

und die Formel [Ib] wie folgt ist:

```
          Ser-Ser-Ser-Arg          ... [Ib];
```

und wobei das Polypeptid, das durch die obige Formel B-A dargestellt wird, die funktionellen Eigenschaften als entwickelter humaner Nekrosefaktor aufweist, dadurch **gekennzeichnet**, daß

(1) humane Macrophagen zusammen mit einem oder mehreren Induktoren kultiviert werden,

(2) eine Fraktion, die einen humanen Tumor-Nekrosefaktor mRNA enthält, aus den Macrophagen abgetrennt wird,

(3) eine einstrangige cDNA aus der mRNA unter Verwendung der Umkehr-Transcriptase und dann Umwandlung in doppelstrangige cDNA hergestellt wird,

(4) die doppelstrangige cDNA in einen Vektor eingesetzt wird,

(5) der rekombinante Vektor in einen Wirt eingeführt wird, um ihn zu transformieren und eine cDNA-Library zu bilden,

(6) die cDNA, die das humane Tumor-Nekrosefaktor-Polypeptid oder sein allelisch mutiertes Polypeptid codiert, aus der Library cloniert und

(7) gegebenenfalls die clonierte cDNA modifiziert.

2. Verfahren nach Anspruch 1 für die Herstellung einer DNA, die eine Basensequenz hat oder enthält, welcher einer Aminosäuresequenz entspricht, die durch die folgende Formel [I] :

$$(Y)_p-(X)_n-(B)_m-A \ [I]$$

dargestellt wird, worin

A, B, Y, m, n und p die in Anspruch 1 gegebenen Definitionen besitzen und X ein Polypeptid der Formel [Ic] bedeutet:

```
Ser Thr Glu Ser Met Ile Arg Asp Val Glu

Leu Ala Glu Glu Ala Leu Pro Lys Lys Thr

Gly Gly Pro Gln Gly Ser Arg Arg Cys Leu

Phe Leu Ser Leu Phe Ser Phe Leu Ile Val

Ala Gly Ala Thr Thr Leu Phe Cys Leu Leu

His Phe Gly Val Ile Gly Pro Gln Arg Glu

Glu Phe Pro Arg Asp Leu Ser Leu Ile Ser

Pro Leu Ala Gln Ala Val Arg    ... [Ic],
```

und wobei das Polypeptid, das durch die obige Formel Y-X-B-A dargestellt ist, die funktionellen Eigenschaften eines Vorstufen-Polypeptids des humanen Tumor-Nekrosefaktors aufweist.

3. Verfahren nach Anspruch 1 für die Herstellung einer DNA, die eine Basenseguenz hat oder enthält, welche dem humanen Tumor-Nekrosefaktor-Vorstufenpolypeptid, das eine Aminosäureseguenz enthält, welche durch die folgende Formel [I] :

$$(Y)_p - (X)_n - (B)_m - A \qquad [I]$$

dargestellt wird, oder seinem allelisch mutierten Polypeptid entspricht, worin
A ein Polypeptid der Formel [Ia], wie sie in Anspruch 1 dargestellt ist, bedeutet,
B ein Peptid der Formel [Ib], wie sie in Anspruch 1 dargestellt ist, bedeutet,
Y die gleiche Definition wie in Anspruch 1 besitzt,
X ein Polypeptid der Formel [Ic], wie sie in Anspruch 2 dargestellt ist, bedeutet und
m, n und p 1 bedeuten.

4. Verfahren nach Anspruch 1 für die Herstellung einer DNA, die eine Basensequenz hat oder enthält, welche dem ausgereiften humanen Tumor-Nekrosefaktor-Polypeptid, das eine Aminosäuresequenz, welche durch die folgende Formel [I] :

$$(Y)_p - (X)_n - (B)_m - A \qquad [I]$$

dargestellt wird, enthält oder seinem allelisch mutierten Polypeptid entspricht, worin
A ein Polypeptid der Formel [Ia], wie sie in Anspruch 1 dargestellt wird, bedeutet,
B ein Peptid der Formel [Ib], wie sie in Anspruch 1 dargestellt wird, bedeutet,
m 1, n 0 bedeuten und
Y und p die gleichen Definitionen wie in Anspruch 1 besitzen.

5. Verfahren nach Anspruch 1 für die Herstellung einer DNA, die eine Basensequenz hat oder enthält, welche dem modifizierten humanen Tumor-Nekrosefaktor-Polypeptid entspricht, das eine Aminosäuresequenz enthält, die durch die folgende Formel [I] :

$$(Y)_p - (X)_n - (B)_m - A \qquad [I]$$

dargestellt wird, worin

A ein Polypeptid der Formel [Ia], wie sie in Anspruch 1 dargestellt wird, bedeutet,
n und m 0 bedeuten und
Y und p die gleichen Definitionen wie in Anspruch 1 besitzen.

6. Verfahren nach Anspruch 1 für die Herstellung einer DNA, die eine Basensequenz hat oder enthält, die dem modifizierten humanen Tumor-Nekrosefaktor-Polypeptid entspricht, das eine Aminosäuresequenz enthält, welche durch die folgende Formel [I] :

$$(Y)_p - (X)_n - (B)_m - A \qquad [I]$$

dargestellt wird, worin

A ein Polypeptid der Formel [Ia] bedeutet, worin eine bis zwölf Aminosäuren weggelassen sein können oder eine oder zwei Aminosäuren durch eine andere Aminosäure(n) ersetzt sein kann bzw. können,
m und n 0 bedeuten und
Y und p die gleichen Definitionen wie in Anspruch 1 besitzen.

7. Verfahren nach Anspruch 1 für die Herstellung einer DNA, die eine Basensequenz hat oder enthält, die dem modifizierten humanen Tumor-Nekrosefaktor-Polypeptid entspricht, welches eine Aminosäuresequenz enthält, die durch die folgende Formel [I] :

$$(Y)_p - (X)_n - (B)_m - A \qquad [I]$$

dargestellt wird, worin

A ein Polypeptid der Formel [Ia] bedeutet, worin Thr in der ersten Stellung von dem N-Terminus, Aminosäuren von dem obigen Thr in der ersten Stellung zu Pro in der 6. Stellung, Aminosäuren von dem obigen Thr in der ersten Stellung zu His in der 9. Stellung oder Aminosäuren von dem obigen Thr in der ersten Stellung zu Ala in der 12. Stellung nicht vorhanden sind,
m und n 0 bedeuten und
Y und p die gleichen Definitionen wie in Anspruch 1 besitzen.

8. Verfahren nach Anspruch 1 für die Herstellung einer DNA, die eine Basensequenz hat oder enthält, die dem modifizierten humanen Tumor-Nekrosefaktor-Polypeptid entspricht, das eine Aminosäuresequenz enthält, die durch die folgende Formel [I] :

$$(Y)_p - (X)_n - (B)_m - A \qquad [I]$$

dargestellt wird, worin

A ein Polypeptid der Formel [Ia] bedeutet, worin Thr und His in den 66. und 67. Stellungen von dem N-Terminus durch His, Thr oder Tyr ersetzt sind,
m und n 0 bedeuten und
Y und p die gleichen Definitionen wie in Anspruch 1 besitzen.

9. Verfahren nach Anspruch 1 für die Herstellung einer DNA, die eine Basensequenz hat oder enthält, welche durch die folgende Formel [II] :

$$(Y')_p - (X')_n - (B')_m - A' \qquad\qquad [II]$$

dargestellt wird, worin

A' eine Basensequenz der folgenden Formel [IIa] bedeutet, worin ein bis zwölf Codon(s) an dem 5'-Terminus weggelassen sein kann bzw. können oder worin eine oder zwei Aminosäuren durch einen anderen Codon bzw. andere Codons ersetzt sein kann bzw. können,

B' eine Basensequenz der folgenden Formel [IIb] bedeutet, worin ein bis drei Codon(s) nicht vorhanden sein können oder durch einen anderen Codon bzw. andere Codons ersetzt sein kann bzw. können,

X' eine Basensequenz der folgenden Formel [IIc] bedeutet,

Y' ATG bedeutet und

m, n und p 1 oder 0 bedeuten, wobei die Formel [IIa] wie folgt ist:

```
(5')-ACC CCG AGT GAC AAG CCT GTA GCC
CAT GTT GTA GCA AAC CCT CAA GCT GAG GGG
CAG CTC CAG TGG CTG AAC CGC CGG GCC AAT
GCC CTC CTG GCC AAT GGC GTG GAG CTG AGA
GAT AAC CAG CTG GTG GTG CCA TCA GAG GGC
CTG TAC CTC ATC TAC TCC CAG GTC CTC TTC
AAG GGC CAA GGC TGC CCC TCC ACC CAT GTG
CTC CTC ACC CAC ACC ATC AGC CGC ATC GCC
GTC TCC TAC CAG ACC AAG GTC AAC CTC CTC
TCT GCC ATC AAG AGC CCC TGC CAG AGG GAG
ACC CCA GAG GGG GCT GAG GCC AAG CCC TGG
TAT GAG CCC ATC TAT CTG GGA GGG GTC TTC
CAG CTG GAG AAG GGT GAC CGA CTC AGC GCT
GAG ATC AAT CGG CCC GAC TAT CTC GAC TTT
GCC GAG TCT GGG CAG GTC TAC TTT GGG ATC
ATT GCC CTG-(3')                    ... [IIa],
```

die Formel [IIb] wie folgt ist:

```
(5')-TCA-TCT-TCT-CGA-(3')            [IIb],
```

die Formel [IIc] wie folgt ist:

```
(5')-AGC ACT GAA AGC ATG ATC CGG GAC

GTG GAG CTG GCC GAG GAG GCG CTC CCC AAG

AAG ACA GGG GGG CCC CAG GGC TCC AGG CGG

TGC TTG TTC CTC AGC CTC TTC TCC TTC CTG

ATC GTG GCA GGC GCC ACC ACG CTC TTC TGC

CTG CTG CAC TTT GGA GTG ATC GGC CCC CAG

AGG GAA GAG TTC CCC AGG GAC CTC TCT CTA

ATC AGC CCT CTG GCC CAG GCA GTC AGA-(3')

                              ... [IIc].
```

**10.** Verfahren zur Herstellung eines Polypeptids, das ein humanes Tumor-Nekrosefaktor-Polypeptid hat oder enthält, sein Vorstufen-Polypeptid oder sein modifiziertes oder allelisch mutiertes Polypeptid, das eine Aminosäuresequenz enthält, die durch die folgende Formel [I] :

$$(Y)_p - (X)_n - (B)_m - A \qquad [I]$$

dargestellt wird, oder eines Derivats oder Salzes davon, worin

A ein Polypeptid der folgenden Formel [Ia] bedeutet, worin ein bis zwölf Aminosäuren nicht vorhanden sein können oder worin eine oder zwei Aminosäuren durch eine andere Aminosäure bzw. andere Aminosäuren ersetzt sein kann bzw. können,

B ein Peptid der folgenden Formel [Ib] bedeutet, worin ein bis drei Aminosäuren nicht vorhanden sein können oder durch eine andere Aminosäure bzw. andere Aminosäuren ersetzt sein kann bzw. können,

X ein Polypeptid bedeutet,

Y Met bedeutet und

m, n und p 1 bzw. 0 bedeuten,

wobei die Formel [Ia] wie folgt ist:

```
Thr Pro Ser Asp Lys Pro Val Ala His Val

Val Ala Asn Pro Gln Ala Glu Gly Gln Leu

Gln Trp Leu Asn Arg Arg Ala Asn Ala Leu

Leu Ala Asn Gly Val Glu Leu Arg Asp Asn

Gln Leu Val Val Pro Ser Glu Gly Leu Tyr

Leu Ile Tyr Ser Gln Val Leu Phe Lys Gly

Gln Gly Cys Pro Ser Thr His Val Leu Leu

Thr His Thr Ile Ser Arg Ile Ala Val Ser

Tyr Gln Thr Lys Val Asn Leu Leu Ser Ala

Ile Lys Ser Pro Cys Gln Arg Glu Thr Pro

Glu Gly Ala Glu Ala Lys Pro Trp Tyr Glu

Pro Ile Tyr Leu Gly Gly Val Phe Gln Leu

Glu Lys Gly Asp Arg Leu Ser Ala Glu Ile

Asn Arg Pro Asp Tyr Leu Asp Phe Ala Glu

Ser Gly Gln Val Tyr Phe Gly Ile Ile Ala

Leu                              ... [Ia],
```

die Formel [Ib] wie folgt ist:

$$Ser-Ser-Ser-Arg \qquad \cdots \quad [Ib]$$

und das Polypeptid, das durch die obige Formel B-A dargestellt wird, die funktionellen Eigenschaften wie ein entwickelter humaner Tumor-Nekrosefaktor aufweist, dadurch gekennzeichnet, daß eine DNA, die eine Basensequenz hat oder enthält, welche das obige Polypeptid codiert, in einen Expressionsvektor eingesetzt wird, ein Wirt mit dem rekombinanten Vektor transformiert wird, der Wirt kultiviert wird und das entstehende Polypeptid isoliert und gegebenenfalls das Polypeptid modifiziert wird.

**11.** Verfahren nach Anspruch 10 für die Herstellung eines Polypeptids, welches ein Polypeptid hat oder enthält, das durch die folgende Formel [I] :

$$(Y)_p - (X)_n - (B)_m - A \qquad [I]$$

dargestellt wird, oder eines Derivats oder Salzes davon, worin

A, B, Y, m, n und p die gleichen Bedeutungen wie in Anspruch 10 besitzen und X ein Polypeptid der Formel [Ic] wie folgt bedeutet:

```
Ser Thr Glu Ser Met Ile Arg Asp Val Glu

Leu Ala Glu Glu Ala Leu Pro Lys Lys Thr

Gly Gly Pro Gln Gly Ser Arg Arg Cys Leu

Phe Leu Ser Leu Phe Ser Phe Leu Ile Val

Ala Gly Ala Thr Thr Leu Phe Cys Leu Leu

His Phe Gly Val Ile Gly Pro Gln Arg Glu

Glu Phe Pro Arg Asp Leu Ser Leu Ile Ser

Pro Leu Ala Gln Ala Val Arg     ... [Ic]
```

und das Polypeptid, das durch die obige Formel Y-X-B-A dargestellt ist, die funktionellen Eigenschaften des Vorstufen-Polypeptids von humanem Tumor-Nekrosefaktor aufweist.

12. Verfahren nach Anspruch 10 für die Herstellung eines Polypeptids, das ein humanes Tumor-Nekrosefaktor-Vorstufenpolypeptid hat oder enthält, das eine Aminosäuresequenz hat oder enthält, die durch die folgende Formel [I] :

$$(Y)_p - (X)_n - (B)_m - A \qquad [I]$$

dargestellt wird, enthält, worin
A ein Polypeptid der Formel [Ia], wie sie in Anspruch 10 gezeigt ist, bedeutet,
B ein Peptid der Formel [Ib], wie sie in Anspruch 10 gezeigt ist, bedeutet,
X ein Polypeptid der Formel [Ic], wie sie in Anspruch 11 gezeigt ist, bedeutet,
m und n 1 bedeuten und
Y und p die gleichen Definitionen wie in Anspruch 10 besitzen.

13. Verfahren nach Anspruch 10 für die Herstellung eines Polypeptids, das ein entwickeltes humanes Tumor-Nekrosefaktor-Polypeptid hat oder enthält, wobei das Polypeptid eine Aminosäuresequenz enthält, die durch die folgende Formel [I] :

$$(Y)_p - (X)_n - (B)_m - A \qquad [I]$$

dargestellt wird, worin
A ein Polypeptid der Formel [Ia], wie sie in Anspruch 10 gezeigt ist, bedeutet,
B ein Peptid der Formel [Ib], wie sie in Anspruch 10 gezeigt ist, bedeutet,
m 1 bedeutet,
n 0 bedeutet und
Y und p die gleichen Definitionen wie in Anspruch 10 besitzen.

14. Verfahren nach Anspruch 10 für die Herstellung eines Polypeptids, das ein modifiziertes humanes Tumor-Nekrosefaktor-Polypeptid hat oder enthält, wobei das Polypeptid eine Aminosäuresequenz enthält, die durch die folgende Formel [I] :

$$(Y)_p - (X)_n - (B)_m - A \qquad\qquad [I]$$

dargestellt ist, worin

A ein Polypeptid der Formel [Ia], wie sie in Anspruch 10 gezeigt ist, bedeutet,

m und n 0 bedeuten und

Y und p die gleichen Definitionen wie in Anspruch 10 besitzen.

15. Verfahren nach Anspruch 10 für die Herstellung eines Polypeptids wie in Anspruch 13 beansprucht, das durch die folgende Formel [I] :

$$(Y)_p - (X)_n - (B)_m - A \qquad\qquad [I]$$

dargestellt wird, worin

A ein polypeptid der Formel [Ia], wie sie in Anspruch 10 angegeben ist, bedeutet,

B ein Peptid der Formel [Ib], wie sie in Anspruch 10 angegeben ist, bedeutet,

m 1 bedeutet und

n und p 0 bedeuten.

16. Verfahren nach Anspruch 10 für die Herstellung eines Polypeptids wie in Anspruch 14 beansprucht, das durch die folgende Formel [I] :

$$(Y)_p - (X)_n - (B)_m - A \qquad\qquad [I]$$

dargestellt wird, worin

A ein Polypeptid der Formel [Ia], wie sie in Anspruch 10 angegeben ist, bedeutet und

m, n und p 0 bedeuten.

17. Verfahren nach Anspruch 10 für die Herstellung eines Polypeptids, das ein modifiziertes humanes Tumor-Nekrosefaktor-Polypeptid hat oder enthält, wobei das Polypeptid eine Aminosäuresequenz enthält, die durch die folgende Formel [I] :

$$(Y)_p - (X)_n - (B)_m - A \qquad\qquad [I]$$

dargestellt wird, worin

A ein Polypeptid der Formel [Ia] bedeutet, worin ein bis zwölf Aminosäuren nicht vorhanden sein können oder worin ein bis zwei Aminosäuren durch eine andere Aminosäure bzw. andere Aminosäuren ersetzt sein kann bzw. können,

m und n 0 bedeuten und

Y und p die gleichen Definitionen wie in Anspruch 10 besitzen.

18. Verfahren nach Anspruch 10 für die Herstellung eines Polypeptids wie in Anspruch 17 beansprucht, das durch die folgende Formel [I] :

$$(Y)_p - (X)_n - (B)_m - A \qquad\qquad [I]$$

dargestellt wird, worin

A ein Polypeptid der Formel [Ia] bedeutet, worin Thr in der ersten Stellung von dem N-Terminus, Aminosäuren von dem obigen Thr in der ersten Stellung zu Pro in der 6. Stellung, Aminosäuren von dem obigen Thr in der ersten Stellung zu His in der 9. Stellung oder Aminosäuren von dem obigen Thr in der ersten Stellung zu Ala in der 12. Stellung nicht vorhanden sein können,

m und n 0 bedeuten und

Y und p die gleichen Definitionen wie in Anspruch 10 besitzen.

**19.** Verfahren nach Anspruch 10 für die Herstellung eines Polypeptids, wie in Anspruch 17 beansprucht, das durch die folgende Formel [I] :

$$(Y)_p - (X)_n - (B)_m - A \qquad \cdot \quad [I]$$

dargestellt wird, worin

A ein Polypeptid der Formel [Ia] bedeutet, worin Thr und His in den 66. und 67. Stellungen von dem N-Terminus durch His, Thr oder Tyr ersetzt sind,

m und n 0 bedeuten und

Y und p die gleichen Definitionen wie in Anspruch 10 besitzen.